(19) Europäisches Patentamt
European Patent Office
Office européen des brevets

(11) EP 3 828 206 A1

(12) **EUROPEAN PATENT APPLICATION**
published in accordance with Art. 153(4) EPC

(43) Date of publication:
**02.06.2021 Bulletin 2021/22**

(21) Application number: **19842020.0**

(22) Date of filing: **24.07.2019**

(51) Int Cl.:
*C07K 16/32* (2006.01) *A61K 9/08* (2006.01)
*A61K 47/18* (2017.01) *A61K 47/22* (2006.01)
*A61K 47/26* (2006.01) *A61K 47/68* (2017.01)
*A61P 35/00* (2006.01) *A61K 31/4745* (2006.01)
*A61K 39/395* (2006.01)

(86) International application number:
**PCT/JP2019/028949**

(87) International publication number:
**WO 2020/022363 (30.01.2020 Gazette 2020/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **25.07.2018 JP 2018139633**

(71) Applicant: **Daiichi Sankyo Company, Limited**
**Tokyo 103-8426 (JP)**

(72) Inventors:
- **YAMAGUCHI Tatsuya**
  **Tokyo 103-8426 (JP)**
- **SAKURATANI Kenji**
  **Tokyo 103-8426 (JP)**

(74) Representative: **Wallace, Sheila Jane**
**Marks & Clerk LLP**
**15 Fetter Lane**
**London EC4A 1BW (GB)**

(54) **EFFECTIVE METHOD FOR MANUFACTURING ANTIBODY-DRUG CONJUGATE**

(57) A method for producing an antibody-drug conjugate in which a drug-linker represented by formula (1) (wherein A represents the connecting position to an antibody) is conjugated to the antibody via a thioether bond, wherein the method comprising the steps of: (i) reducing the antibody with a reducing agent; (ii) reacting a drug-linker intermediate with the antibody reduced in step (i); (iii) adding a reagent having a thiol group to react with the residual drug-linker intermediate in step (ii); and then (iv) removing by-products derived from the drug-linker intermediate through ultrafiltration using a buffer solution containing a salt consisting of a strong acid and a strong base, and a method for producing a pharmaceutical composition containing the antibody-drug conjugate.

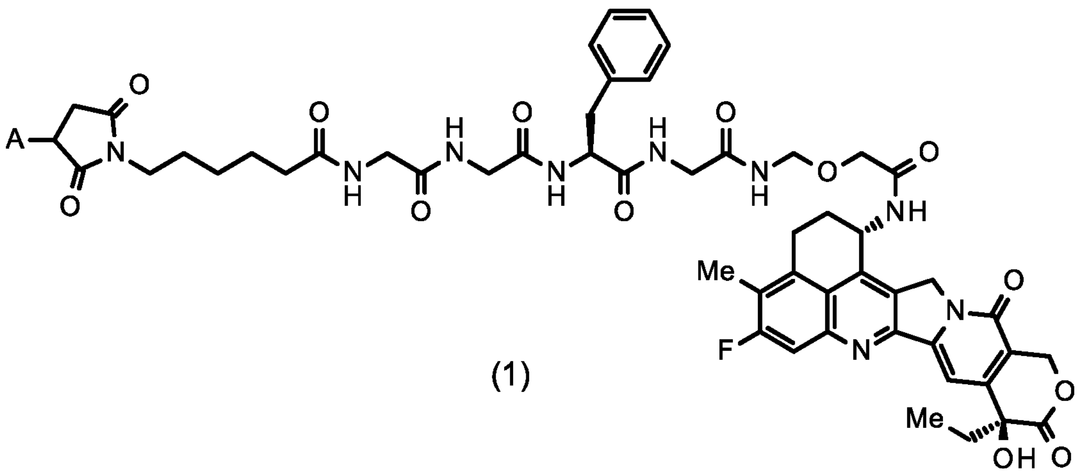

(1)



Printed by Jouve, 75001 PARIS (FR)

## Description

### Technical Field

The present invention relates to a method for producing an antibody-drug conjugate, which reduces the generation of aggregates and comprises a purification step for effectively removing by-products, and a method for producing a pharmaceutical composition containing the antibody-drug conjugate.

### Background Art

An antibody-drug conjugate (ADC) having a drug with cytotoxicity conjugated to an antibody which binds to an antigen expressed on the surface of cancer cells and is also capable of cellular internalization can deliver the drug selectively to cancer cells, and is thus expected to cause accumulation of the drug within cancer cells and to kill the cancer cells (Non-Patent References 1 to 5).

Examples in Patent References 1 to 9 describe methods for producing an antibody-drug conjugate. These production methods each include a purification step involving chromatography such as hydrophobic chromatography and gel filtration chromatography.

As one such antibody-drug conjugate, an antibody-drug conjugate comprising an antibody and a derivative of exatecan, which is a topoisomerase I inhibitor, as its components is known (Patent References 10 to 14, Non-Patent References 6 to 9). Since these antibody-drug conjugates exert a particularly superior antitumor effect and are safe, they are currently under clinical studies.

Patent References 10 to 14 describe methods for conjugating an antibody and a drug-linker intermediate as methods for producing the above antibody-drug conjugate, and methods for purifying the antibody-drug conjugate obtained.

Examples in Patent References 11 to 13 disclose that the above antibody-drug conjugate was purified through ultrafiltration with a sorbitol-containing acetate buffer solution. Patent Reference 14 discloses that the above antibody-drug conjugate can be purified through ultrafiltration with an acetate buffer solution, a histidine buffer solution, or a phosphate buffer solution. Nevertheless, development of an industrially better method for producing an antibody-drug conjugate is still demanded.

### Citation List

#### Patent Literature

Patent Reference 1: International Publication No. 2002/098883
Patent Reference 2: International Publication No. 2005/037992
Patent Reference 3: International Publication No. 2005/084390
Patent Reference 4: International Publication No. 2006/086733
Patent Reference 5: International Publication No. 2007/024536
Patent Reference 6: International Publication No. 2010/141566
Patent Reference 7: International Publication No. 2011/039724
Patent Reference 8: International Publication No. 2012/135517
Patent Reference 9: International Publication No. 2015/104359
Patent Reference 10: International Publication No. 2014/057687
Patent Reference 11: International Publication No. 2015/098099
Patent Reference 12: International Publication No. 2015/115091
Patent Reference 13: International Publication No. 2015/155998
Patent Reference 14: International Publication No. 2017/002776


#### Non-Patent Literature

Non-Patent Reference 1: Ducry, L., et al., Bioconjugate Chem. (2010) 21, 5-13.
Non-Patent Reference 2: Alley, S. C., et al., Current Opinion in Chemical Biology (2010) 14, 529-537.
Non-Patent Reference 3: Damle N. K. Expert Opin. Biol. Ther. (2004) 4, 1445-1452.

Non-Patent Reference 4: Senter P. D., et al., Nature Biotechnology (2012) 30 631-637.
Non-Patent Reference 5: Howard A. et al., J Clin Oncol 29: 398-405.
Non-Patent Reference 6: Ogitani Y. et al., Clinical Cancer Research (2016) 22(20), 5097-5108.
Non-Patent Reference 7: Ogitani Y. et al., Cancer Science (2016) 107, 1039-1046.
Non-Patent Reference 8: Doi T, et al., Lancet Oncol 2017; 18: 1512-22.
Non-Patent Reference 9: Takegawa N, et al., Int. J. Cancer: 141, 1682-1689 (2017).

Summary of Invention

Technical Problem

The antibody-drug conjugate according to the production method of the present invention is an antibody-drug conjugate, in which a drug-linker represented by formula (1)

[Chem. 1]

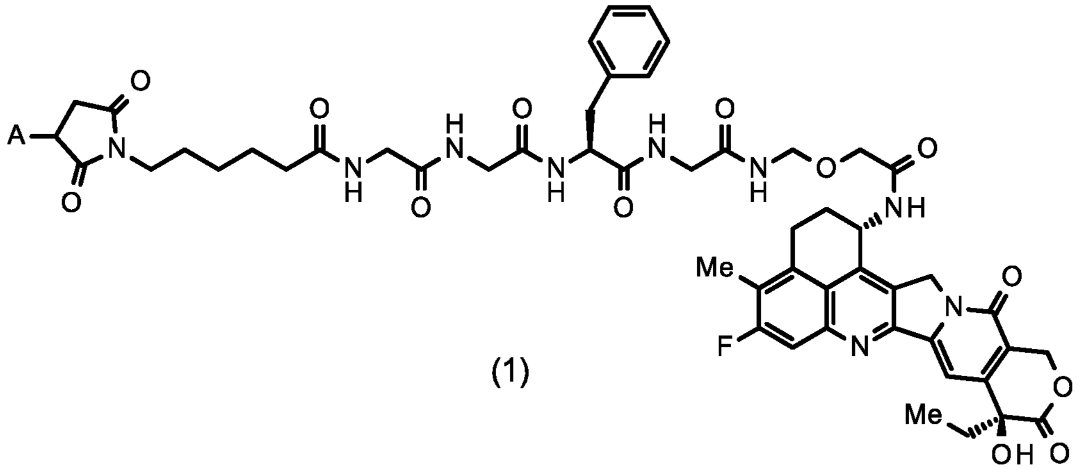

(1)

wherein A represents the connecting position to an antibody,
is conjugated to an antibody via a thioether bond.

Examples of methods for producing such an antibody-drug conjugate include a method comprising the steps of:

(i) reducing an antibody with a reducing agent;
(ii) reacting a compound represented by formula (2)

[Chem. 2]

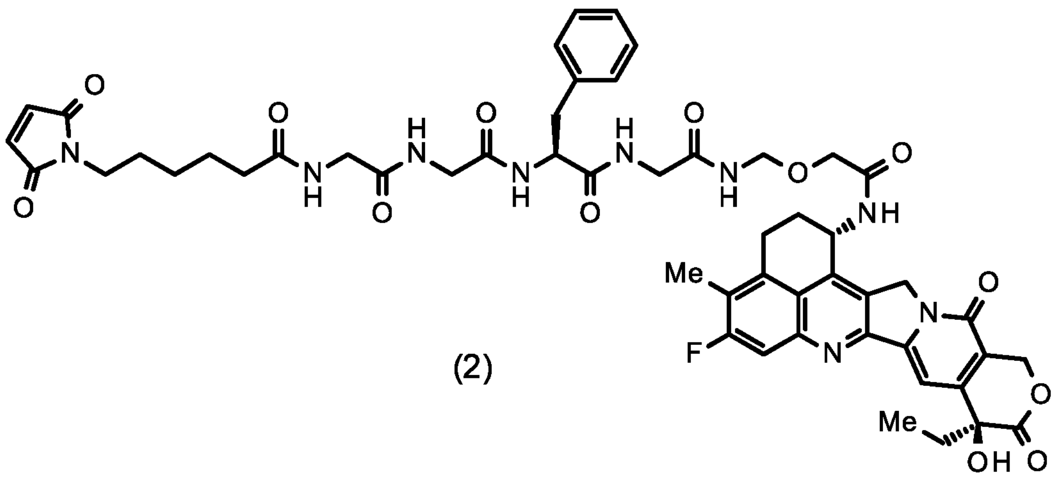

(2)

with the antibody reduced in step (i); and then

(iii) adding a reagent having a thiol group to react with the residual compound represented by formula (2) in step (ii).

To produce the antibody-drug conjugate, in particular, an antibody-drug conjugate having an average number of units of the drug-linker conjugated per antibody molecule is in the range of from 7 to 8, a huge amount of the compound represented by formula (2) is required, and this can lead to generation of a huge amount of by-products derived from the compound represented by formula (2). Suppression of the generation of aggregates is demanded in production of an antibody-drug conjugate, and examination of reaction/purification conditions is needed while considering physical properties unique to each of the antibody moiety and drug-linker moiety.

An object of the present invention is to provide an industrially excellent method for producing an antibody-drug conjugate, which comprises a purification step for effectively removing by-products derived from the compound represented by formula (2), and suppresses the generation of aggregates. Another object of the present invention is to establish an industrially excellent method for producing a pharmaceutical composition containing the antibody-drug conjugate.

Solution to Problem

As a result of diligent studies in order to solve the above problems, the present inventors have found that by-products derived from the compound represented by formula (2) can be effectively removed through ultrafiltration with a buffer solution containing a salt consisting of a strong acid and a strong base in the step of purifying an antibody-drug conjugate. In addition, the present inventors have found reaction/purification conditions that can suppress the generation of aggregates in the steps of producing an antibody-drug conjugate and purifying the antibody-drug conjugate. These findings successfully provided an industrially excellent method for producing a pharmaceutical composition containing the antibody-drug conjugate.

Thus, the present invention provides the following [1] to [410].

[1] A method for producing an antibody-drug conjugate, in which a drug-linker represented by formula (1)

[Chem. 3]

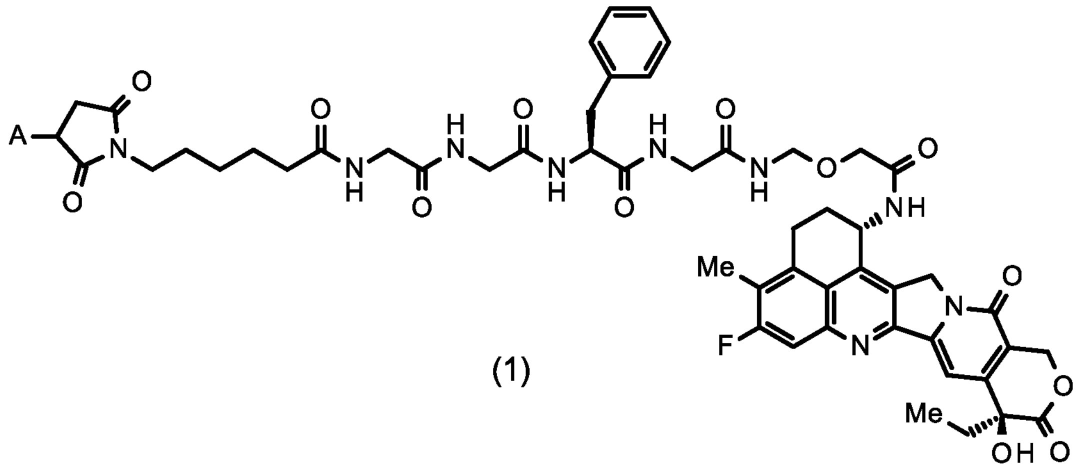

(1)

wherein A represents the connecting position to an antibody, is conjugated to the antibody via a thioether bond, wherein the method comprises the steps of:

(i) reducing the antibody with a reducing agent;

(ii) reacting a compound represented by formula (2)

[Chem. 4]

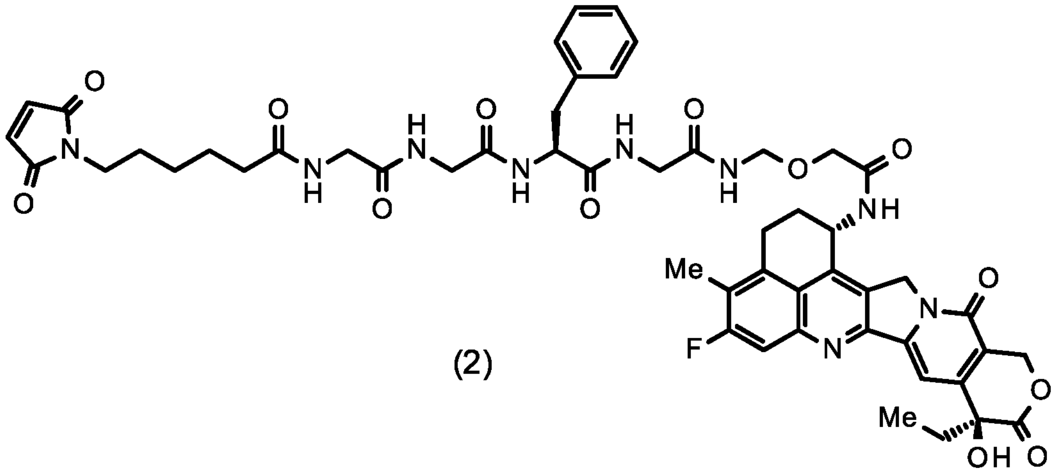

(2)

with the antibody reduced in step (i);
(iii) adding a reagent having a thiol group to react with the residual compound represented by formula (2) in step (ii); and then
(iv) removing a compound in which the reducing agent used in step (i) is added to the maleimidyl group of the compound represented by formula (2), and a compound in which the reagent having a thiol group used in step (iii) is added to the maleimidyl group of the compound represented by formula (2), through ultrafiltration using a buffer solution containing a salt consisting of a strong acid and a strong base.

[2] The production method according to [1], wherein the reducing agent used in step (i) is tris(2-carboxyethyl)phosphine or a salt thereof.
[3] The production method according to [1], wherein the reducing agent used in step (i) is tris(2-carboxyethyl)phosphine hydrochloride.
[4] The production method according to any one of [1] to [3], wherein step (i) is performed in a buffer solution.
[5] The production method according to [4], wherein the pH of the buffer solution is adjusted to 6 to 8 by using an aqueous solution of disodium hydrogen phosphate.
[6] The production method according to [4] or [5], wherein the buffer solution is an acetate buffer solution.
[7] The production method according to any one of [1] to [6], wherein step (i) is performed in the presence of a chelating agent.
[8] The production method according to [7], wherein the chelating agent is ethylenediaminetetraacetic acid.
[9] The production method according to any one of [4] to [8], wherein the buffer solution used in step (i) contains a surfactant.
[10] The production method according to [9], wherein the surfactant is polysorbate 20.
[11] The production method according to [9], wherein the surfactant is polysorbate 80.
[12] The production method according to any one of [1] to [11], wherein the reagent having a thiol group used in step (iii) is N-acetylcysteine.
[13] The production method according to any one of [1] to [12], wherein the pH of the buffer solution used in step (iv) is about 5.
[14] The production method according to any one of [1] to [12], wherein the pH of the buffer solution used in step (iv) is in the range of from 4.7 to 5.3.
[15] The production method according to any one of [1] to [12], wherein the pH of the buffer solution used in step (iv) is in the range of from 4.8 to 5.2.
[16] The production method according to any one of [1] to [12], wherein the pH of the buffer solution used in step (iv) is in the range of from 4.9 to 5.1.
[17] The production method according to any one of [1] to [12], wherein the pH of the buffer solution used in step (iv) is 5.0.
[18] The production method according to any one of [1] to [17], wherein the buffer solution used in step (iv) is a histidine buffer solution.
[19] The production method according to any one of [1] to [18], wherein the concentration of the salt consisting of a strong acid and a strong base used in step (iv) is in the range of from 0.2 wt% to 1 wt% with respect to the buffer

solution used in step (iv).

[20] The production method according to any one of [1] to [18], wherein the concentration of the salt consisting of a strong acid and a strong base used in step (iv) is about 0.5 wt% with respect to the buffer solution used in step (iv).

[21] The production method according to any one of [1] to [18], wherein the concentration of the salt consisting of a strong acid and a strong base used in step (iv) is in the range of from 0.4 wt% to 0.6 wt% with respect to the buffer solution used in step (iv).

[22] The production method according to any one of [1] to [18], wherein the concentration of the salt consisting of a strong acid and a strong base used in step (iv) is 0.5 wt% with respect to the buffer solution used in step (iv).

[23] The production method according to any one of [1] to [22], wherein the salt consisting of a strong acid and a strong base used in step (iv) is sodium chloride.

[24] The production method according to any one of [1] to [23], comprising a step subsequent to step (iv) of
(v) removing the salt consisting of a strong acid and a strong base through ultrafiltration using a buffer solution.

[25] The production method according to [24], wherein the pH of the buffer solution used in step (v) is in the range of from 4 to 6.

[26] The production method according to [24], wherein the pH of the buffer solution used in step (v) is about 5.

[27] The production method according to [24], wherein the pH of the buffer solution used in step (v) is in the range of from 4.7 to 5.3.

[28] The production method according to [24], wherein the pH of the buffer solution used in step (v) is in the range of from 4.8 to 5.2.

[29] The production method according to [24], wherein the pH of the buffer solution used in step (v) is in the range of from 4.9 to 5.1.

[30] The production method according to [24], wherein the pH of the buffer solution used in step (v) is 5.0.

[31] The production method according to any one of [24] to [30], wherein the buffer solution used in step (v) is a histidine buffer solution.

[32] The production method according to any one of [1] to [31], wherein the average number of units of the drug-linker conjugated per antibody molecule in the antibody-drug conjugate is in the range of from 7 to 8.

[33] The production method according to any one of [1] to [31], wherein the average number of units of the drug-linker conjugated per antibody molecule in the antibody-drug conjugate is in the range of from 7.5 to 8.

[34] The production method according to any one of [1] to [33], wherein the antibody is an anti-HER2 antibody.

[35] The production method according to [34], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[36] The production method according to [34], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[37] The production method according to any one of [1] to [33], wherein the antibody is an anti-HER3 antibody.

[38] The production method according to [37], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[39] The production method according to [38], wherein the anti-HER3 antibody lacks a lysine residue at the carboxyl terminus of the heavy chain.

[40] The production method according to any one of [1] to [33], wherein the antibody is an anti-GPR20 antibody.

[41] The production method according to [40], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.

[42] The production method according to [41], wherein the anti-GPR20 antibody lacks a lysine residue at the carboxyl terminus of the heavy chain.

[43] The production method according to any one of [1] to [33], wherein the antibody is an anti-CDH6 antibody.

[44] The production method according to [43], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[45] The production method according to [44], wherein the anti-CDH6 antibody lacks a lysine residue at the carboxyl terminus of the heavy chain.

[46] The production method according to any one of [1] to [45], comprising no purification step involving chromatography.

[47] The production method according to [46], wherein the chromatography is at least one selected from the group consisting of gel filtration chromatography, ion exchange chromatography, hydrophobic chromatography, and affinity chromatography.

[48] A method for producing a pharmaceutical composition containing an antibody-drug conjugate, a buffer solution, and an excipient, by producing the antibody-drug conjugate by the production method according to any one of [1] to [47], and then
performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[49] The production method according to [48], wherein the buffer solution is a histidine buffer solution.
[50] The production method according to [48] or [49], wherein the excipient is sucrose.
[51] The production method according to [48] or [49], wherein the excipient is trehalose.
[52] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [48] to [51], and then performing the step of
(x) adding the surfactant to the pharmaceutical composition.
[53] The production method according to [52], wherein the surfactant is polysorbate 80.
[54] The production method according to [52], wherein the surfactant is polysorbate 20.
[55] A method for producing an antibody-drug conjugate, in which
a drug-linker represented by formula (1)

[Chem. 5]

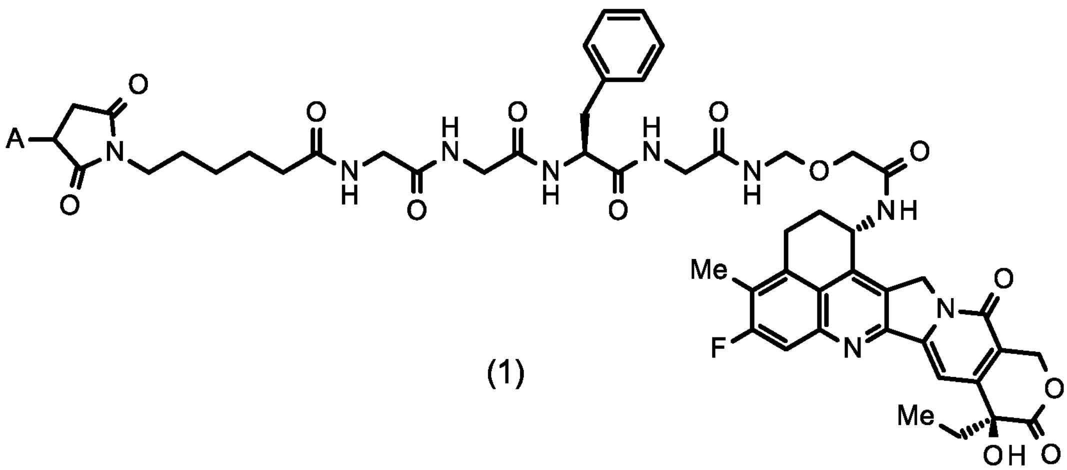

(1)

wherein A represents the connecting position to an antibody,
is conjugated to the antibody via a thioether bond, wherein the method comprises the steps of:

(i) reducing the antibody with tris(2-carboxyethyl)phosphine hydrochloride;
(ii) reacting a compound represented by formula (2)

[Chem. 6]

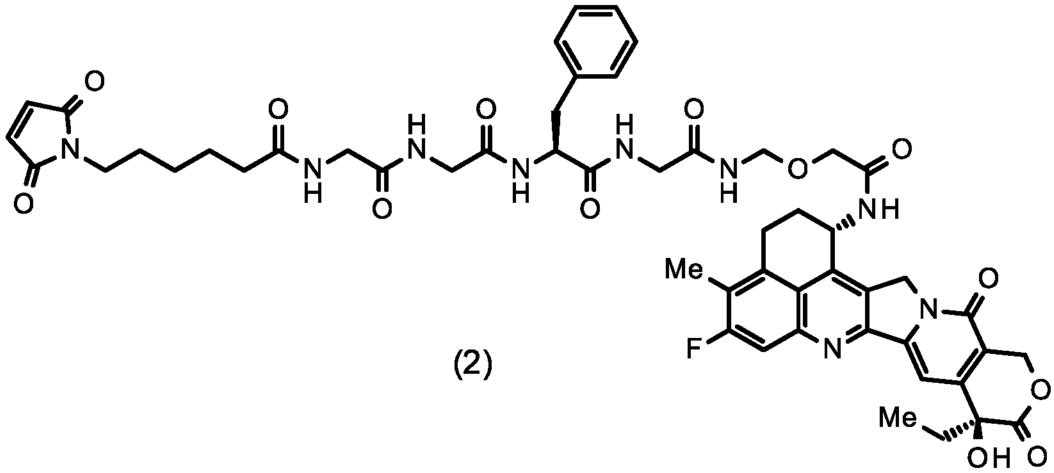

(2)

with the antibody reduced in step (i);

(iii) adding N-acetylcysteine to react with the residual compound represented by formula (2) in step (ii); and then

(iv) removing a compound represented by formula (3)

[Chem. 7]

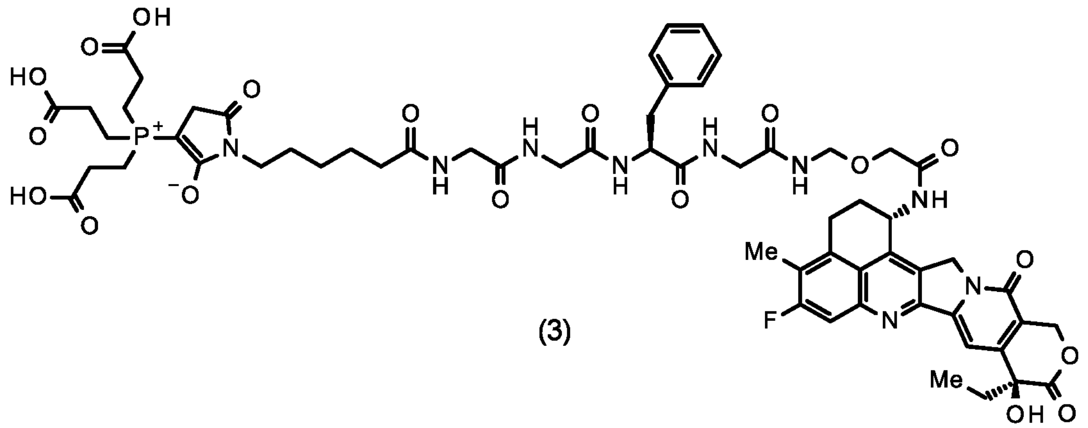

(3)

and a compound represented by formula (4)

[Chem. 8]

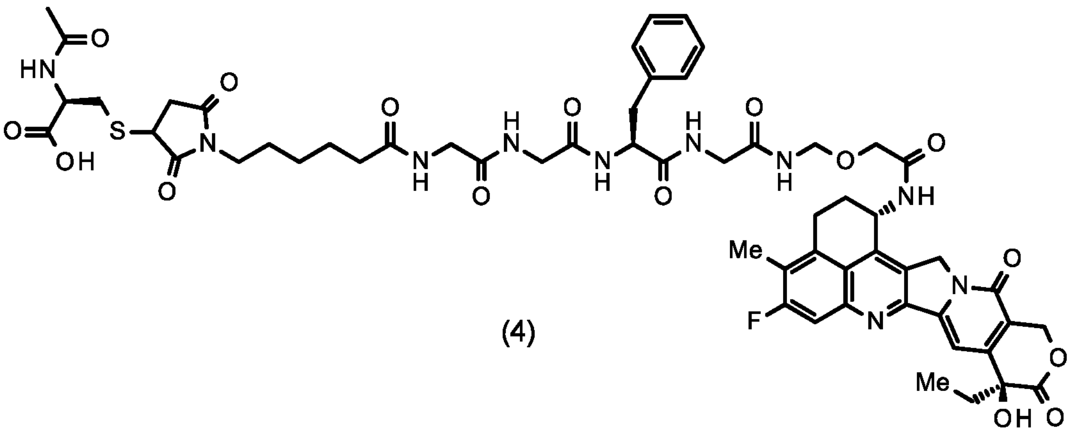

(4)

through ultrafiltration using a histidine buffer solution containing sodium chloride.

[56] The production method according to [55], wherein step (i) is performed in a buffer solution.

[57] The production method according to [56], wherein the pH of the buffer solution is adjusted to 6 to 8 by using an aqueous solution of disodium hydrogen phosphate.

[58] The production method according to [56] or [57], wherein the buffer solution is an acetate buffer solution.

[59] The production method according to any one of [55] to [58], wherein step (i) is performed in the presence of a chelating agent.

[60] The production method according to [59], wherein the chelating agent is ethylenediaminetetraacetic acid.

[61] The production method according to any one of [56] to [60], wherein the buffer solution used in step (i) contains a surfactant.

[62] The production method according to [61], wherein the surfactant is polysorbate 20.

[63] The production method according to [61], wherein the surfactant is polysorbate 80.

[64] The production method according to any one of [55] to [63], wherein the pH of the buffer solution used in step (iv) is about 5.

[65] The production method according to any one of [55] to [63], wherein the pH of the buffer solution used in step (iv) is in the range of from 4.7 to 5.3.

[66] The production method according to any one of [55] to [63], wherein the pH of the buffer solution used in step (iv) is in the range of from 4.8 to 5.2.

[67] The production method according to any one of [55] to [63], wherein the pH of the buffer solution used in step (iv) is in the range of from 4.9 to 5.1.

[68] The production method according to any one of [55] to [63], wherein the pH of the buffer solution used in step (iv) is 5.0.

[69] The production method according to any one of [55] to [68], wherein the concentration of sodium chloride used in step (iv) is in the range of from 0.2 wt% to 1 wt% with respect to the buffer solution used in step (iv).

[70] The production method according to any one of [55] to [68], wherein the concentration of sodium chloride used in step (iv) is about 0.5 wt% with respect to the buffer solution used in step (iv).

[71] The production method according to any one of [55] to [68], wherein the concentration of sodium chloride used in step (iv) is in the range of from 0.4 wt% to 0.6 wt% with respect to the buffer solution used in step (iv).

[72] The production method according to any one of [55] to [68], wherein the concentration of sodium chloride used in step (iv) is 0.5 wt% with respect to the buffer solution used in step (iv).

[73] The production method according to any one of [55] to [72], comprising a step subsequent to step (iv) of

(v) removing sodium chloride through ultrafiltration using a histidine buffer solution.

[74] The production method according to [73], wherein the pH of the buffer solution used in step (v) is in the range of from 4 to 6.

[75] The production method according to [73], wherein the pH of the buffer solution used in step (v) is about 5.

[76] The production method according to [73], wherein the pH of the buffer solution used in step (v) is in the range of from 4.7 to 5.3.

[77] The production method according to [73], wherein the pH of the buffer solution used in step (v) is in the range of from 4.8 to 5.2.

[78] The production method according to [73], wherein the pH of the buffer solution used in step (v) is in the range

of from 4.9 to 5.1.
[79] The production method according to [73], wherein the pH of the buffer solution used in step (v) is 5.0.
[80] The production method according to any one of [55] to [79], wherein the average number of units of the drug-linker conjugated per antibody molecule in the antibody-drug conjugate is in the range of from 7 to 8.
[81] The production method according to any one of [55] to [79], wherein the average number of units of the drug-linker conjugated per antibody molecule in the antibody-drug conjugate is in the range of from 7.5 to 8.
[82] The production method according to any one of [55] to [81], wherein the antibody is an anti-HER2 antibody.
[83] The production method according to [82], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[84] The production method according to [82], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[85] The production method according to any one of [55] to [81], wherein the antibody is an anti-HER3 antibody.
[86] The production method according to [85], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[87] The production method according to [86], wherein the anti-HER3 antibody lacks a lysine residue at the carboxyl terminus of the heavy chain.
[88] The production method according to any one of [55] to [81], wherein the antibody is an anti-GPR20 antibody.
[89] The production method according to [88], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.
[90] The production method according to [89], wherein the anti-GPR20 antibody lacks a lysine residue at the carboxyl terminus of the heavy chain.
[91] The production method according to any one of [55] to [81], wherein the antibody is an anti-CDH6 antibody.
[92] The production method according to [91], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[93] The production method according to [92], wherein the anti-CDH6 antibody lacks a lysine residue at the carboxyl terminus of the heavy chain.
[94] The production method according to any one of [55] to [93], comprising no purification step involving chromatography.
[95] The production method according to [94], wherein the chromatography is at least one selected from the group consisting of gel filtration chromatography, ion exchange chromatography, hydrophobic chromatography, and affinity chromatography.
[96] A method for producing a pharmaceutical composition containing an antibody-drug conjugate, a buffer solution, and an excipient, by producing the antibody-drug conjugate by the production method according to any one of [55] to [95], and then
performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[97] The production method according to [96], wherein the buffer solution is a histidine buffer solution.
[98] The production method according to [96] or [97], wherein the excipient is sucrose.
[99] The production method according to [96] or [97], wherein the excipient is trehalose.
[100] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [96] to [99], and then
performing the step of
(x) adding the surfactant to the pharmaceutical composition.
[101] The production method according to [100], wherein the surfactant is polysorbate 80.
[102] The production method according to [100], wherein the surfactant is polysorbate 20.
[103] A method for producing an antibody-drug conjugate, in which

a drug-linker represented by formula (1)

[Chem. 9]

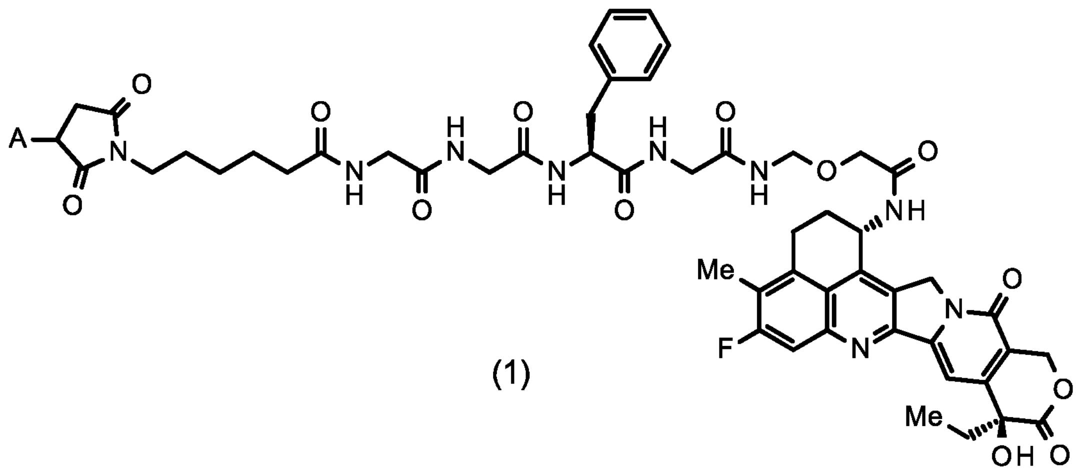

(1)

wherein A represents the connecting position to an antibody,
is conjugated to the antibody via a thioether bond, by obtaining a solution containing an unpurified product or crude product of the antibody-drug conjugate through the steps of:

(i) reducing the antibody with a reducing agent;
(ii) reacting a compound represented by formula (2)

[Chem. 10]

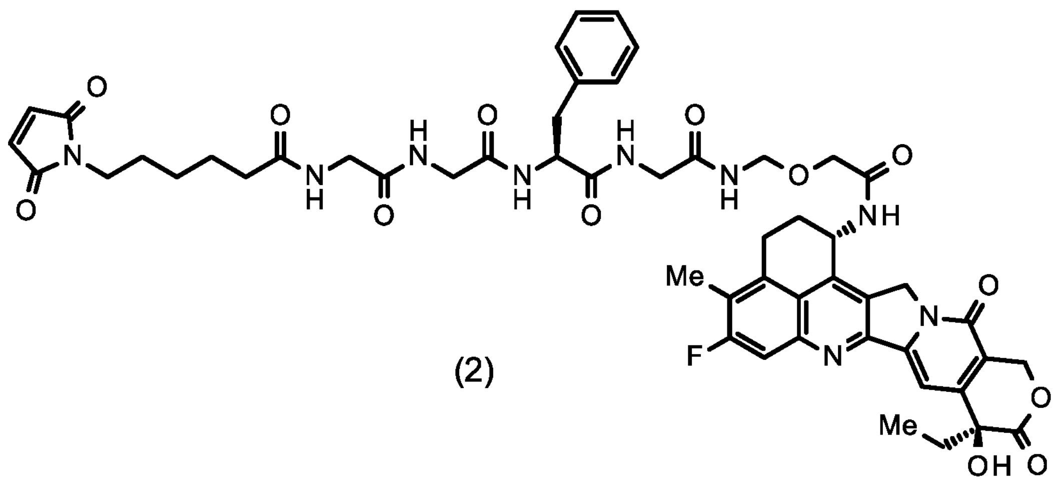

(2)

with the antibody reduced in step (i); and then
(iii) adding a reagent having a thiol group, and purifying the solution containing an unpurified product or crude product of the antibody-drug conjugate through the step of
(iv) removing a compound in which the reducing agent used in step (i) is added to the maleimidyl group of the compound represented by formula (2), and a compound in which the reagent having a thiol group used in step (iii) is added to the maleimidyl group of the compound represented by formula (2), through ultrafiltration using a buffer solution containing a salt consisting of a strong acid and a strong base.

[104] The production method according to [103], wherein the reducing agent used in step (i) is tris(2-carboxyethyl)phosphine or a salt thereof.
[105] The production method according to [103], wherein the reducing agent used in step (i) is tris(2-carboxyethyl)phosphine hydrochloride.

[106] The production method according to any one of [103] to [105], wherein step (i) is performed in a buffer solution.

[107] The production method according to [106], wherein the pH of the buffer solution is adjusted to 6 to 8 by using an aqueous solution of disodium hydrogen phosphate.

[108] The production method according to [106] or [107], wherein the buffer solution is an acetate buffer solution.

[109] The production method according to any one of [103] to [108], wherein step (i) is performed in the presence of a chelating agent.

[110] The production method according to [109], wherein the chelating agent is ethylenediaminetetraacetic acid.

[111] The production method according to any one of [106] to [110], wherein the buffer solution used in step (i) contains a surfactant.

[112] The production method according to [111], wherein the surfactant is polysorbate 20.

[113] The production method according to [111], wherein the surfactant is polysorbate 80.

[114] The production method according to any one of [103] to [113], wherein the reagent having a thiol group used in step (iii) is N-acetylcysteine.

[115] The production method according to any one of [103] to [114], wherein the pH of the buffer solution used in step (iv) is about 5.

[116] The production method according to any one of [103] to [114], wherein the pH of the buffer solution used in step (iv) is in the range of from 4.7 to 5.3.

[117] The production method according to any one of [103] to [114], wherein the pH of the buffer solution used in step (iv) is in the range of from 4.8 to 5.2.

[118] The production method according to any one of [103] to [114], wherein the pH of the buffer solution used in step (iv) is in the range of from 4.9 to 5.1.

[119] The production method according to any one of [103] to [114], wherein the pH of the buffer solution used in step (iv) is 5.0.

[120] The production method according to any one of [103] to [119], wherein the buffer solution used in step (iv) is a histidine buffer solution.

[121] The production method according to any one of [103] to [120], wherein the concentration of the salt consisting of a strong acid and a strong base used in step (iv) is in the range of from 0.2 wt% to 1 wt% with respect to the buffer solution used in step (iv).

[122] The production method according to any one of [103] to [120], wherein the concentration of the salt consisting of a strong acid and a strong base used in step (iv) is about 0.5 wt% with respect to the buffer solution used in step (iv).

[123] The production method according to any one of [103] to [120], wherein the concentration of the salt consisting of a strong acid and a strong base used in step (iv) is in the range of from 0.4 wt% to 0.6 wt% with respect to the buffer solution used in step (iv).

[124] The production method according to any one of [103] to [120], wherein the concentration of the salt consisting of a strong acid and a strong base used in step (iv) is 0.5 wt% with respect to the buffer solution used in step (iv).

[125] The production method according to any one of [103] to [124], wherein the salt consisting of a strong acid and a strong base used in step (iv) is sodium chloride.

[126] The production method according to any one of [103] to [125], comprising a step subsequent to step (iv) of (v) removing the salt consisting of a strong acid and a strong base through ultrafiltration using a buffer solution.

[127] The production method according to [126], wherein the pH of the buffer solution used in step (v) is in the range of from 4 to 6.

[128] The production method according to [126], wherein the pH of the buffer solution used in step (v) is about 5.

[129] The production method according to [126], wherein the pH of the buffer solution used in step (v) is in the range of from 4.7 to 5.3.

[130] The production method according to [126], wherein the pH of the buffer solution used in step (v) is in the range of from 4.8 to 5.2.

[131] The production method according to [126], wherein the pH of the buffer solution used in step (v) is in the range of from 4.9 to 5.1.

[132] The production method according to [126], wherein the pH of the buffer solution used in step (v) is 5.0.

[133] The production method according to any one of [126] to [132], wherein the buffer solution used in step (v) is a histidine buffer solution.

[134] The production method according to any one of [103] to [133], wherein the average number of units of the drug-linker conjugated per antibody molecule in the antibody-drug conjugate is in the range of from 7 to 8.

[135] The production method according to any one of [103] to [133], wherein the average number of units of the drug-linker conjugated per antibody molecule in the antibody-drug conjugate is in the range of from 7.5 to 8.

[136] The production method according to any one of [103] to [135], wherein the antibody is an anti-HER2 antibody.

[137] The production method according to [136], wherein the anti-HER2 antibody is an antibody comprising a heavy

chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[138] The production method according to [136], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[139] The production method according to any one of [103] to [135], wherein the antibody is an anti-HER3 antibody.
[140] The production method according to [139], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[141] The production method according to [140], wherein the anti-HER3 antibody lacks a lysine residue at the carboxyl terminus of the heavy chain.
[142] The production method according to any one of [103] to [135], wherein the antibody is an anti-GPR20 antibody.
[143] The production method according to [142], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.
[144] The production method according to [143], wherein the anti-GPR20 antibody lacks a lysine residue at the carboxyl terminus of the heavy chain.
[145] The production method according to any one of [103] to [135], wherein the antibody is an anti-CDH6 antibody.
[146] The production method according to [145], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[147] The production method according to [146], wherein the anti-CDH6 antibody lacks a lysine residue at the carboxyl terminus of the heavy chain.
[148] The production method according to any one of [103] to [147], comprising no purification step involving chromatography.
[149] The production method according to [148], wherein the chromatography is at least one selected from the group consisting of gel filtration chromatography, ion exchange chromatography, hydrophobic chromatography, and affinity chromatography.
[150] A method for producing a pharmaceutical composition containing an antibody-drug conjugate, a buffer solution, and an excipient, by producing the antibody-drug conjugate by the production method according to any one of [103] to [149], and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[151] The production method according to [150], wherein the buffer solution is a histidine buffer solution.
[152] The production method according to [150] or [151], wherein the excipient is sucrose.
[153] The production method according to [150] or [151], wherein the excipient is trehalose.
[154] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [150] to [153], and then performing the step of
(x) adding the surfactant to the pharmaceutical composition.
[155] The production method according to [154], wherein the surfactant is polysorbate 80.
[156] The production method according to [154], wherein the surfactant is polysorbate 20.
[157] A method for producing an antibody-drug conjugate, in which
a drug-linker represented by formula (1)

[Chem. 11]

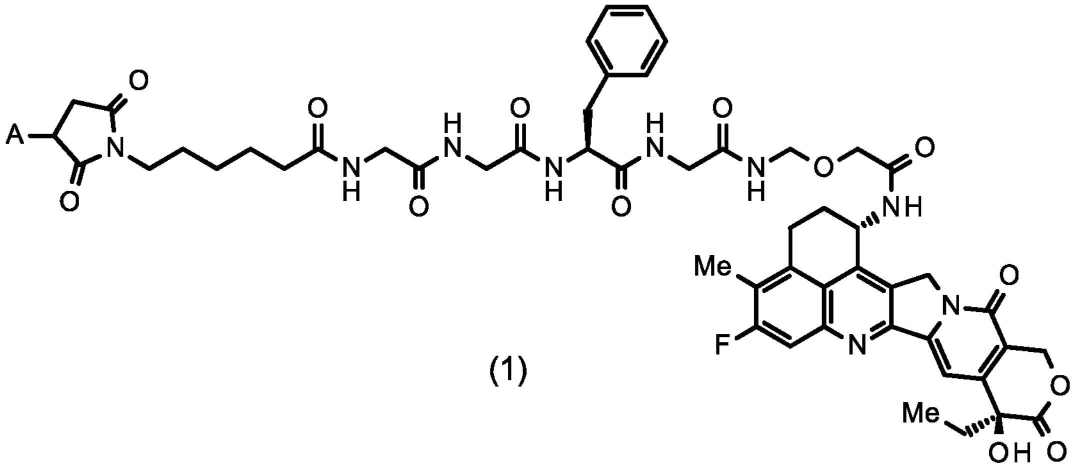

(1)

wherein A represents the connecting position to an antibody, is conjugated to the antibody via a thioether bond, by obtaining a solution containing an unpurified product or crude product of the antibody-drug conjugate through the steps of:

(i) reducing the antibody with tris(2-carboxyethyl)phosphine hydrochloride;
(ii) reacting a compound represented by formula (2)

[Chem. 12]

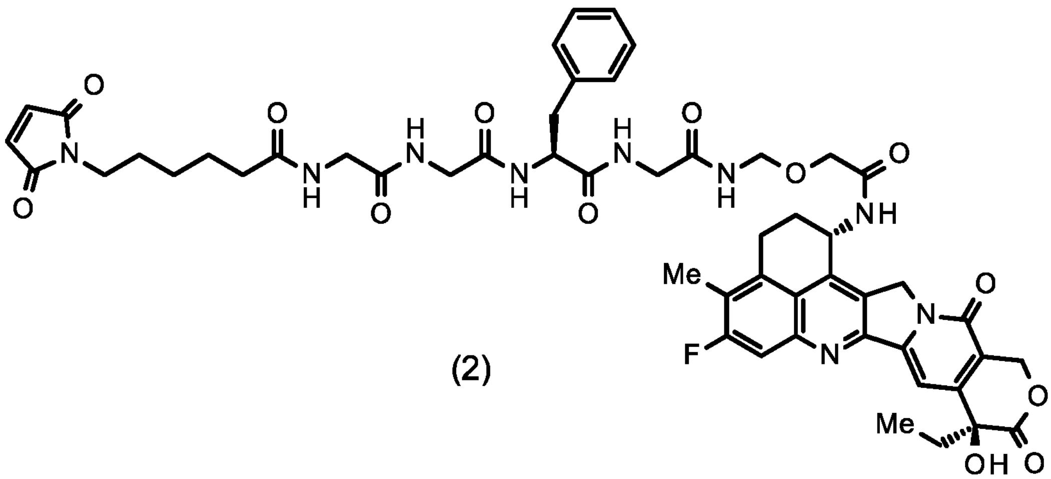

(2)

with the antibody reduced in step (i); and then
(iii) adding N-acetylcysteine, and
purifying the solution containing an unpurified product or crude product of the antibody-drug conjugate through the step of
(iv) removing a compound represented by formula (3)

[Chem. 13]

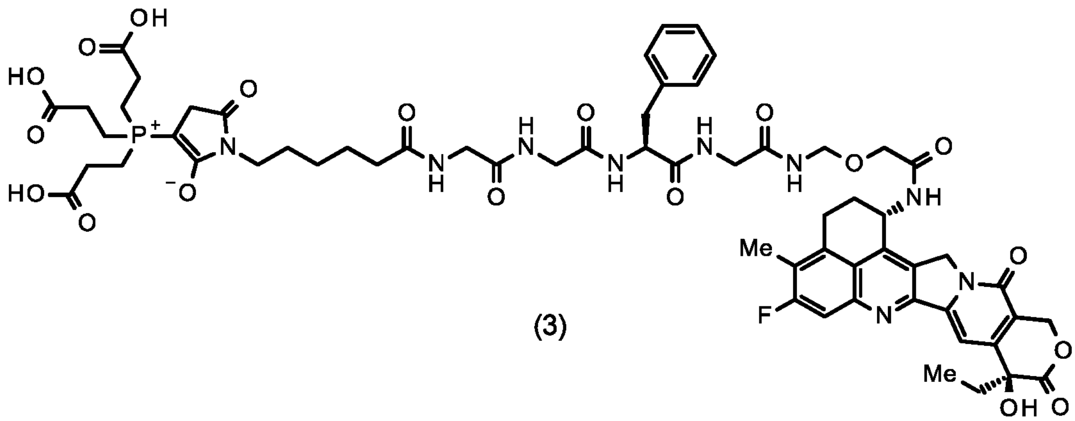

(3)

and a compound represented by formula (4)

[Chem. 14]

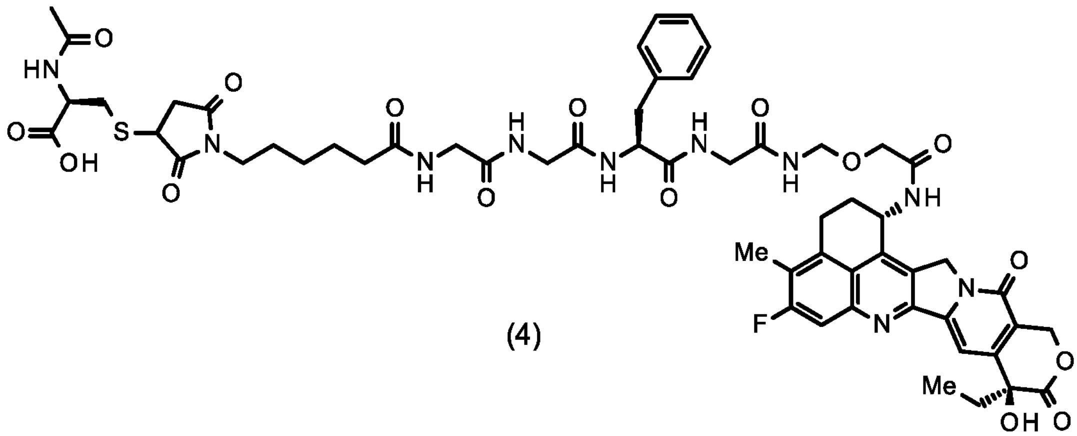

(4)

through ultrafiltration using a histidine buffer solution containing sodium chloride.

[158] The production method according to [157], wherein step (i) is performed in a buffer solution.

[159] The production method according to [158], wherein the pH of the buffer solution is adjusted to 6 to 8 by using an aqueous solution of disodium hydrogen phosphate.

[160] The production method according to [158] or [159], wherein the buffer solution is an acetate buffer solution.

[161] The production method according to any one of [157] to [160], wherein step (i) is performed in the presence of a chelating agent.

[162] The production method according to [161], wherein the chelating agent is ethylenediaminetetraacetic acid.

[163] The production method according to any one of [158] to [162], wherein the buffer solution used in step (i) contains a surfactant.

[164] The production method according to [163], wherein the surfactant is polysorbate 20.

[165] The production method according to [163], wherein the surfactant is polysorbate 80.

[166] The production method according to any one of [157] to [165], wherein the pH of the buffer solution used in step (iv) is about 5.

[167] The production method according to any one of [157] to [165], wherein the pH of the buffer solution used in step (iv) is in the range of from 4.7 to 5.3.

[168] The production method according to any one of [157] to [165], wherein the pH of the buffer solution used in step (iv) is in the range of from 4.8 to 5.2.

[169] The production method according to any one of [157] to [165], wherein the pH of the buffer solution used in step (iv) is in the range of from 4.9 to 5.1.

[170] The production method according to any one of [157] to [165], wherein the pH of the buffer solution used in

step (iv) is 5.0.
[171] The production method according to any one of [157] to [170], wherein the concentration of sodium chloride used in step (iv) is in the range of from 0.2 wt% to 1 wt% with respect to the buffer solution used in step (iv).
[172] The production method according to any one of [157] to [170], wherein the concentration of sodium chloride used in step (iv) is about 0.5 wt% with respect to the buffer solution used in step (iv).
[173] The production method according to any one of [157] to [170], wherein the concentration of sodium chloride used in step (iv) is in the range of from 0.4 wt% to 0.6 wt% with respect to the buffer solution used in step (iv).
[174] The production method according to any one of [157] to [170], wherein the concentration of sodium chloride used in step (iv) is 0.5 wt% with respect to the buffer solution used in step (iv).
[175] The production method according to any one of [157] to [174], comprising a step subsequent to step (iv) of (v) removing sodium chloride through ultrafiltration using a histidine buffer solution.
[176] The production method according to [175], wherein the pH of the buffer solution used in step (v) is in the range of from 4 to 6.
[177] The production method according to [175], wherein the pH of the buffer solution used in step (v) is about 5.
[178] The production method according to [175], wherein the pH of the buffer solution used in step (v) is in the range of from 4.7 to 5.3.
[179] The production method according to [175], wherein the pH of the buffer solution used in step (v) is in the range of from 4.8 to 5.2.
[180] The production method according to [175], wherein the pH of the buffer solution used in step (v) is in the range of from 4.9 to 5.1.
[181] The production method according to [175], wherein the pH of the buffer solution used in step (v) is 5.0.
[182] The production method according to any one of [157] to [181], wherein the average number of units of the drug-linker conjugated per antibody molecule in the antibody-drug conjugate is in the range of from 7 to 8.
[183] The production method according to any one of [157] to [181], wherein the average number of units of the drug-linker conjugated per antibody molecule in the antibody-drug conjugate is in the range of from 7.5 to 8.
[184] The production method according to any one of [157] to [183], wherein the antibody is an anti-HER2 antibody.
[185] The production method according to [184], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[186] The production method according to [184], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[187] The production method according to any one of [157] to [183], wherein the antibody is an anti-HER3 antibody.
[188] The production method according to [187], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[189] The production method according to [188], wherein the anti-HER3 antibody lacks a lysine residue at the carboxyl terminus of the heavy chain.
[190] The production method according to any one of [157] to [183], wherein the antibody is an anti-GPR20 antibody.
[191] The production method according to [190], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.
[192] The production method according to [191], wherein the anti-GPR20 antibody lacks a lysine residue at the carboxyl terminus of the heavy chain.
[193] The production method according to any one of [157] to [183], wherein the antibody is an anti-CDH6 antibody.
[194] The production method according to [193], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[195] The production method according to [194], wherein the anti-CDH6 antibody lacks a lysine residue at the carboxyl terminus of the heavy chain.
[196] The production method according to any one of [157] to [195], comprising no purification step involving chromatography.
[197] The production method according to [196], wherein the chromatography is at least one selected from the group consisting of gel filtration chromatography, ion exchange chromatography, hydrophobic chromatography, and affinity chromatography.
[198] A method for producing a pharmaceutical composition containing an antibody-drug conjugate, a buffer solution, and an excipient, by producing the antibody-drug conjugate by the production method according to any one of [157] to [197], and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[199] The production method according to [198], wherein the buffer solution is a histidine buffer solution.
[200] The production method according to [198] or [199], wherein the excipient is sucrose.
[201] The production method according to [198] or [199], wherein the excipient is trehalose.
[202] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [198] to [201], and then performing the step of
(x) adding the surfactant to the pharmaceutical composition.
[203] The production method according to [202], wherein the surfactant is polysorbate 80.
[204] The production method according to [202], wherein the surfactant is polysorbate 20.
[205] A method for producing an antibody-drug conjugate represented by formula (6)

[Chem. 15]

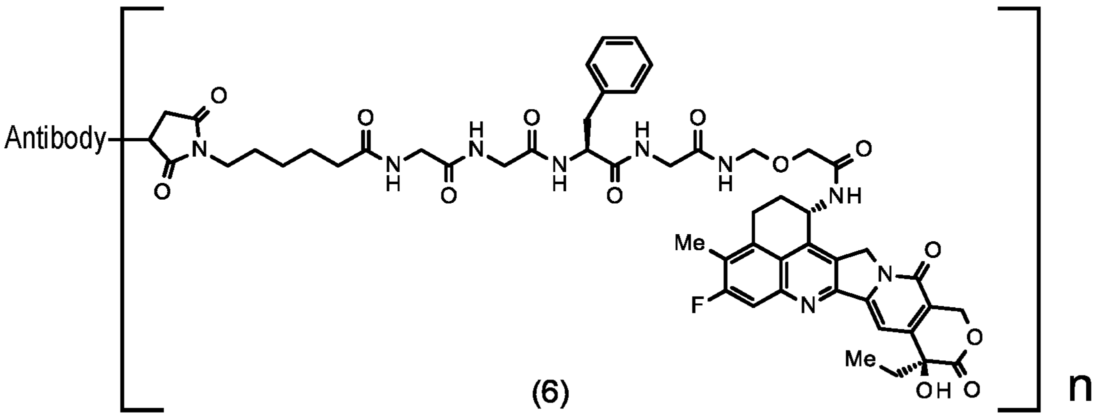

(6)

wherein a drug-linker is conjugated to the antibody via a thioether bond, and n represents the average number of units of the drug-linker conjugated per antibody molecule, wherein the method comprises the steps of:

(i) reducing the antibody with a reducing agent;
(ii) reacting a compound represented by formula (2)

[Chem. 16]

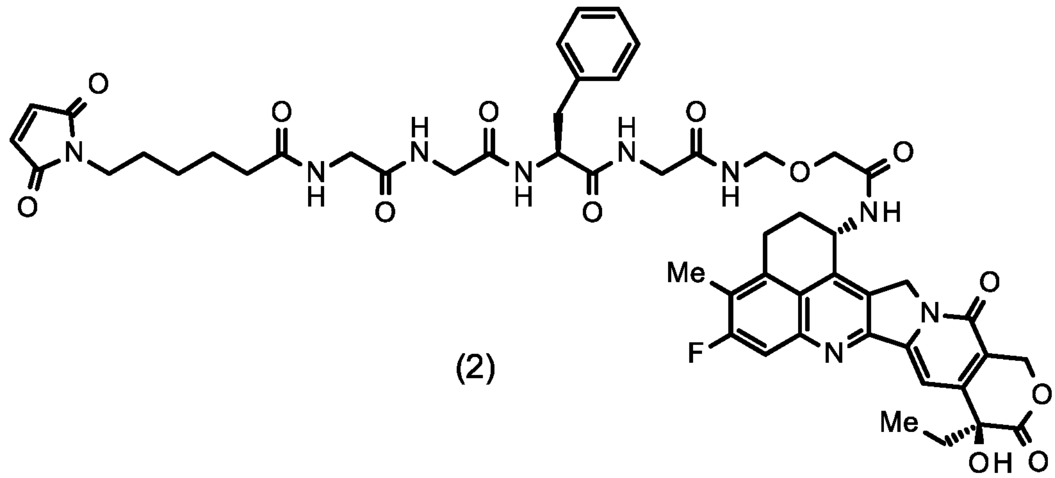

(2)

with the antibody reduced in step (i);
(iii) adding a reagent having a thiol group to react with the residual compound represented by formula (2) in step (ii); and then
(iv) removing a compound in which the reducing agent used in step (i) is added to the maleimidyl group of the compound represented by formula (2), and a compound in which the reagent having a thiol group used in step (iii) is added to the maleimidyl group of the compound represented by formula (2), through ultrafiltration using a buffer solution containing a salt consisting of a strong acid and a strong base.

[206] The production method according to [205], wherein the reducing agent used in step (i) is tris(2-carboxyethyl)phosphine or a salt thereof.
[207] The production method according to [205], wherein the reducing agent used in step (i) is tris(2-carboxyethyl)phosphine hydrochloride.
[208] The production method according to any one of [205] to [207], wherein step (i) is performed in a buffer solution.
[209] The production method according to [208], wherein the pH of the buffer solution is adjusted to 6 to 8 by using an aqueous solution of disodium hydrogen phosphate.
[210] The production method according to [208] or [209], wherein the buffer solution is an acetate buffer solution.
[211] The production method according to any one of [205] to [210], wherein step (i) is performed in the presence of a chelating agent.
[212] The production method according to [211], wherein the chelating agent is ethylenediaminetetraacetic acid.
[213] The production method according to any one of [208] to [212], wherein the buffer solution used in step (i) contains a surfactant.
[214] The production method according to [213], wherein the surfactant is polysorbate 20.
[215] The production method according to [213], wherein the surfactant is polysorbate 80.
[216] The production method according to any one of [205] to [215], wherein the reagent having a thiol group used in step (iii) is N-acetylcysteine.
[217] The production method according to any one of [205] to [216], wherein the pH of the buffer solution used in step (iv) is about 5.
[218] The production method according to any one of [205] to [216], wherein the pH of the buffer solution used in step (iv) is in the range of from 4.7 to 5.3.
[219] The production method according to any one of [205] to [216], wherein the pH of the buffer solution used in step (iv) is in the range of from 4.8 to 5.2.
[220] The production method according to any one of [205] to [216], wherein the pH of the buffer solution used in step (iv) is in the range of from 4.9 to 5.1.
[221] The production method according to any one of [205] to [216], wherein the pH of the buffer solution used in step (iv) is 5.0.
[222] The production method according to any one of [205] to [221], wherein the buffer solution used in step (iv) is a histidine buffer solution.
[223] The production method according to any one of [205] to [222], wherein the concentration of the salt consisting of a strong acid and a strong base used in step (iv) is in the range of from 0.2 wt% to 1 wt% with respect to the

buffer solution used in step (iv).
[224] The production method according to any one of [205] to [222], wherein the concentration of the salt consisting of a strong acid and a strong base used in step (iv) is about 0.5 wt% with respect to the buffer solution used in step (iv).
[225] The production method according to any one of [205] to [222], wherein the concentration of the salt consisting of a strong acid and a strong base used in step (iv) is in the range of from 0.4 wt% to 0.6 wt% with respect to the buffer solution used in step (iv).
[226] The production method according to any one of [205] to [222], wherein the concentration of the salt consisting of a strong acid and a strong base used in step (iv) is 0.5 wt% with respect to the buffer solution used in step (iv).
[227] The production method according to any one of [205] to [226], wherein the salt consisting of a strong acid and a strong base used in step (iv) is sodium chloride.
[228] The production method according to any one of [205] to [227], comprising a step subsequent to step (iv) of (v) removing the salt consisting of a strong acid and a strong base through ultrafiltration using a buffer solution.
[229] The production method according to [228], wherein the pH of the buffer solution used in step (v) is in the range of from 4 to 6.
[230] The production method according to [228], wherein the pH of the buffer solution used in step (v) is about 5.
[231] The production method according to [228], wherein the pH of the buffer solution used in step (v) is in the range of from 4.7 to 5.3.
[232] The production method according to [228], wherein the pH of the buffer solution used in step (v) is in the range of from 4.8 to 5.2.
[233] The production method according to [228], wherein the pH of the buffer solution used in step (v) is in the range of from 4.9 to 5.1.
[234] The production method according to [228], wherein the pH of the buffer solution used in step (v) is 5.0.
[235] The production method according to any one of [228] to [234], wherein the buffer solution used in step (v) is a histidine buffer solution.
[236] The production method according to any one of [205] to [235], wherein the average number of units of the drug-linker conjugated per antibody molecule in the antibody-drug conjugate is in the range of from 7 to 8.
[237] The production method according to any one of [205] to [235], wherein the average number of units of the drug-linker conjugated per antibody molecule in the antibody-drug conjugate is in the range of from 7.5 to 8.
[238] The production method according to any one of [205] to [237], wherein the antibody is an anti-HER2 antibody.
[239] The production method according to [238], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[240] The production method according to [238], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[241] The production method according to any one of [205] to [237], wherein the antibody is an anti-HER3 antibody.
[242] The production method according to [241], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[243] The production method according to [242], wherein the anti-HER3 antibody lacks a lysine residue at the carboxyl terminus of the heavy chain.
[244] The production method according to any one of [205] to [237], wherein the antibody is an anti-GPR20 antibody.
[245] The production method according to [244], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.
[246] The production method according to [245], wherein the anti-GPR20 antibody lacks a lysine residue at the carboxyl terminus of the heavy chain.
[247] The production method according to any one of [205] to [237], wherein the antibody is an anti-CDH6 antibody.
[248] The production method according to [247], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[249] The production method according to [248], wherein the anti-CDH6 antibody lacks a lysine residue at the carboxyl terminus of the heavy chain.
[250] The production method according to any one of [205] to [249], comprising no purification step involving chromatography.
[251] The production method according to [250], wherein the chromatography is at least one selected from the group consisting of gel filtration chromatography, ion exchange chromatography, hydrophobic chromatography, and affinity chromatography.

[252] A method for producing a pharmaceutical composition containing an antibody-drug conjugate, a buffer solution, and an excipient, by producing the antibody-drug conjugate by the production method according to any one of [205] to [251], and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[253] The production method according to [252], wherein the buffer solution is a histidine buffer solution.
[254] The production method according to [251] or [252], wherein the excipient is sucrose.
[255] The production method according to [251] or [252], wherein the excipient is trehalose.
[256] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [252] to [255], and then performing the step of
(x) adding the surfactant to the pharmaceutical composition.
[257] The production method according to [256], wherein the surfactant is polysorbate 80.
[258] The production method according to [256], wherein the surfactant is polysorbate 20.
[259] A method for producing an antibody-drug conjugate represented by formula (6)

[Chem. 17]

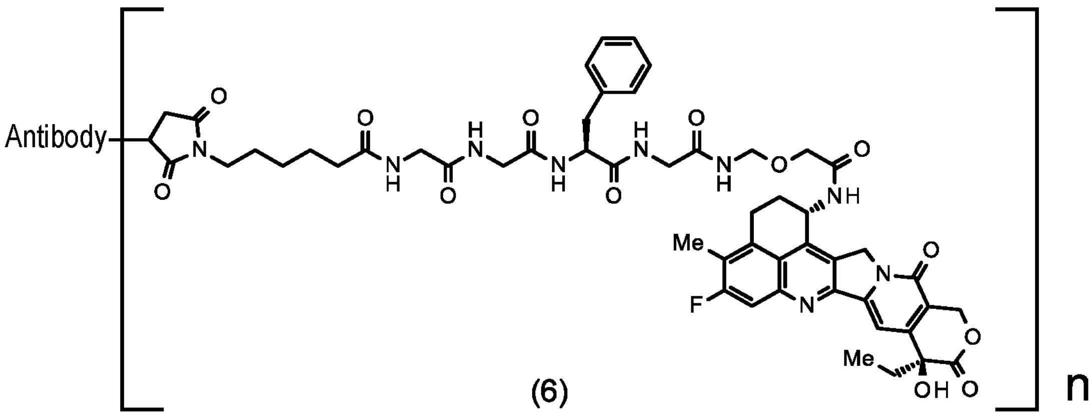

(6)

wherein a drug-linker is conjugated to the antibody via a thioether bond, and n represents the average number of units of the drug-linker conjugated per antibody molecule, wherein the method comprises the steps of:

(i) reducing the antibody with tris(2-carboxyethyl)phosphine hydrochloride;
(ii) reacting a compound represented by formula (2)

[Chem. 18]

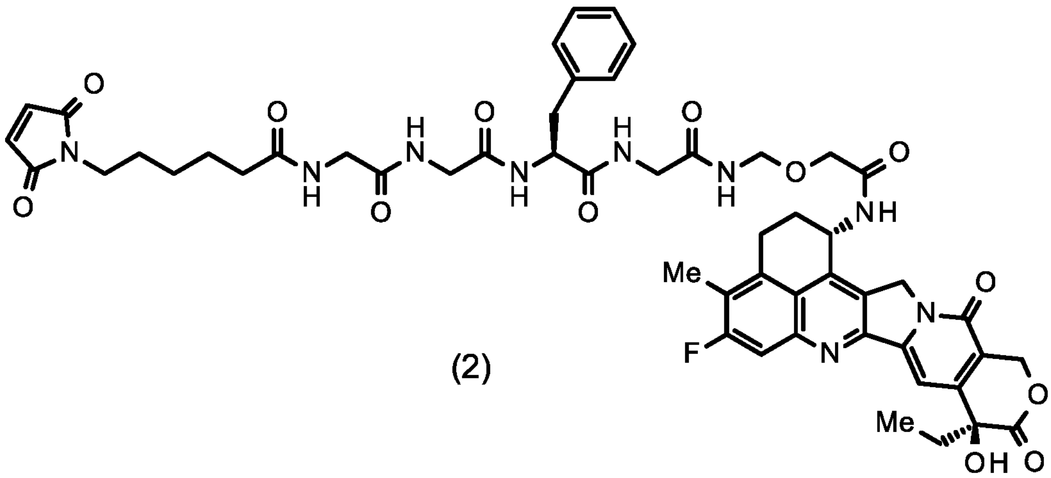

(2)

with the antibody reduced in step (i);
(iii) adding N-acetylcysteine to react with the residual compound represented by formula (2) in step (ii); and then
(iv) removing a compound represented by formula (3)

[Chem. 19]

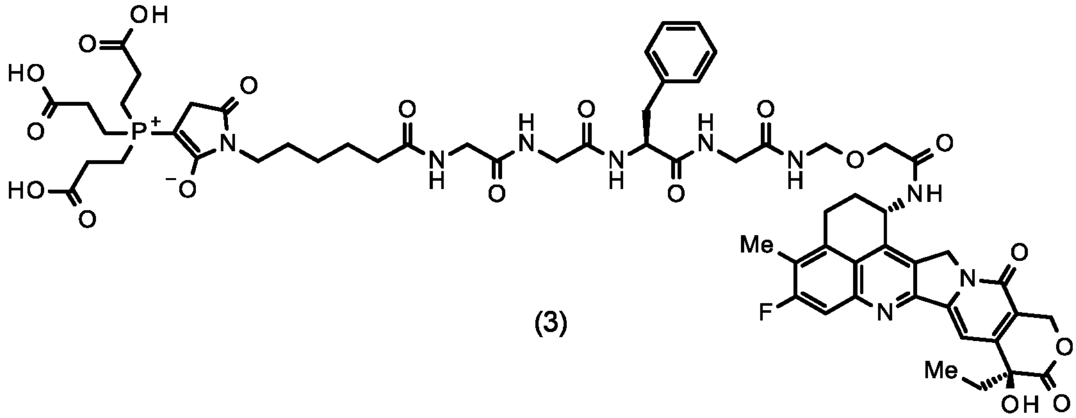

(3)

and a compound represented by formula (4)

[Chem. 20]

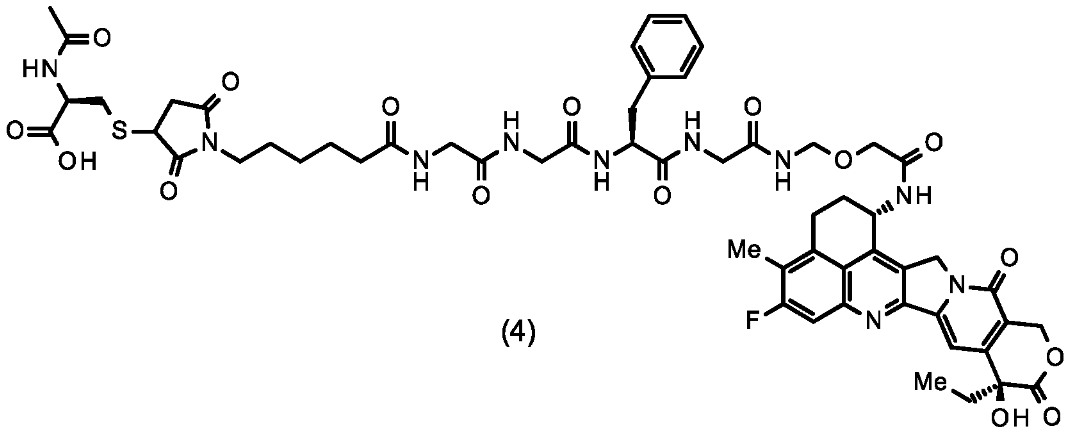

(4)

through ultrafiltration using a histidine buffer solution containing sodium chloride.

[260] The production method according to [259], wherein step (i) is performed in a buffer solution.

[261] The production method according to [260], wherein the pH of the buffer solution is adjusted to 6 to 8 by using an aqueous solution of disodium hydrogen phosphate.

[262] The production method according to [260] or [261], wherein the buffer solution is an acetate buffer solution.

[263] The production method according to any one of [259] to [262], wherein step (i) is performed in the presence of a chelating agent.

[264] The production method according to [263], wherein the chelating agent is ethylenediaminetetraacetic acid.

[265] The production method according to any one of [260] to [264], wherein the buffer solution used in step (i) contains a surfactant.

[266] The production method according to [265], wherein the surfactant is polysorbate 20.

[267] The production method according to [265], wherein the surfactant is polysorbate 80.

[268] The production method according to any one of [259] to [267], wherein the pH of the buffer solution used in step (iv) is about 5.

[269] The production method according to any one of [259] to [267], wherein the pH of the buffer solution used in step (iv) is in the range of from 4.7 to 5.3.

[270] The production method according to any one of [259] to [267], wherein the pH of the buffer solution used in step (iv) is in the range of from 4.8 to 5.2.

[271] The production method according to any one of [259] to [267], wherein the pH of the buffer solution used in step (iv) is in the range of from 4.9 to 5.1.

[272] The production method according to any one of [259] to [267], wherein the pH of the buffer solution used in step (iv) is 5.0.

[273] The production method according to any one of [259] to [272], wherein the concentration of sodium chloride used in step (iv) is in the range of from 0.2 wt% to 1 wt% with respect to the buffer solution used in step (iv).

[274] The production method according to any one of [259] to [272], wherein the concentration of sodium chloride used in step (iv) is about 0.5 wt% with respect to the buffer solution used in step (iv).

[275] The production method according to any one of [259] to [272], wherein the concentration of sodium chloride used in step (iv) is in the range of from 0.4 wt% to 0.6 wt% with respect to the buffer solution used in step (iv).

[276] The production method according to any one of [259] to [272], wherein the concentration of sodium chloride used in step (iv) is 0.5 wt% with respect to the buffer solution used in step (iv).

[277] The production method according to any one of [259] to [276], comprising a step subsequent to step (iv) of (v) removing sodium chloride through ultrafiltration using a histidine buffer solution.

[278] The production method according to [277], wherein the pH of the buffer solution used in step (v) is in the range of from 4 to 6.

[279] The production method according to [277], wherein the pH of the buffer solution used in step (v) is about 5.

[280] The production method according to [277], wherein the pH of the buffer solution used in step (v) is in the range of from 4.7 to 5.3.

[281] The production method according to [277], wherein the pH of the buffer solution used in step (v) is in the range of from 4.8 to 5.2.

[282] The production method according to [277], wherein the pH of the buffer solution used in step (v) is in the range

of from 4.9 to 5.1.

[283] The production method according to [277], wherein the pH of the buffer solution used in step (v) is 5.0.

[284] The production method according to any one of [259] to [283], wherein the average number of units of the drug-linker conjugated per antibody molecule in the antibody-drug conjugate is in the range of from 7 to 8.

[285] The production method according to any one of [259] to [283], wherein the average number of units of the drug-linker conjugated per antibody molecule in the antibody-drug conjugate is in the range of from 7.5 to 8.

[286] The production method according to any one of [259] to [285], wherein the antibody is an anti-HER2 antibody.

[287] The production method according to [286], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[288] The production method according to [286], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[289] The production method according to any one of [259] to [285], wherein the antibody is an anti-HER3 antibody.

[290] The production method according to [289], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[291] The production method according to [290], wherein the anti-HER3 antibody lacks a lysine residue at the carboxyl terminus of the heavy chain.

[292] The production method according to any one of [259] to [285], wherein the antibody is an anti-GPR20 antibody.

[293] The production method according to [292], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.

[294] The production method according to [293], wherein the anti-GPR20 antibody lacks a lysine residue at the carboxyl terminus of the heavy chain.

[295] The production method according to any one of [259] to [285], wherein the antibody is an anti-CDH6 antibody.

[296] The production method according to [295], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[297] The production method according to [296], wherein the anti-CDH6 antibody lacks a lysine residue at the carboxyl terminus of the heavy chain.

[298] The production method according to any one of [259] to [297], comprising no purification step involving chromatography.

[299] The production method according to [298], wherein the chromatography is at least one selected from the group consisting of gel filtration chromatography, ion exchange chromatography, hydrophobic chromatography, and affinity chromatography.

[300] A method for producing a pharmaceutical composition containing an antibody-drug conjugate, a buffer solution, and an excipient, by producing the antibody-drug conjugate by the production method according to any one of [259] to [299], and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[301] The production method according to [300], wherein the buffer solution is a histidine buffer solution.

[302] The production method according to [300] or [301], wherein the excipient is sucrose.

[303] The production method according to [300] or [301], wherein the excipient is trehalose.

[304] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [300] to [303], and then performing the step of

(x) adding the surfactant to the pharmaceutical composition.

[305] The production method according to [304], wherein the surfactant is polysorbate 80.

[306] The production method according to [304], wherein the surfactant is polysorbate 20.

[307] A method for producing an antibody-drug conjugate represented by formula (6)

[Chem. 21]

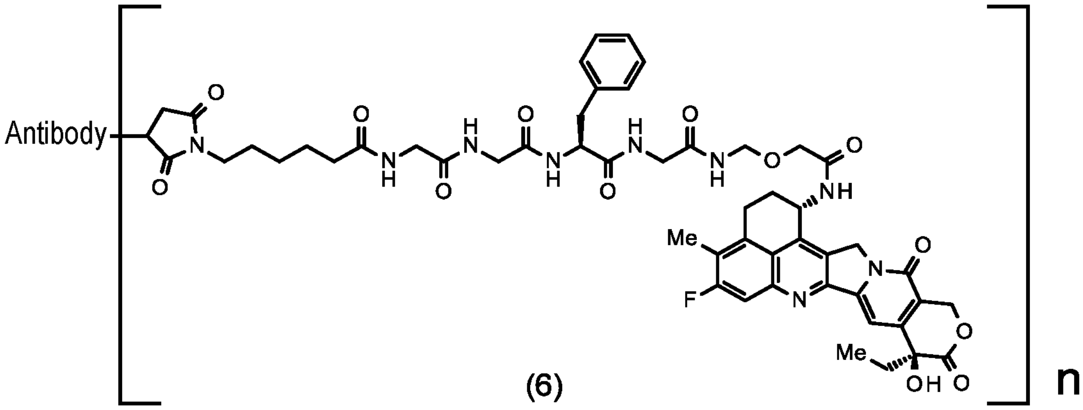

(6)

wherein a drug-linker is conjugated to the antibody via a thioether bond, and n represents the average number of units of the drug-linker conjugated per antibody molecule, wherein the method comprises the steps of:

(i) reducing the antibody with a reducing agent;
(ii) reacting a compound represented by formula (2)

[Chem. 22]

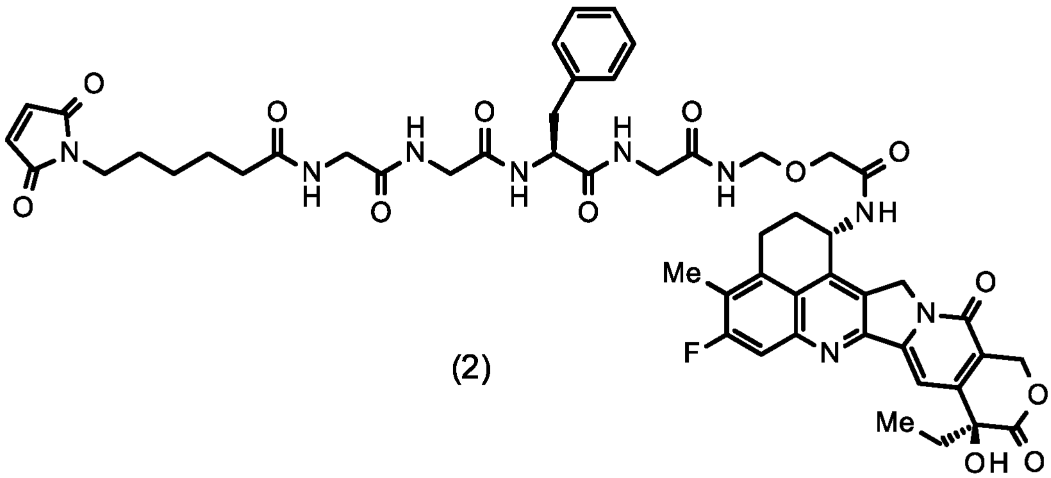

(2)

with the antibody reduced in step (i); and then
(iii) adding a reagent having a thiol group to obtain the solution containing an unpurified product or crude product of the antibody-drug conjugate, and purifying the solution containing an unpurified product or crude product of the antibody-drug conjugate through the step of
(iv) removing a compound in which the reducing agent used in step (i) is added to the maleimidyl group of the compound represented by formula (2), and a compound in which the reagent having a thiol group used in step (iii) is added to the maleimidyl group of the compound represented by formula (2), through ultrafiltration using a buffer solution containing a salt consisting of a strong acid and a strong base.

[308] The production method according to [307], wherein the reducing agent used in step (i) is tris(2-carboxyethyl)phosphine or a salt thereof.
[309] The production method according to [307], wherein the reducing agent used in step (i) is tris(2-carboxyethyl)phosphine hydrochloride.
[310] The production method according to any one of [307] to [309], wherein step (i) is performed in a buffer solution.
[311] The production method according to [310], wherein the pH of the buffer solution is adjusted to 6 to 8 by using an aqueous solution of disodium hydrogen phosphate.

[312] The production method according to [310] or [311], wherein the buffer solution is an acetate buffer solution.

[313] The production method according to any one of [307] to [312], wherein step (i) is performed in the presence of a chelating agent.

[314] The production method according to [313], wherein the chelating agent is ethylenediaminetetraacetic acid.

[315] The production method according to any one of [310] to [314], wherein the buffer solution used in step (i) contains a surfactant.

[316] The production method according to [315], wherein the surfactant is polysorbate 20.

[317] The production method according to [315], wherein the surfactant is polysorbate 80.

[318] The production method according to any one of [307] to [317], wherein the reagent having a thiol group used in step (iii) is N-acetylcysteine.

[319] The production method according to any one of [307] to [318], wherein the pH of the buffer solution used in step (iv) is about 5.

[320] The production method according to any one of [307] to [318], wherein the pH of the buffer solution used in step (iv) is in the range of from 4.7 to 5.3.

[321] The production method according to any one of [307] to [318], wherein the pH of the buffer solution used in step (iv) is in the range of from 4.8 to 5.2.

[322] The production method according to any one of [307] to [318], wherein the pH of the buffer solution used in step (iv) is in the range of from 4.9 to 5.1.

[323] The production method according to any one of [307] to [318], wherein the pH of the buffer solution used in step (iv) is 5.0.

[324] The production method according to any one of [307] to [323], wherein the buffer solution used in step (iv) is a histidine buffer solution.

[325] The production method according to any one of [307] to [324], wherein the concentration of the salt consisting of a strong acid and a strong base used in step (iv) is in the range of from 0.2 wt% to 1 wt% with respect to the buffer solution used in step (iv).

[326] The production method according to any one of [307] to [324], wherein the concentration of the salt consisting of a strong acid and a strong base used in step (iv) is about 0.5 wt% with respect to the buffer solution used in step (iv).

[327] The production method according to any one of [307] to [324], wherein the concentration of the salt consisting of a strong acid and a strong base used in step (iv) is in the range of from 0.4 wt% to 0.6 wt% with respect to the buffer solution used in step (iv).

[328] The production method according to any one of [307] to [324], wherein the concentration of the salt consisting of a strong acid and a strong base used in step (iv) is 0.5 wt% with respect to the buffer solution used in step (iv).

[329] The production method according to any one of [307] to [328], wherein the salt consisting of a strong acid and a strong base used in step (iv) is sodium chloride.

[330] The production method according to any one of [307] to [329], comprising a step subsequent to step (iv) of (v) removing the salt consisting of a strong acid and a strong base through ultrafiltration using a buffer solution.

[331] The production method according to [330], wherein the pH of the buffer solution used in step (v) is in the range of from 4 to 6.

[332] The production method according to [330], wherein the pH of the buffer solution used in step (v) is about 5.

[333] The production method according to [330], wherein the pH of the buffer solution used in step (v) is in the range of from 4.7 to 5.3.

[334] The production method according to [330], wherein the pH of the buffer solution used in step (v) is in the range of from 4.8 to 5.2.

[335] The production method according to [330], wherein the pH of the buffer solution used in step (v) is in the range of from 4.9 to 5.1.

[336] The production method according to [330], wherein the pH of the buffer solution used in step (v) is 5.0.

[337] The production method according to any one of [330] to [336], wherein the buffer solution used in step (v) is a histidine buffer solution.

[338] The production method according to any one of [307] to [337], wherein the average number of units of the drug-linker conjugated per antibody molecule in the antibody-drug conjugate is in the range of from 7 to 8.

[339] The production method according to any one of [307] to [337], wherein the average number of units of the drug-linker conjugated per antibody molecule in the antibody-drug conjugate is in the range of from 7.5 to 8.

[340] The production method according to any one of [307] to [339], wherein the antibody is an anti-HER2 antibody.

[341] The production method according to [340], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[342] The production method according to [340], wherein the anti-HER2 antibody is an antibody comprising a heavy chain consisting of an amino acid sequence represented by SEQ ID NO: 1 and a light chain consisting of an amino

acid sequence represented by SEQ ID NO: 2.
[343] The production method according to any one of [307] to [339], wherein the antibody is an anti-HER3 antibody.
[344] The production method according to [343], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[345] The production method according to [344], wherein the anti-HER3 antibody lacks a lysine residue at the carboxyl terminus of the heavy chain.
[346] The production method according to any one of [307] to [339], wherein the antibody is an anti-GPR20 antibody.
[347] The production method according to [346], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.
[348] The production method according to [347], wherein the anti-GPR20 antibody lacks a lysine residue at the carboxyl terminus of the heavy chain.
[349] The production method according to any one of [307] to [339], wherein the antibody is an anti-CDH6 antibody.
[350] The production method according to [349], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[351] The production method according to [350], wherein the anti-CDH6 antibody lacks a lysine residue at the carboxyl terminus of the heavy chain.
[352] The production method according to any one of [307] to [351], comprising no purification step involving chromatography.
[353] The production method according to [352], wherein the chromatography is at least one selected from the group consisting of gel filtration chromatography, ion exchange chromatography, hydrophobic chromatography, and affinity chromatography.
[354] A method for producing a pharmaceutical composition containing an antibody-drug conjugate, a buffer solution, and an excipient, by producing the antibody-drug conjugate by the production method according to any one of [307] to [353], and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[355] The production method according to [354], wherein the buffer solution is a histidine buffer solution.
[356] The production method according to [354] or [355], wherein the excipient is sucrose.
[357] The production method according to [354] or [355], wherein the excipient is trehalose.
[358] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [354] to [357], and then performing the step of
(x) adding the surfactant to the pharmaceutical composition.
[359] The production method according to [358], wherein the surfactant is polysorbate 80.
[360] The production method according to [358], wherein the surfactant is polysorbate 20.
[361] A method for producing an antibody-drug conjugate represented by formula (6)

[Chem. 23]

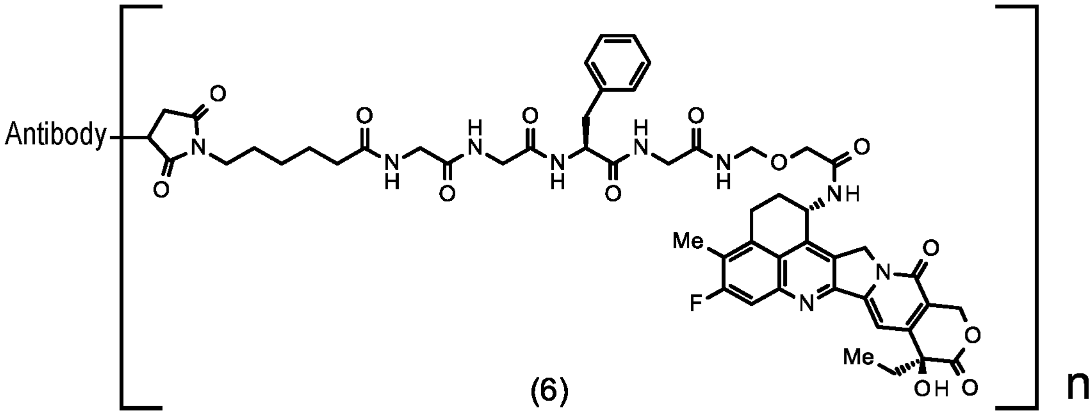

(6)

wherein a drug-linker is conjugated to the antibody via a thioether bond, and n represents the average number of units of the drug-linker conjugated per antibody molecule, wherein the method comprises the steps of:

(i) reducing the antibody with tris(2-carboxyethyl)phosphine hydrochloride;
(ii) reacting a compound represented by formula (2)

[Chem. 24]

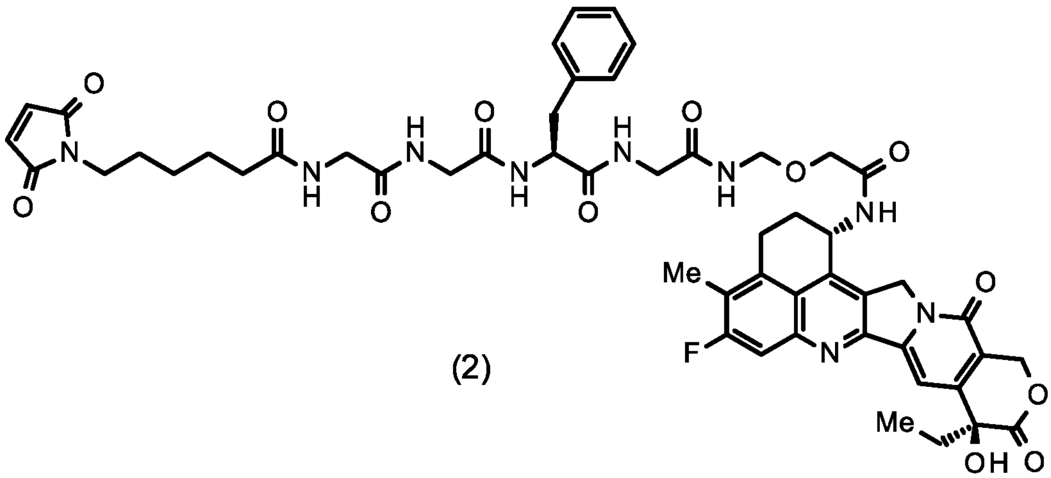

(2)

with the antibody reduced in step (i); and then
(iii) adding N-acetylcysteine to obtain the solution containing an unpurified product or crude product of the antibody-drug conjugate, and
purifying the solution containing an unpurified product or crude product of the antibody-drug conjugate through the step of
(iv) removing a compound represented by formula (3)

[Chem. 25]

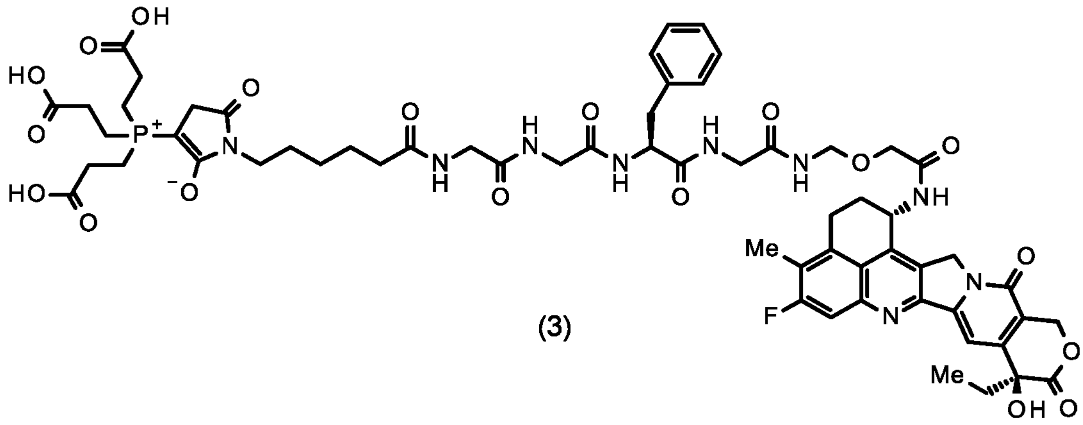

(3)

and a compound represented by formula (4)

[Chem. 26]

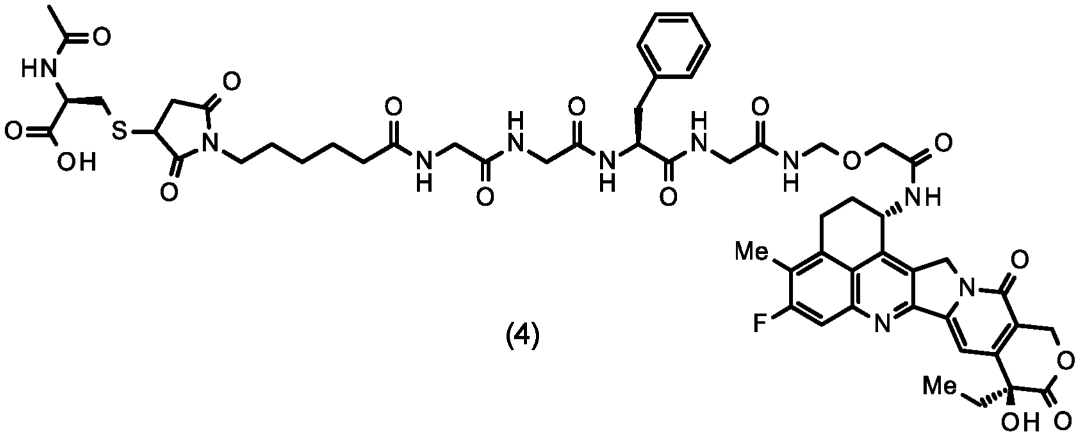

(4)

through ultrafiltration using a histidine buffer solution containing sodium chloride.

[362] The production method according to [361], wherein step (i) is performed in a buffer solution.

[363] The production method according to [362], wherein the pH of the buffer solution is adjusted to 6 to 8 by using an aqueous solution of disodium hydrogen phosphate.

[364] The production method according to [362] or [363], wherein the buffer solution is an acetate buffer solution.

[365] The production method according to any one of [361] to [364], wherein step (i) is performed in the presence of a chelating agent.

[366] The production method according to [365], wherein the chelating agent is ethylenediaminetetraacetic acid.

[367] The production method according to any one of [362] to [366], wherein the buffer solution used in step (i) contains a surfactant.

[368] The production method according to [367], wherein the surfactant is polysorbate 20.

[369] The production method according to [367], wherein the surfactant is polysorbate 80.

[370] The production method according to any one of [361] to [369], wherein the pH of the buffer solution used in step (iv) is about 5.

[371] The production method according to any one of [361] to [369], wherein the pH of the buffer solution used in step (iv) is in the range of from 4.7 to 5.3.

[372] The production method according to any one of [361] to [369], wherein the pH of the buffer solution used in step (iv) is in the range of from 4.8 to 5.2.

[373] The production method according to any one of [361] to [369], wherein the pH of the buffer solution used in step (iv) is in the range of from 4.9 to 5.1.

[374] The production method according to any one of [361] to [369], wherein the pH of the buffer solution used in

step (iv) is 5.0.
[375] The production method according to any one of [361] to [374], wherein the concentration of sodium chloride used in step (iv) is in the range of from 0.2 wt% to 1 wt% with respect to the buffer solution used in step (iv).
[376] The production method according to any one of [361] to [374], wherein the concentration of sodium chloride used in step (iv) is about 0.5 wt% with respect to the buffer solution used in step (iv).
[377] The production method according to any one of [361] to [374], wherein the concentration of sodium chloride used in step (iv) is in the range of from 0.4 wt% to 0.6 wt% with respect to the buffer solution used in step (iv).
[378] The production method according to any one of [361] to [374], wherein the concentration of sodium chloride used in step (iv) is 0.5 wt% with respect to the buffer solution used in step (iv).
[379] The production method according to any one of [361] to [378], comprising a step subsequent to step (iv) of (v) removing sodium chloride through ultrafiltration using a histidine buffer solution.
[380] The production method according to [379], wherein the pH of the buffer solution used in step (v) is in the range of from 4 to 6.
[381] The production method according to [379], wherein the pH of the buffer solution used in step (v) is about 5.
[382] The production method according to [379], wherein the pH of the buffer solution used in step (v) is in the range of from 4.7 to 5.3.
[383] The production method according to [379], wherein the pH of the buffer solution used in step (v) is in the range of from 4.8 to 5.2.
[384] The production method according to [379], wherein the pH of the buffer solution used in step (v) is in the range of from 4.9 to 5.1.
[385] The production method according to [379], wherein the pH of the buffer solution used in step (v) is 5.0.
[386] The production method according to any one of [361] to [385], wherein the average number of units of the drug-linker conjugated per antibody molecule in the antibody-drug conjugate is in the range of from 7 to 8.
[387] The production method according to any one of [361] to [385], wherein the average number of units of the drug-linker conjugated per antibody molecule in the antibody-drug conjugate is in the range of from 7.5 to 8.
[388] The production method according to any one of [361] to [387], wherein the antibody is an anti-HER2 antibody.
[389] The production method according to [388], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[390] The production method according to [388], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[391] The production method according to any one of [361] to [387], wherein the antibody is an anti-HER3 antibody.
[392] The production method according to [391], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[393] The production method according to [392], wherein the anti-HER3 antibody lacks a lysine residue at the carboxyl terminus of the heavy chain.
[394] The production method according to any one of [361] to [387], wherein the antibody is an anti-GPR20 antibody.
[395] The production method according to [394], wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.
[396] The production method according to [395], wherein the anti-GPR20 antibody lacks a lysine residue at the carboxyl terminus of the heavy chain.
[397] The production method according to any one of [361] to [387], wherein the antibody is an anti-CDH6 antibody.
[398] The production method according to [397], wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[399] The production method according to [398], wherein the anti-CDH6 antibody lacks a lysine residue at the carboxyl terminus of the heavy chain.
[400] The production method according to any one of [361] to [399], comprising no purification step involving chromatography.
[401] The production method according to [400], wherein the chromatography is at least one selected from the group consisting of gel filtration chromatography, ion exchange chromatography, hydrophobic chromatography, and affinity chromatography.
[402] A method for producing a pharmaceutical composition containing an antibody-drug conjugate, a buffer solution, and an excipient, by producing the antibody-drug conjugate by the production method according to any one of [361] to [401], and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

[403] The production method according to [402], wherein the buffer solution is a histidine buffer solution.
[404] The production method according to [401] or [402], wherein the excipient is sucrose.
[405] The production method according to [401] or [402], wherein the excipient is trehalose.
[406] A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of [402] to [405], and then performing the step of
(x) adding the surfactant to the pharmaceutical composition.
[407] The production method according to [406], wherein the surfactant is polysorbate 80.
[408] The production method according to [406], wherein the surfactant is polysorbate 20.
[409] An antibody-drug conjugate produced through the production method according to any one of [1] to [47], [55] to [95], [103] to [149], [157] to [197], [205] to [251], [259] to [299], [307] to [353], and [361] to [401].
[410] A pharmaceutical composition produced through the production method according to any one of [48] to [54], [96] to [102], [150] to [156], [198] to [204], [252] to [258], [300] to [306], [354] to [360], and [402] to [408].

Advantageous Effects of Invention

The present invention can provide a method for producing an antibody-drug conjugate, which comprises a purification step for effectively removing by-products derived from the compound represented by the formula (2) with the generation of aggregates suppressed. In addition, the present invention can provide an industrially excellent method for producing a pharmaceutical composition containing the antibody-drug conjugate.

Brief Description of Drawings

[Figure 1] Figure 1 is a diagram showing compound (3) and compound (4) content when ultrafiltration was performed with histidine buffer solution (pH 5.0) or 0.5% sodium chloride-containing histidine buffer solution (pH 5.0).
[Figure 2] Figure 2 is a diagram showing aggregate content when ultrafiltration was performed with histidine buffer solution (pH 5.0 or pH 5.8) or 0.4% sodium chloride-containing histidine buffer solution (pH 5.0 or pH 5.8).

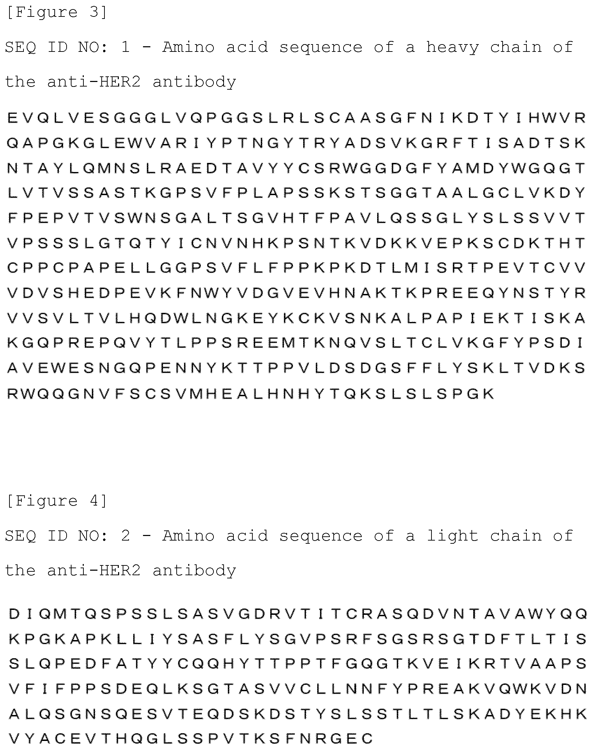

[Figure 3] Figure 3 is a diagram showing an amino acid sequence of a heavy chain of an anti-HER2 antibody (SEQ ID NO: 1).
[Figure 4] Figure 4 is a diagram showing an amino acid sequence of a light chain of an anti-HER2 antibody (SEQ ID NO: 2).

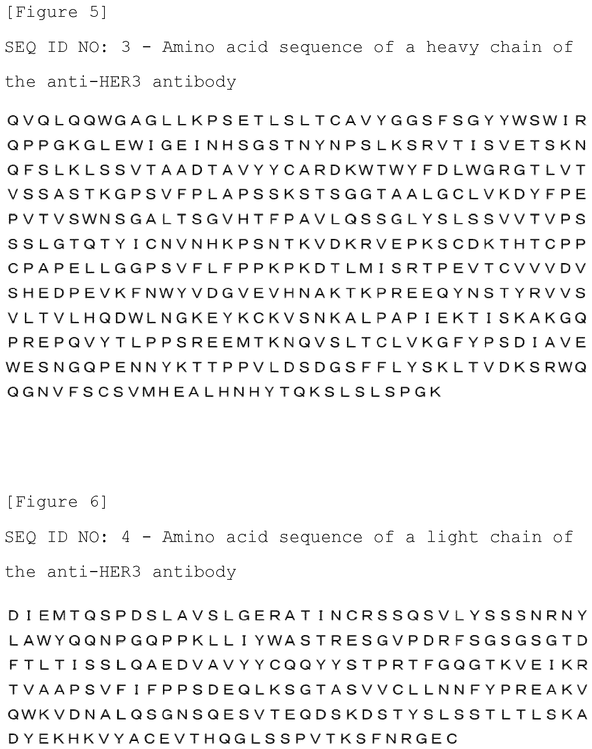

[Figure 5] Figure 5 is a diagram showing an amino acid sequence of a heavy chain of an anti-HER3 antibody (SEQ ID NO: 3).
[Figure 6] Figure 6 is a diagram showing an amino acid sequence of a light chain of an anti-HER3 antibody (SEQ ID NO: 4).

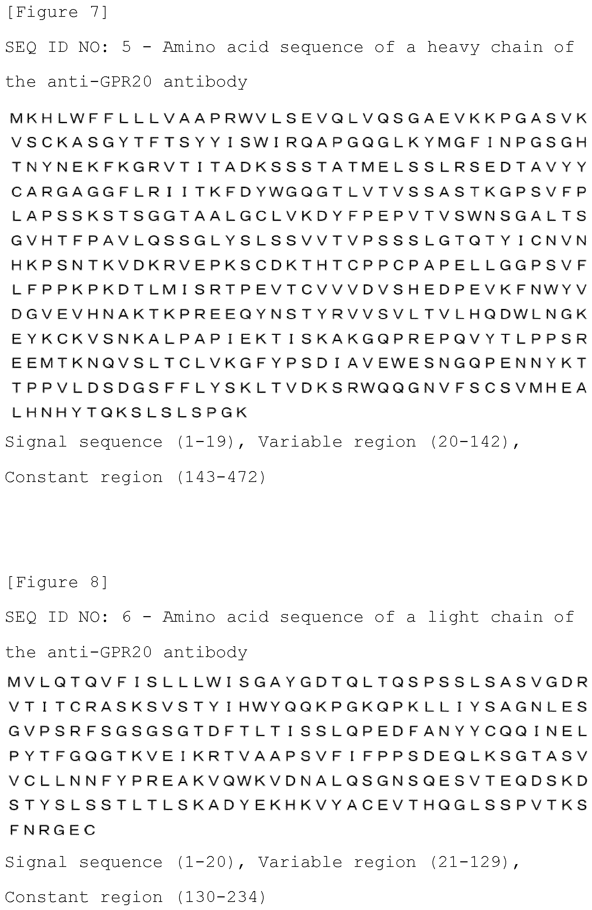

[Figure 7] Figure 7 is a diagram showing an amino acid sequence of a heavy chain of an anti-GPR20 antibody (SEQ ID NO: 5).
[Figure 8] Figure 8 is a diagram showing an amino acid sequence of a light chain of an anti-GPR20 antibody (SEQ ID NO: 6).

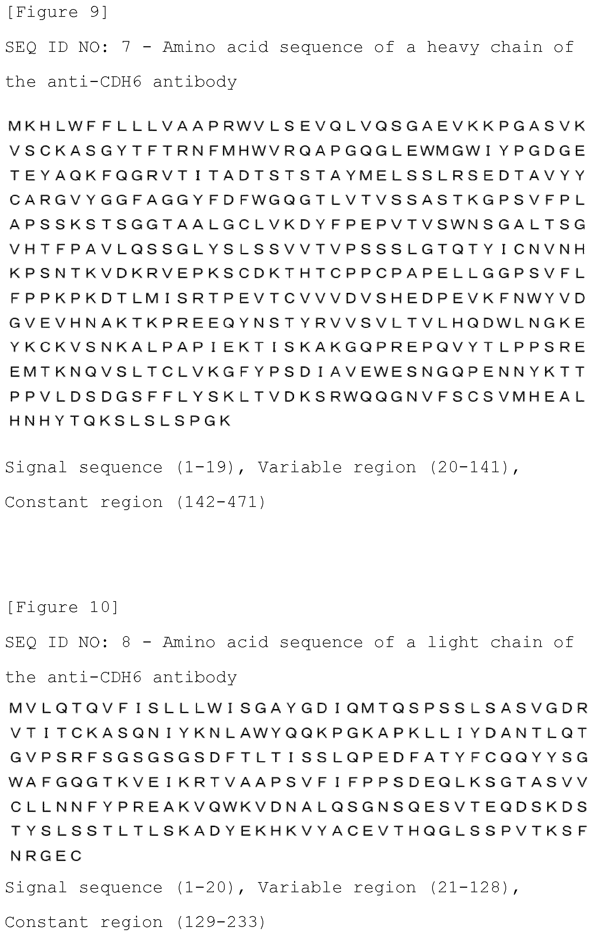

[Figure 9] Figure 9 is a diagram showing an amino acid sequence of a heavy chain of an anti-CDH6 antibody (SEQ ID NO: 7).
[Figure 10] Figure 10 is a diagram showing an amino acid sequence of a light chain of an anti-CDH6 antibody (SEQ ID NO: 8).

Description of Embodiments

Hereinafter, preferred modes for carrying out the present invention are described with reference to the drawings. The embodiments described below are given merely for illustrating one example of a typical embodiment of the present invention and are not intended to limit the scope of the present invention.

1. Antibody-drug conjugate

The antibody-drug conjugate produced by the present invention is an antibody-drug conjugate in which a drug-linker represented by formula (1)

[Chem. 27]

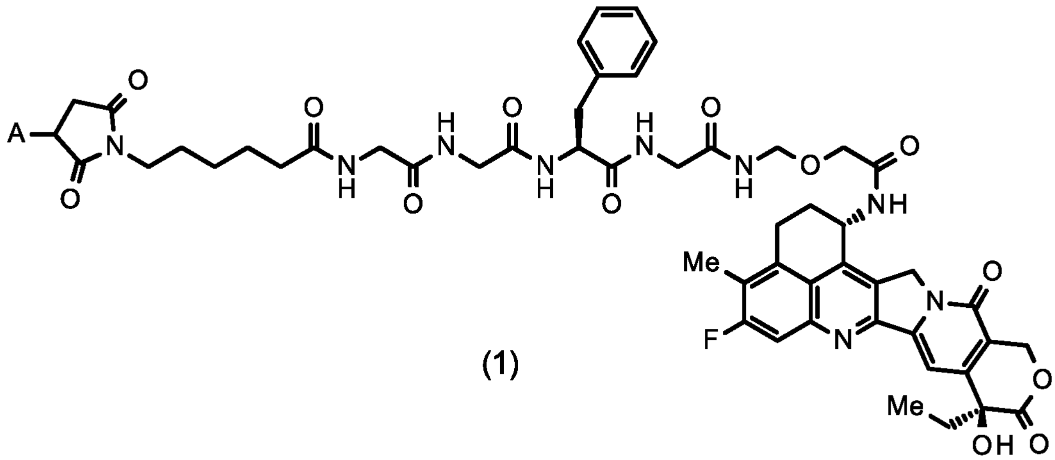

(1)

wherein A represents the connecting position to an antibody,
is conjugated to the antibody via a thioether bond.

In the present invention, the partial structure consisting of a linker and a drug in the antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

The drug-linker of the antibody-drug conjugate produced by the present invention includes exatecan, which is a topoisomerase I inhibitor, as a component. Exatecan is a camptothecin derivative having an antitumor effect, represented by formula (5):

[Chem. 28]

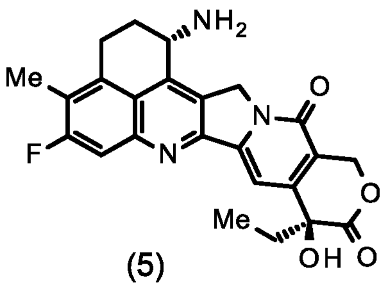

(5)

The antibody-drug conjugate produced by the present invention can be also represented by formula (6):

[Chem. 29]

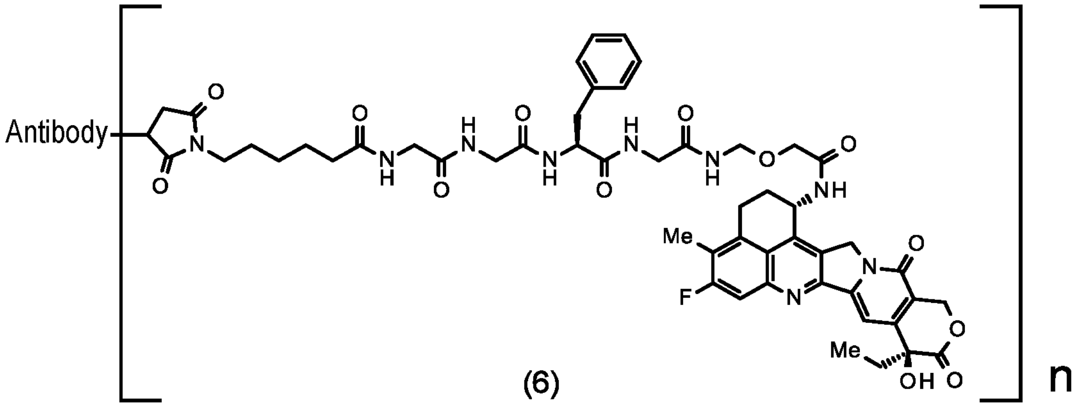

(6)

wherein, the drug-linker is conjugated to an antibody via a thioether bond. The meaning of n is the same as that of what is called the average number of conjugated drug molecules (DAR; Drug-to-Antibody Ratio), and indicates the average number of units of the drug-linker conjugated per antibody molecule.

After migrating into cancer cells, the antibody-drug conjugate produced by the present invention releases a compound represented by formula (7):

[Chem. 30]

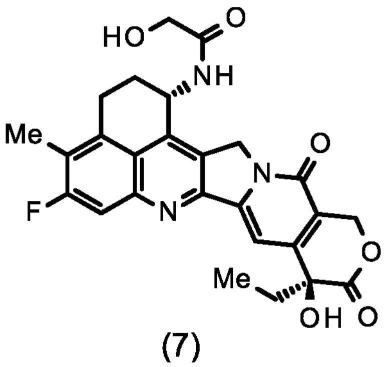

(7)

The compound represented by formula (7) is inferred to be the original source of the antitumor activity of the antibody-drug conjugate produced by the present invention, and has been confirmed to have topoisomerase I inhibitory effect (Ogitani Y. et al., Clinical Cancer Research, 2016, Oct 15; 22(20): 5097-5108, Epub 2016 Mar 29) .

The compound represented by formula (7) is inferred to be formed by decomposition of an aminal structure of the compound represented by formula (8):

[Chem. 31]

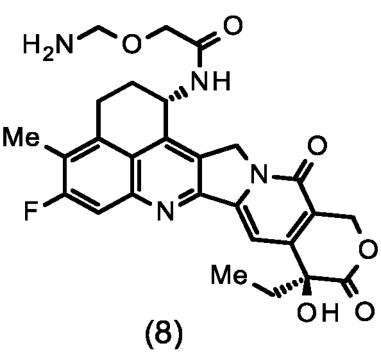

(8)

which is inferred to be formed by cleavage at the linker part of the antibody-drug conjugate produced by the present invention.

The antibody-drug conjugate produced by the present invention is also known to have a bystander effect (Ogitani Y. et al., Cancer Science (2016) 107, 1039-1046). The bystander effect is exerted through a process such that the antibody-drug conjugate produced by the present invention is internalized in cancer cells expressing a target, and the compound represented by formula (7) is released and then exerts an antitumor effect also on cancer cells which are present therearound and not expressing the target.

2. Antibody for use in production of antibody-drug conjugate

The antibody for use in production of the antibody-drug conjugate of the present invention may be derived from any species, and is preferably an antibody derived from a human, a rat, a mouse, or a rabbit. In cases when the antibody is derived from species other than human species, it is preferably chimerized or humanized using a well known technique. The antibody of the present invention may be a polyclonal antibody or a monoclonal antibody and is preferably a monoclonal antibody.

The antibody for use in production of the antibody-drug conjugate of the present invention is an antibody preferably having a characteristic of being capable of targeting cancer cells, and is preferably an antibody possessing, for example, a property of recognizing a cancer cell, a property of binding to a cancer cell, a property of being incorporated and internalized in a cancer cell, and/or cytocidal activity against cancer cells.

The binding activity of the antibody against cancer cells can be confirmed using flow cytometry. The internalization of the antibody into cancer cells can be confirmed using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added at varying concentrations into the culture system to determine inhibitory activity against focus formation, colony formation, and spheroid growth. The antitumor activity can be confirmed in vivo, for example, by administering the antibody to a nude mouse with a transplanted cancer cell line highly expressing the target protein, and determining change in the cancer cell.

Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of being internalized to migrate into cancer cells.

The antibody for use in production of the antibody-drug conjugate of the present invention can be obtained by a procedure known in the art. For example, the antibody of the present invention can be obtained using a method conventionally carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen are fused with myeloma cells according to a method known in the art (e.g., Kohler and Milstein, Nature (1975) 256, p. 495-497; and Kennet, R. ed., Monoclonal Antibodies, p. 365-367, Plenum Press, N.Y. (1980)) to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The antibody for use in production of the antibody-drug conjugate of the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)) .

As the humanized antibody, an antibody obtained by integrating only the complementarity determining region (CDR) of a heterologous antibody into a human-derived antibody (Nature (1986) 321, pp. 522-525), and an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology:Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002)43 (7), p.2301-2308; Carmen, S. et. al., Briefings in Functional Genomics and Proteomics (2002), 1(2), p.189-203; Siriwardena, D. et. al., Ophthalmology (2002) 109(3), p.427-431, etc.) can be exemplified.

In the present invention, modified variants of the antibody for use in production of the antibody-drug conjugate of the present invention are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, WO 99/54342, WO 00/61739, WO 02/31140, etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (the activation of complement, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions. However, an antibody in which one amino acid residue at the carboxyl terminus has been deleted in both of the two heavy chains in the antibody according to the present invention can be preferably exemplified.

As isotypes of the antibody according to the present invention, for example, IgG (IgG1, IgG2, IgG3, IgG4) can be exemplified, and IgG1 or IgG2 can be preferably exemplified.

Examples of antibodies applicable to production of the antibody-drug conjugate of the present invention can include, but are not particularly limited to, an anti-HER2 antibody, an anti-HER3 antibody, an anti-TROP2 antibody, an anti-B7-H3 antibody, an anti-CD3 antibody, an anti-CD30 antibody, an anti-CD33 antibody, an anti-CD37 antibody, an

anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, an anti-CD20 antibody, an anti-CD22 antibody, an anti-CD70 antibody, an anti-PSMA antibody, an anti-CEA antibody, an anti-Mesothelin antibody, an anti-A33 antibody, an anti-CanAg antibody, an anti-Cripto antibody, an anti-G250 antibody, an anti-MUC1 antibody, an anti-GPNMB antibody, an anti-Integrin antibody, an anti-Tenascin-C antibody, an anti-SLC44A4 antibody, an anti-GPR20 antibody, and an anti-CDH6 antibody. Further, an anti-HER2 antibody, an anti-HER3 antibody, an anti-TROP2 antibody, an anti-B7-H3 antibody, an anti-GPR20 antibody, and an anti-CDH6 antibody can be preferably exemplified, and an anti-HER2 antibody, an anti-HER3 antibody, an anti-GPR20 antibody, and an anti-CDH6 antibody can be more preferably exemplified.

In the present invention, the term "anti-HER2 antibody" refers to an antibody which binds specifically to HER2 (Human Epidermal Growth Factor Receptor Type 2; ErbB-2), and preferably has an activity of internalization in HER2-expressing cells by binding to HER2.

Examples of the anti-HER2 antibody include trastuzumab (U.S. Patent No. 5821337) and pertuzumab (International Publication No. WO 01/00245), and trastuzumab can be preferably exemplified.

In the present invention, the term "anti-HER3 antibody" refers to an antibody which binds specifically to HER3 (Human Epidermal Growth Factor Receptor Type 3; ErbB-3), and preferably has an activity of internalization in HER3-expressing cells by binding to HER3.

Examples of the anti-HER3 antibody include patritumab (U3-1287), U1-59 (International Publication No. WO 2007/077028), MM-121 (seribantumab), an anti-ERBB3 antibody described in International Publication No. WO 2008/100624, RG-7116 (lumretuzumab), and LJM-716 (elgemtumab), and patritumab and U1-59 can be preferably exemplified.

In the present invention, the term "anti-TROP2 antibody" refers to an antibody which binds specifically to TROP2 (TACSTD2: Tumor-associated calcium signal transducer 2; EGP-1), and preferably has an activity of internalization in TROP2-expressing cells by binding to TROP2.

Examples of the anti-TROP2 antibody include hTINA1-H1L1 (International Publication No. WO 2015/098099) .

In the present invention, the term "anti-B7-H3 antibody" refers to an antibody which binds specifically to B7-H3 (B cell antigen #7 homolog 3; PD-L3; CD276), and preferably has an activity of internalization in B7-H3-expressing cells by binding to B7-H3.

Examples of the anti-B7-H3 antibody include M30-H1-L4 (International Publication No. WO 2014/057687).

In the present invention, the term "anti-GPR20 antibody" refers to an antibody which binds specifically to GPR20 (G Protein-coupled receptor 20), and preferably has an activity of internalization in GPR20-expressing cells by binding to GPR20.

Examples of the anti-GPR20 antibody include h046-H4e/L7 (International Publication No. WO 2018/135501).

In the present invention, the term "anti-CDH6 antibody" refers to an antibody which binds specifically to CDH6 (cadherin-6), and preferably has an activity of internalization in CDH6-expressing cells by binding to CDH6.

Examples of the anti-CDH6 antibody include H01L02 (International Publication No. WO 2018/212136).

3. Drug-linker intermediate for use in production of antibody-drug conjugate

A drug-linker intermediate for use in production of the antibody-drug conjugate of the present invention is a compound represented by formula (2).

[Chem. 32]

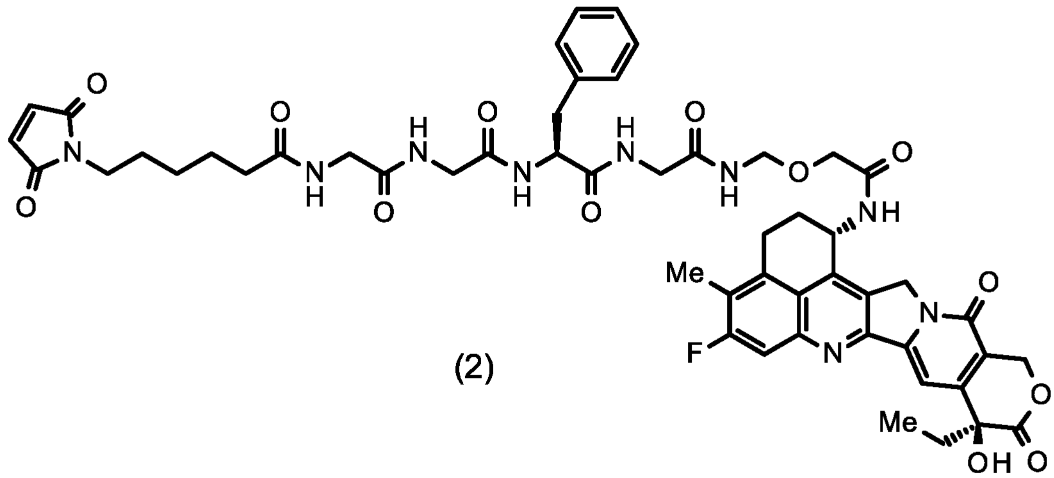

(2)

The compound represented by formula (2) can be produced with reference to descriptions in International Publication No. WO 2014/057687, International Publication No. WO 2015/098099, International Publication No. WO 2015/115091, International Publication No. WO 2015/155998, International Publication No. WO 2019/044947, and so on.

4. Conjugation of antibody and drug-linker intermediate

Conjugation of an antibody and a drug-linker intermediate in the method for producing an antibody-drug conjugate in the present invention includes the steps of:

(i) reducing the antibody with a reducing agent;
(ii) reacting a compound represented by formula (2) with the antibody reduced in step (i); and
(iii) adding a reagent having a thiol group to react with the residual compound represented by formula (2) in step (ii).

The reducing agent used in the step (i) is not particularly limited as long as it is capable of reducing an interchain disulfide of the antibody, and, for example, tris(2-carboxyethyl)phosphine or a salt thereof, dithiothreitol, or 2-mercaptoethanol can be used, tris(2-carboxyethyl)phosphine or a salt thereof can be preferably used, and tris(2-carboxyethyl)phosphine hydrochloride can be more preferably used.

The equivalent (hereinafter, an "equivalent" refers to a molar equivalent in the present invention) of the reducing agent used in step (i) per antibody molecule can be appropriately selected in accordance with the average number of units of the drug-linker conjugated per antibody molecule in the antibody-drug conjugate to be produced and the type of antibody.

For example, in the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), preferably 4.1 to 5.1 equivalents, more preferably 4.4 to 4.8 equivalents, even more preferably about 4.6 equivalents, of the reducing agent can be used per antibody molecule. Here, the phrase "about 4.6 equivalents" preferably refers to 4.5 to 4.7 equivalents, and more preferably refers to 4.6 equivalents.

In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), preferably 5.5 to 6.5 equivalents, more preferably 5.8 to 6.2 equivalents, even more preferably about 6 equivalents, of the reducing agent can be used per antibody molecule. Here, the phrase "about 6 equivalents" preferably refers to 5.9 to 6.1 equivalents, and more preferably refers to 6.0 equivalents.

In the case that the average number of units of the drug-linker conjugated per antibody molecule in the antibody-drug conjugate to be produced is in the range of from 7 to 8 and the antibody is an anti-GPR20 antibody (preferably, an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted), preferably 4.3 to 5.3 equivalents, more preferably 4.6 to 5 equivalents, even more preferably about 4.8 equivalents, of the reducing agent can be used per antibody molecule. Here, the phrase "about 4.8 equivalents" preferably refers to 4.7 to 4.9 equivalents, and more preferably refers to 4.8 equivalents.

In the case that the average number of units of the drug-linker conjugated per antibody molecule in the antibody-drug conjugate to be produced is in the range of from 7 to 8 and the antibody is an anti-CDH6 antibody (preferably, an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted), preferably 4.3 to 5.3 equivalents, more preferably 4.6 to 5 equivalents, even more preferably about 4.8 equivalents, of the reducing agent can be used per antibody molecule. Here, the phrase "about 4.8 equivalents" preferably refers to 4.7 to 4.9 equivalents, and more preferably refers to 4.8 equivalents.

Step (i) can be preferably performed in a buffer solution.

The pH of the buffer solution used in step (i) is preferably 6 to 8, more preferably 6.5 to 7.5, even more preferably 6.9 to 7.4, and even more preferably 7.0 to 7.3.

The buffer solution used in step (i) is not particularly limited as long as it can be used in reducing an interchain disulfide of the antibody, and, for example, an acetate buffer solution, a histidine buffer solution, a phosphate buffer solution, a piperazine-1,4-bis(2-ethanesulfonic acid) (hereinafter, also referred to as "PIPES") buffer solution, or a 4-(2-hydroxyethyl)-1-piperazine ethanesulfonic acid (hereinafter, also referred to as "HEPES") buffer solution can be used.

For example, in the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), an acetate buffer solution with the pH adjusted to 6 to 8 can be preferably used, an acetate buffer solution with the pH adjusted to 6 to 8 by using an aqueous solution of disodium hydrogen phosphate can be more preferably used, an acetate buffer solution with the pH adjusted to 6.8 to 7.8 by using an aqueous solution of disodium hydrogen phosphate can be even more preferably used, and an acetate buffer solution with the pH adjusted to about 7.3 by using an aqueous solution of disodium hydrogen phosphate can be even more preferably used. Here, the phrase "about 7.3" preferably refers to the range of from 7.1 to 7.5, more preferably refers to the range of from 7.2 to 7.4, and even more preferably refers to 7.3.

In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), an acetate buffer solution with the pH adjusted to 6 to 8 can be preferably used, an acetate buffer solution with the pH adjusted to 6 to 8 by using an aqueous solution of disodium hydrogen phosphate can be more preferably used, an acetate buffer solution with the pH adjusted to 6.5 to 7.5 by using an aqueous solution of disodium hydrogen phosphate can be even more preferably used, and an acetate buffer solution with the pH adjusted to about 7 by using an aqueous solution of disodium hydrogen phosphate can be even more preferably used. Here, the phrase "about 7" preferably refers to the range of from 6.8 to 7.2, more preferably refers to the range of from 6.9 to 7.1, and even more preferably refers to 7.0.

Use of such a buffer solution can minimize the generation of aggregates. The buffer solution used in step (i) may contain a buffer solution derived from antibody production.

Step (i) is preferably performed in the presence of a chelating agent. The chelating agent is not particularly limited as long as it can be used in reducing an interchain disulfide of the antibody, and, for example, ethylenediaminetetraacetic acid (hereinafter, also referred to as "EDTA"), diethylenetriaminepentaacetic acid, or glycol ether diaminetetraacetic acid can be used, and ethylenediaminetetraacetic acid can be preferably used.

Preferably, 1 to 20 equivalents of the chelating agent can be used per antibody molecule, 3 to 8 equivalents of the chelating agent can be more preferably used per antibody molecule, 4 to 6 equivalents of the chelating agent can be even more preferably used per antibody molecule, and 5 equivalents of the chelating agent can be even more preferably used per antibody molecule.

The buffer solution used in step (i) may contain a surfactant. The term "surfactant" in the present invention refers to a substance which has a hydrophilic group and a hydrophobic group and can be used as one of the components of a pharmaceutical preparation. Examples of such surfactants include polysorbates (including polysorbate 80 (Tween

80), polysorbate 20 (Tween 20), and polysorbate 60 (Tween 60)), polyoxyethylene (160) polyoxypropylene (30) glycol, polyoxyethylene hydrogenated castor oil 60, polyoxyethylene castor oil, and sodium laurylsulfate, and polysorbate 20 and polysorbate 80 can be more preferably exemplified.

Inclusion or exclusion of the surfactant in or from the buffer solution used in step (i), and the type of surfactant can be appropriately selected in accordance with the average number of units of the drug-linker conjugated per antibody molecule in the antibody-drug conjugate to be produced and the type of antibody.

For example, in the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), the buffer solution used in step (i) preferably contains no surfactant.

In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), the buffer solution used in step (i) can preferably contain polysorbate 20.

In the case that the average number of units of the drug-linker conjugated per antibody molecule in the antibody-drug conjugate to be produced is in the range of from 7 to 8 and the antibody is an anti-GPR20 antibody (preferably, an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted), the buffer solution used in step (i) can preferably contain polysorbate 80.

In the case that the average number of units of the drug-linker conjugated per antibody molecule in the antibody-drug conjugate to be produced is in the range of from 7 to 8 and the antibody is an anti-CDH6 antibody (preferably, an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted), the buffer solution used in step (i) can preferably contain polysorbate 80.

Step (i) can be performed preferably at an inner temperature of 25 to 50°C.

In the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), step (i) can be preferably performed at an inner temperature of 30 to 40°C, and can be more preferably performed at an inner temperature of about 35°C. Here, the phrase "about 35°C" preferably refers to 33°C to 37°C, more preferably refers to 34°C to 36°C, and even more preferably refers to 35°C.

In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), step (i) can be preferably performed at an inner temperature of 30 to 40°C, and can be more preferably performed at an inner temperature of about 35°C. Here, the phrase "about 35°C" preferably refers to 33°C to 37°C, more preferably refers to 34°C to 36°C, and even more preferably refers to 35°C.

In the case that the average number of units of the drug-linker conjugated per antibody molecule in the antibody-drug conjugate to be produced is in the range of from 7 to 8 and the antibody is an anti-GPR20 antibody (preferably, an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted), step (i) can be preferably performed at an inner temperature of 25 to 35°C, and can be more preferably performed at an inner temperature of about 30°C. Here, the phrase "about 30°C" preferably refers to 28°C to 32°C, more preferably refers to 29°C to 31°C, and even more preferably refers to 30°C.

In the case that the average number of units of the drug-linker conjugated per antibody molecule in the antibody-drug conjugate to be produced is in the range of from 7 to 8 and the antibody is an anti-CDH6 antibody (preferably, an

antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted), step (i) can be preferably performed at an inner temperature of 25 to 35°C, and can be more preferably performed at an inner temperature of about 30°C. Here, the phrase "about 30°C" preferably refers to 28°C to 32°C, more preferably refers to 29°C to 31°C, and even more preferably refers to 30°C.

The reaction time for step (i) is preferably 1 to 4 hours.

The equivalent of the compound represented by formula (2) used in step (ii) per antibody molecule can be appropriately selected in accordance with the average number of units of the drug-linker conjugated per antibody molecule in the antibody-drug conjugate to be produced and the type of antibody.

For example, in the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), preferably 8 to 10 equivalents, more preferably 8.2 to 9.2 equivalents, even more preferably about 8.7 equivalents, of the compound represented by formula (2) can be used per antibody molecule. Here, the phrase "about 8.7 equivalents" preferably refers to 8.5 to 8.9 equivalents, more preferably refers to 8.6 to 8.8 equivalents, and even more preferably refers to 8.7 equivalents.

In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), preferably 8 to 10 equivalents, more preferably 9 to 10 equivalents, even more preferably about 9.5 equivalents, of the compound represented by formula (2) can be used per antibody molecule. Here, the phrase "about 9.5 equivalents" preferably refers to 9.3 to 9.7 equivalents, more preferably refers to 9.4 to 9.6 equivalents, and even more preferably refers to 9.5 equivalents.

In the case that the average number of units of the drug-linker conjugated per antibody molecule in the antibody-drug conjugate to be produced is in the range of from 7 to 8 and the antibody is an anti-GPR20 antibody (preferably, an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted), preferably 8 to 10 equivalents, more preferably 8.3 to 9.3 equivalents, even more preferably about 8.8 equivalents, of the compound represented by formula (2) can be used per antibody molecule. Here, the phrase "about 8.8 equivalents" preferably refers to 8.6 to 9.0 equivalents, more preferably refers to 8.7 to 8.9 equivalents, and even more preferably refers to 8.8 equivalents.

In the case that the average number of units of the drug-linker conjugated per antibody molecule in the antibody-drug conjugate to be produced is in the range of from 7 to 8 and the antibody is an anti-CDH6 antibody (preferably, an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted), preferably 8 to 10 equivalents, more preferably 8.6 to 9.6 equivalents, even more preferably about 9.1 equivalents, of the compound represented by formula (2) can be used per antibody molecule. Here, the phrase "about 9.1 equivalents" preferably refers to 8.9 to 9.3 equivalents, more preferably refers to 9.0 to 9.2 equivalents, and even more preferably refers to 9.1 equivalents.

The compound represented by formula (2) in a state dissolved in a solvent can be preferably added to the reaction solution obtained in step (i). The solvent is not particularly limited as long as it can be used in the binding reaction with the antibody, and dimethylsulfoxide, an aqueous solution of dimethylsulfoxide, acetone, or an aqueous solution of acetone can be preferably used, an aqueous solution of dimethylsulfoxide can be more preferably used, and an 80% aqueous solution of dimethylsulfoxide can be even more preferably used. Further, any of these solvents can be preferably used with acetic acid contained therein. Preferably, 9 to 10 equivalents of acetic acid can be used per antibody molecule.

Step (ii) can be preferably performed at an inner temperature of 5 to 25°C, more preferably performed at an inner temperature of 10 to 20°C, and even more preferably performed at an inner temperature of about 15°C. Here, the phrase "about 15°C" preferably refers to 13°C to 17°C, more preferably refers to 14°C to 16°C, and even more preferably refers to 15°C.

The reaction time for step (ii) is preferably 0.5 to 2 hours.

The reagent having a thiol group can be used in step (iii) to react with the residual compound represented by

formula (2) in step (ii). In other words, the reagent having a thiol group can be used in step (iii) to quench an excess portion of the compound represented by formula (2) .

The reagent having a thiol group used in step (iii) is not particularly limited as long as it can react with the maleimidyl group of the compound represented by formula (2), and, for example, N-acetylcysteine or cysteine can be used, and N-acetylcysteine can be preferably used.

In step (iii), 10 to 50 equivalents of the reagent having a thiol group can be preferably used per antibody molecule, and 10 to 30 equivalents of the reagent having a thiol group can be more preferably used.

Step (iii) can be preferably performed at an inner temperature of 5 to 25°C, more preferably performed at an inner temperature of 10 to 20°C, and even more preferably performed at an inner temperature of about 15°C. Here, the phrase "about 15°C" preferably refers to 13°C to 17°C, more preferably refers to 14°C to 16°C, and even more preferably refers to 15°C.

The reaction time for step (iii) is preferably 0.5 to 2 hours.

5. Purification of antibody-drug conjugate

The method for producing an antibody-drug conjugate in the present invention is characterized by obtaining a solution containing an unpurified product or crude product of the antibody-drug conjugate through the steps of:

(i) reducing an antibody with a reducing agent;
(ii) reacting a compound represented by formula (2)

[Chem. 33]

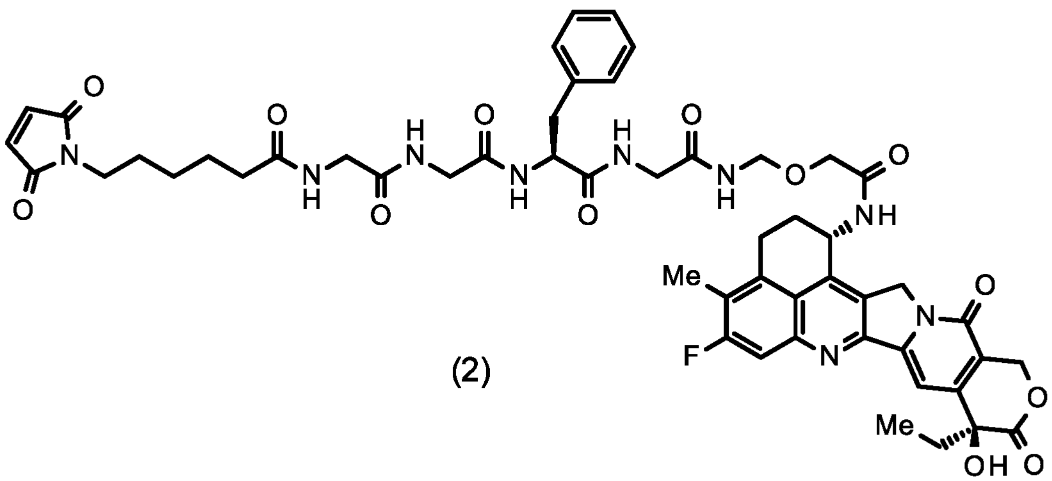

(2)

with the antibody reduced in step (i); and then
(iii) adding a reagent having a thiol group, and purifying the solution containing an unpurified product or crude product of the antibody-drug conjugate through the step of:
(iv) removing a compound in which the reducing agent used in step (i) is added to the maleimidyl group of the compound represented by formula (2), and a compound in which the reagent having a thiol group used in step (iii) is added to the maleimidyl group of the compound represented by formula (2), through ultrafiltration using a buffer solution containing a salt consisting of a strong acid and a strong base.

The phrase "an unpurified product or crude product of the antibody-drug conjugate" in the present invention refers to the antibody-drug conjugate in an unpurified state immediately after performing steps (i) to (iii), or the antibody-drug conjugate in a state partially purified through an operation other than chromatography (e.g., simple filtration other than ultrafiltration). Examples of simple filtration other than ultrafiltration include microfiltration (filtration with a membrane filter).

The "unpurified product or crude product of the antibody-drug conjugate" may be a product with the pH adjusted so that the pH of the buffer solution used in step (iv) can reach a suitable pH. Such pH adjustment can be preferably performed with an aqueous solution of acetic acid, and can be more preferably performed with a 10% aqueous solution of acetic acid.

The term "ultrafiltration" in the present invention refers to a purification method to separate large solute molecules

and small solute molecules or separate solute molecules and solvent molecules by filtration through a membrane (ultrafiltration membrane) having a pore size of from about 0.001 μm to about 0.05 μm. In general, ultrafiltration membranes have a molecular weight cutoff (MWCO) in the range of from 1 kDa to 1000 kDa. MWCO is generally defined as the molecular weight of a spherical solute such that 90% of the spherical solute molecules are retained by the membrane. The ultrafiltration in the present invention can be preferably performed by using an ultrafiltration membrane with MWCO of 1 kDa to 100 kDa, and more preferably performed by using an ultrafiltration membrane with MWCO of 30 kDa. Examples of the material of the ultrafiltration membrane include regenerated cellulose, cellulose acetate, aromatic polyamide, polyvinyl alcohol, polysulfone, polyether sulfone, polyvinylidene fluoride, polyethylene, polyacrylonitrile, nylon, and ceramics. The ultrafiltration in the present invention can be preferably performed by using an ultrafiltration membrane the material of which is regenerated cellulose, though the material is not limited thereto. Examples of the ultrafiltration membrane used in the present invention can include a Pellicon (R) XL Cassette Ultracel (R) (produced by Merck KGaA), a Pellicon (R) 2 Ultracel (R) (produced by Merck KGaA), and a Pellicon (R) 3 Ultracel (R) (produced by Merck KGaA). Ultrafiltration may refer to a method of forcibly filtering through pressure control or centrifugation in a narrow sense. On the other hand, methods of filtering through passive diffusion may be generally referred to as "diafiltration". However, any of the methods using an ultrafiltration membrane is included in the scope of "ultrafiltration" in the present invention.

The term "aggregate" in the present invention refers to a fine particle which consists of an association of protein molecules and any other component and can be stable under a wide range of pH and electrical conductivity. The formation of aggregates in an antibody-drug conjugate formulation can have medically undesirable impacts, for example, possibly causing immunogenicity or vein disorder to a patient to whom the formulation is to be administered. Accordingly, it is required to suppress the formation of aggregates in formulating the antibody-drug conjugate.

In the case that the reducing agent used in step (i) is tris(2-carboxyethyl)phosphine hydrochloride, the compound in which the reducing agent used in step (i) is added to the maleimidyl group of the compound represented by formula (2) is a compound represented by formula (3).

[Chem. 34]

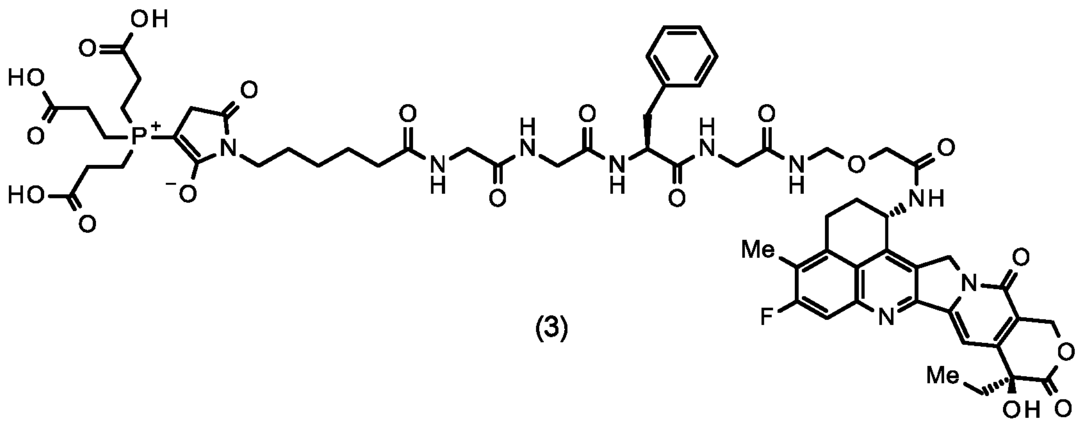

(3)

In the case that the reagent having a thiol group used in step (iii) is N-acetylcysteine, the compound in which the reagent having a thiol group used in step (iii) is added to the maleimidyl group of the compound represented by formula (2) is a compound represented by formula (4).

[Chem. 35]

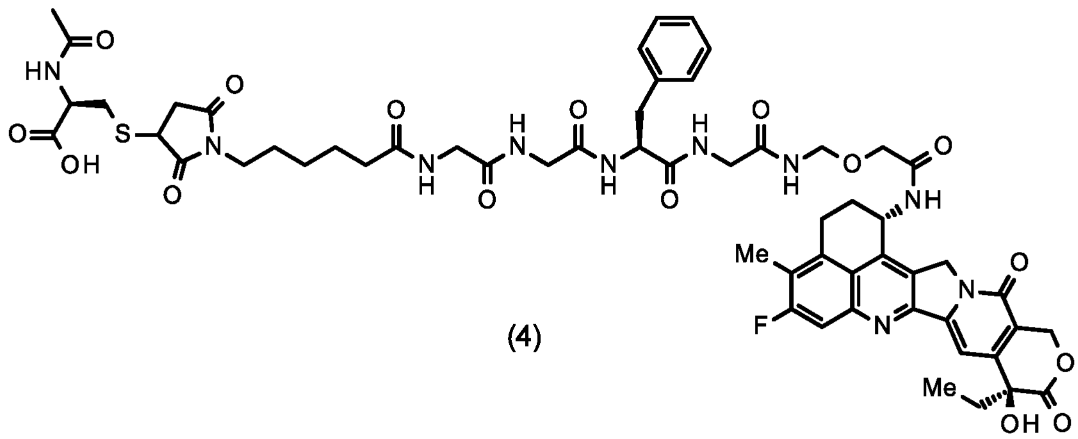

(4)

Preferably 97%, more preferably 98%, even more preferably 99%, even more preferably 100%, of the compound represented by formula (3) and the compound represented by formula (4) before step (iv) can be removed through step (iv).

The pH of the buffer solution used in step (iv) is about 5. Here, "about 5" is preferably the range of from 4.7 to 5.3, more preferably the range of from 4.8 to 5.2, even more preferably the range of from 4.9 to 5.1, and even more preferably 5.0. The generation of aggregates can be suppressed through ultrafiltration at such a pH. The amount of aggregates after this step is preferably 4% or less, and more preferably 2% or less. A certain amount of aggregates is contained in the raw material antibody, and the amount of aggregates in this step is detected as the total amount including them.

Examples of the buffer solution used in step (iv) include a histidine buffer solution containing a salt consisting of a strong acid and a strong base, an acetate buffer solution containing a salt consisting of a strong acid and a strong base, and a phosphate buffer solution containing a salt consisting of a strong acid and a strong base, and a histidine buffer solution containing a salt consisting of a strong acid and a strong base can be preferably exemplified.

The concentration of the salt consisting of the strong acid and the strong base is preferably 0.2 to 1 wt%, more preferably 0.3 to 0.9 wt%, even more preferably 0.3 to 0.8 wt%, even more preferably 0.4 to 0.7 wt%, and even more preferably about 0.5 wt%, with respect to the buffer solution used in step (iv). Here, "about 0.5 wt%" is preferably 0.4 to 0.6 wt%, and even more preferably 0.5 wt%.

The salt consisting of the strong acid and the strong base contained in the buffer solution used in step (iv) is not particularly limited as long as the advantageous effects of the present invention are exhibited, and, for example, is at least one salt selected from the group consisting of sodium chloride, potassium chloride, sodium sulfate, and potassium sulfate, or a salt comprising a combination of two or more of them, and is preferably sodium chloride.

The production method of the present invention comprises a step subsequent to step (iv) of (v) removing the salt consisting of a strong acid and a strong base through ultrafiltration using a buffer solution.

The pH of the buffer solution used in step (v) is not particularly limited as long as the advantageous effects of the present invention are exhibited, and, for example, is in the range of from 4 to 6, and preferably about 5. Here, "about 5" is preferably the range of from 4.7 to 5.3, more preferably the range of from 4.8 to 5.2, even more preferably the range of from 4.9 to 5.1, and even more preferably 5.0.

The buffer solution used in step (v) is not particularly limited as long as the advantageous effects of the present invention are exhibited, and a histidine buffer solution is preferably used. This histidine buffer solution is substantially free of a salt consisting of a strong acid and a strong base.

The above method can remove by-products derived from the compound represented by formula (2) through ultrafiltration, and further minimize the generation of aggregates, and can provide an industrially excellent purification method without need of purification by chromatography (for example, at least one selected from the group consisting of gel filtration chromatography, ion exchange chromatography, hydrophobic chromatography, and affinity chromatography).

6. Calculation of average number of conjugated drug molecules in antibody-drug conjugate

The average number of conjugated drug molecules per antibody molecule of the antibody-drug conjugate produced can be determined, for example, by a method of calculation based on measurement of UV absorbance for the antibody-drug conjugate and the conjugation precursor thereof at two wavelengths of 280 nm and 370 nm (UV method),

or a method of calculation based on quantification through HPLC measurement for fragments obtained by treating the antibody-drug conjugate with a reducing agent (HPLC method).

Calculation of the average number of conjugated drug molecules per antibody molecule of the antibody-drug conjugate can be performed with reference to descriptions in International Publication No. WO 2014/057687, International Publication No. WO 2015/098099, International Publication No. WO 2015/115091, International Publication No. WO 2015/155998, International Publication No. WO 2018/135501, International Publication No. WO 2018/212136, and so on.

The HPLC method can be performed, for example, in the following manner.

(1) Preparation of sample for HPLC analysis (reduction of antibody-drug conjugate)

An antibody-drug conjugate solution (about 1 mg/mL, 60 μL) is mixed with an aqueous solution of dithiothreitol (DTT) (100 mM, 15 μL). A sample in which the interchain disulfide bond of the antibody-drug conjugate has been cleaved by incubating the mixture for 30 minutes at 37°C is used in HPLC analysis.

(2) HPLC analysis

HPLC analysis can be performed under measurement conditions according to the characteristics of the antibody.

For example, in the case that the antibody is an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2), or an anti-HER3 antibody (an antibody comprising a heavy chain consisting of the amino acid sequence represented by SEQ ID NO: 3 and a light chain consisting of the amino acid sequence represented by SEQ ID NO: 4), HPLC analysis can be performed under the following measurement conditions.

HPLC system: Agilent 1290 HPLC system (Agilent Technologies, Inc.)
Detector: ultraviolet absorption spectrometer (measurement wavelength: 280 nm)
Column: PLRP-S (2.1 × 50 mm, 8 μm, 1000 angstroms; Agilent Technologies, Inc., P/N PL1912-1802)
Column temperature: 80°C
Mobile phase A: aqueous solution containing 0.04% trifluoroacetic acid (TFA)
Mobile phase B: acetonitrile solution containing 0.04% TFA
Gradient program: 29%-36% (0-12.5 min), 36%-42% (12.5-15 min), 42%-29% (15-15.1 min), 29%-29% (15.1-25 min)
Sample injection volume: 15 μL

In the case that the antibody is an anti-GPR20 antibody (an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6), or an anti-CDH6 antibody (an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8), HPLC analysis can be performed under the following measurement conditions.

HPLC system: Agilent 1290 HPLC system (Agilent Technologies, Inc.)
Detector: ultraviolet absorption spectrometer (measurement wavelength: 280 nm)
Column: ACQUITY UPLC BEH Phenyl (2.1 × 50 mm, 1.7 μm, 130 angstroms; Waters Corporation, P/N 186002884)
Column temperature: 80°C
Mobile phase A: aqueous solution containing 0.10% trifluoroacetic acid (TFA) and 15% 2-propanol
Mobile phase B: acetonitrile solution containing 0.075% TFA and 15% 2-propanol
Gradient program: 14%-36% (0-15 min), 36%-80% (15-17 min), 80%-14% (17-17.01 min), 14% (17.01-25 min)
Sample injection volume: 10 μL

(3) Data analysis

Compared with non-conjugated antibody light (Lo) and heavy ($H_0$) chains, drug-conjugated light (light chain connected to one drug molecule: $L_1$) and heavy (heavy chain connected to one drug molecule: $H_1$, heavy chain connected to two drug molecules: $H_2$, heavy chain connected to three drug molecules: $H_3$) chains exhibit higher hydrophobicity in proportion to the number of conjugated drug molecules and thus have a larger retention time. These chains are therefore eluted in the order of $L_0$ and $L_1$ or $H_0$, $H_1$, $H_2$, and $H_3$. Detection peaks can be assigned to any of $L_0$, $L_1$, $H_0$, $H_1$, $H_2$, and $H_3$ by the comparison of retention times with $L_0$ and $H_0$.

Since the drug-linker has UV absorption, peak area values are corrected in response to the number of conjugated

drug-linker molecules according to the following expression using the molar absorption coefficients of the light or heavy chain and the drug-linker.

[Math. 1]

$$\text{Corrected value of the peak area of the light chain (Li)} = \text{Peak area} \times \frac{\text{Molar absorption coefficient of the light chain}}{\text{Molar absorption coefficient of the light chain} + \text{The number of conjugated drug molecules} \times \text{Molar absorption coefficient of the drug-linker}}$$

[Math. 2]

$$\text{Corrected value of the peak area of the heavy chain (Hi)} = \text{Peak area} \times \frac{\text{Molar absorption coefficient of the heavy chain}}{\text{Molar absorption coefficient of the heavy chain} + \text{The number of conjugated drug molecules} \times \text{Molar absorption coefficient of the drug-linker}}$$

Here, as the molar absorption coefficient (280 nm) of the light or heavy chain of each antibody, a value estimated from the amino acid sequence of the light or heavy chain of each antibody by a known calculation method (Protein Science, 1995, vol. 4, 2411-2423) can be used.

For example, in the case that the antibody is an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2), a molar absorption coefficient of 26150 and a molar absorption coefficient of 81290 can be used as estimated values for the light and heavy chains, respectively.

In the case that the antibody is an anti-HER3 antibody (an antibody comprising a heavy chain consisting of the amino acid sequence represented by SEQ ID NO: 3 and a light chain consisting of the amino acid sequence represented by SEQ ID NO: 4), a molar absorption coefficient of 34690 and a molar absorption coefficient of 95000 can be used as estimated values for the light and heavy chains, respectively.

In the case that the antibody is an anti-GPR20 antibody (an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6), a molar absorption coefficient of 26210 and a molar absorption coefficient of 68990 can be used as estimated values for the light and heavy chains, respectively.

In the case that the antibody is an anti-CDH6 antibody (an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8), a molar absorption coefficient of 31710 and a molar absorption coefficient of 79990 can be used as estimated values for the light and heavy chains, respectively.

As the molar absorption coefficient (280 nm) of the drug-linker, the measured molar absorption coefficient (280 nm) of a compound in which the maleimidyl group is converted to succinimide thioether by the reaction of each drug-linker intermediate with mercaptoethanol or N-acetylcysteine can be used.

The peak area ratio (%) of each chain is calculated for the total of the corrected values of peak areas according to the following expression.

[Math. 3]

$$\text{Peak area ratio of the light chain} = \frac{A_{Li}}{A_{L0} + A_{L1}} \times 100$$

$$\text{Peak area ratio of the heavy chain} = \frac{A_{Hi}}{A_{H0} + A_{H1} + A_{H2} + A_{H3}} \times 100$$

$A_{Li}$, $A_{Hi}$: Corrected values of respective peak areas of $L_i$, $H_i$

The average number of conjugated drug molecules in the antibody-drug conjugate is calculated according to the following expression.

Average number of conjugated drug molecules = ($L_0$ peak area ratio × 0 + $L_1$ peak area ratio × 1 + $H_0$ peak area ratio × 0 + $H_1$ peak area ratio × 1 + $H_2$ peak area ratio × 2 + $H_3$ peak area ratio × 3) / 100 × 2

In the present invention, the term "anti-HER2 antibody-drug conjugate" refers to an antibody-drug conjugate such that the antibody in an antibody-drug conjugate produced in the present invention is an anti-HER2 antibody.

The anti-HER2 antibody is preferably an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2.

The average number of units of the drug-linker conjugated per antibody molecule in the anti-HER2 antibody-drug conjugate used in the present invention is preferably 7 to 8, more preferably 7.5 to 8, and even more preferably about 8.

In the present invention, the term "anti-HER3 antibody-drug conjugate" refers to an antibody-drug conjugate such that the antibody in an antibody-drug conjugate produced in the present invention is an anti-HER3 antibody.

The anti-HER3 antibody is preferably an antibody comprising a heavy chain including CDRH1 consisting of an amino acid sequence consisting of amino acid residues 26 to 35 of SEQ ID NO: 3, CDRH2 consisting of an amino acid sequence consisting of amino acid residues 50 to 65 of SEQ ID NO: 3, and CDRH3 consisting of an amino acid sequence consisting of amino acid residues 98 to 106 of SEQ ID NO: 3, and a light chain including CDRL1 consisting of an amino acid sequence consisting of amino acid residues 24 to 39 of SEQ ID NO: 4, CDRL2 consisting of an amino acid sequence consisting of amino acid residues 56 to 62 of SEQ ID NO: 4, and CDRL3 consisting of an amino acid sequence consisting of amino acid residues 95 to 103 of SEQ ID NO: 4,

more preferably an antibody comprising a heavy chain including a heavy chain variable region consisting of an amino acid sequence consisting of amino acid residues 1 to 117 of SEQ ID NO: 3 and a light chain including a light chain variable region consisting of an amino acid sequence consisting of amino acid residues 1 to 113 of SEQ ID NO: 4,

and even more preferably an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted.

The average number of units of the drug-linker conjugated per antibody molecule in the anti-HER3 antibody-drug conjugate is preferably 7 to 8, more preferably 7.5 to 8, and even more preferably about 8.

In the present invention, the term "anti-GPR20 antibody-drug conjugate" refers to an antibody-drug conjugate such that the antibody in an antibody-drug conjugate produced in the present invention is an anti-GPR20 antibody.

The anti-GPR20 antibody is preferably an antibody comprising a heavy chain including CDRH1 consisting of an amino acid sequence consisting of amino acid residues 45 to 54 of SEQ ID NO: 5, CDRH2 consisting of an amino acid sequence consisting of amino acid residues 69 to 78 of SEQ ID NO: 5, and CDRH3 consisting of an amino acid sequence consisting of amino acid residues 118 to 131 of SEQ ID NO: 5, and a light chain including CDRL1 consisting of an amino acid sequence consisting of amino acid residues 44 to 54 of SEQ ID NO: 6, CDRL2 consisting of an amino acid sequence consisting of amino acid residues 70 to 76 of SEQ ID NO: 6, and CDRL3 consisting of an amino acid

sequence consisting of amino acid residues 109 to 117 of SEQ ID NO: 6,
more preferably an antibody comprising a heavy chain including a heavy chain variable region consisting of an amino acid sequence consisting of amino acid residues 20 to 142 of SEQ ID NO: 5 and a light chain including a light chain variable region consisting of an amino acid sequence consisting of amino acid residues 21 to 129 of SEQ ID NO: 6,
and even more preferably an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted.

The average number of units of the drug-linker conjugated per antibody molecule in the anti-GPR20 antibody-drug conjugate is preferably 2 to 8, more preferably 3 to 8, even more preferably 7 to 8, even more preferably 7.5 to 8, and even more preferably about 8.

In the present invention, the term "anti-CDH6 antibody-drug conjugate" refers to an antibody-drug conjugate such that the antibody in an antibody-drug conjugate produced in the present invention is an anti-CDH6 antibody.

The anti-CDH6 antibody is preferably an antibody comprising a heavy chain including CDRH1 consisting of an amino acid sequence consisting of amino acid residues 45 to 54 of SEQ ID NO: 7, CDRH2 consisting of an amino acid sequence consisting of amino acid residues 69 to 78 of SEQ ID NO: 7, and CDRH3 consisting of an amino acid sequence consisting of amino acid residues 118 to 130 of SEQ ID NO: 7, and a light chain including CDRL1 consisting of an amino acid sequence consisting of amino acid residues 44 to 54 of SEQ ID NO: 8, CDRL2 consisting of an amino acid sequence consisting of amino acid residues 70 to 76 of SEQ ID NO: 8, and CDRL3 consisting of an amino acid sequence consisting of amino acid residues 109 to 116 of SEQ ID NO: 8,
more preferably an antibody comprising a heavy chain including a heavy chain variable region consisting of an amino acid sequence consisting of amino acid residues 20 to 141 of SEQ ID NO: 7 and a light chain including a light chain variable region consisting of an amino acid sequence consisting of amino acid residues 21 to 128 of SEQ ID NO: 8,
and even more preferably an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted.

The average number of units of the drug-linker conjugated per antibody molecule in the anti-CDH6 antibody-drug conjugate is preferably 2 to 8, more preferably 3 to 8, even more preferably 7 to 8, even more preferably 7.5 to 8, and even more preferably about 8.

7. Production of pharmaceutical composition

The pharmaceutical composition according to the present invention is a pharmaceutical composition containing the antibody-drug conjugate according to the present invention, a buffer solution, and an excipient.

The pharmaceutical composition can be produced by producing (including purification) an antibody-drug conjugate by the above-described method, and then performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

The buffer solution added in step (vi) is preferably the same as the buffer solution used in step (v). Thus, a histidine buffer solution can be preferably used as the buffer solution used in step (vi).

In the case that the buffer solution used in step (vi) is a histidine buffer solution, an aqueous solution of histidine can be preferably used for pH adjustment to be performed in step (viii).

The term "excipient" in the present invention refers to a substance to be added to provide a specific size or concentration, for example, for the purpose of improving a pharmaceutical with respect to molding, handling, and convenience of administration. The excipient is not particularly limited as long as the advantageous effects of the present invention are exhibited, and examples thereof include sucrose, trehalose, and sorbitol.

For the excipient added in step (ix), sucrose can be preferably used.

Preferably, the pharmaceutical composition according to the present invention further contains a surfactant. Thus, the pharmaceutical composition according to the present invention is more preferably a pharmaceutical composition containing the antibody-drug conjugate, a buffer solution, an excipient, and a surfactant.

This pharmaceutical composition can be produced by performing the steps including the above-described step (ix) and the additional subsequent step (x) of adding a surfactant.

For the surfactant added in step (x), polysorbate 80 or polysorbate 20 can be preferably used.

The buffer solution, excipient, surfactant, and the concentration of the antibody-drug conjugate in the pharmaceutical composition, and the pH of the pharmaceutical composition can be appropriately selected in accordance with the average number of units of the drug-linker conjugated per antibody molecule in the antibody-drug conjugate to be produced and the type of antibody.

For example, in the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), a histidine buffer solution (preferably, a 25 mM histidine buffer solution), sucrose (preferably, 9% sucrose), and polysorbate 80 (preferably, 0.03% polysorbate 80) can be preferably used for the buffer solution, excipient, and surfactant in the pharmaceutical composition. In that case, the concentration of the antibody-drug conjugate in the pharmaceutical composition is preferably 20 mg/mL, and the pH of the pharmaceutical composition is preferably 5.5.

In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), a histidine buffer solution (preferably, a 25 mM histidine buffer solution), sucrose (preferably, 9% sucrose), and polysorbate 20 (preferably, 0.03% polysorbate 20) can be preferably used for the buffer solution, excipient, and surfactant in the pharmaceutical composition. In that case, the concentration of the antibody-drug conjugate in the pharmaceutical composition is preferably 20 mg/mL, and the pH of the pharmaceutical composition is preferably 5.4.

In the case that the average number of units of the drug-linker conjugated per antibody molecule in the antibody-drug conjugate to be produced is in the range of from 7 to 8 and the antibody is an anti-GPR20 antibody (preferably, an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted), a histidine buffer solution (preferably, a 10 mM histidine buffer solution), sucrose (preferably, 9% sucrose), and polysorbate 80 (preferably, 0.03% polysorbate 80) can be preferably used for the buffer solution, excipient, and surfactant in the pharmaceutical composition. In that case, the concentration of the antibody-drug conjugate in the pharmaceutical composition is preferably 20 mg/mL, and the pH of the pharmaceutical composition is preferably 5.4.

In the case that the average number of units of the drug-linker conjugated per antibody molecule in the antibody-drug conjugate to be produced is in the range of from 7 to 8 and the antibody is an anti-CDH6 antibody (preferably, an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted), a histidine buffer solution (preferably, a 10 mM histidine buffer solution), sucrose (preferably, 9% sucrose), and polysorbate 80 (preferably, 0.03% polysorbate 80) can be preferably used for the buffer solution, excipient, and surfactant in the pharmaceutical composition. In that case, the concentration of the antibody-drug conjugate in the pharmaceutical composition is preferably 20 mg/mL, and the pH of the pharmaceutical composition is preferably 5.4.

8. Use of pharmaceutical composition

The pharmaceutical composition of the present invention can be expected to exert a therapeutic effect by application as systemic therapy to patients, and additionally, by local application to cancer tissues.

The pharmaceutical composition of the present invention can be preferably used for a mammal, but is more preferably used for a human.

Examples of the administration route applicable to administration of the pharmaceutical composition of the present invention can include intravenous, intradermal, subcutaneous, intramuscular, and intraperitoneal routes, and intravenous routes are preferred.

In the case that the pharmaceutical composition of the present invention is an aqueous injection, it can preferably be diluted with a suitable diluent and then given as an intravenous infusion. For the diluent, a dextrose solution, physiological saline, and the like, can be exemplified, a dextrose solution can be preferably exemplified, and a 5% dextrose solution can be more preferably exemplified.

In the case that the pharmaceutical composition of the present invention is a lyophilized injection, it can preferably be dissolved in water for injection, subsequently a required amount can be diluted with a suitable diluent and then given

as an intravenous infusion. For the diluent, a dextrose solution physiological saline, and the like, can be exemplified, a dextrose solution can be preferably exemplified, and 5% dextrose solution can be more preferably exemplified.

The pharmaceutical composition of the present invention can exhibit a pharmaceutical effect even at a small dosage when the antibody-drug conjugate of the present invention has a higher affinity for an antigen, that is, a higher affinity (= lower Kd value) in terms of the dissociation constant (that is, Kd value) for the antigen. Thus, the dosage of the pharmaceutical composition of the present invention can be determined in view of the situation relating to the affinity with the antigen. When the pharmaceutical composition of the present invention is administered to a human, for example, about 0.001 to 100 mg/kg in terms of the antibody-drug conjugate (here, "mg/kg" refers to the dosage of the antibody-drug conjugate per kg body weight of a human) needs to be administered once or administered in several portions with intervals of 1 to 180 days, and a method of administering 0.8 mg/kg to 8 mg/kg once every three weeks can be preferably exemplified.

The dosage and administration interval of the antibody-drug conjugate can be appropriately selected in accordance with the average number of units of the drug-linker conjugated per antibody molecule in the antibody-drug conjugate to be produced and the type of antibody. For example, in the case that the average number of units of the drug-linker conjugated per antibody molecule in the antibody-drug conjugate to be produced is in the range of from 7 to 8 and the antibody is an anti-HER2 antibody (preferably, an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2, or an antibody comprising a heavy chain consisting of the amino acid sequence represented by SEQ ID NO: 1 and a light chain consisting of the amino acid sequence represented by SEQ ID NO: 2), a method of administering 0.8 mg/kg, 1.6 mg/kg, 3.2 mg/kg, 5.4 mg/kg, 6.4 mg/kg, 7.4 mg/kg, or 8 mg/kg of the antibody-drug conjugate once every three weeks can be preferably exemplified, a method of administering 5.4 mg/kg, 6.4 mg/kg, 7.4 mg/kg, or 8 mg/kg of the antibody-drug conjugate once every three weeks can be more preferably exemplified, and a method of administering 5.4 mg/kg or 6.4 mg/kg of the antibody-drug conjugate once every three weeks can be even more preferably exemplified. In the case that the average number of units of the drug-linker conjugated per antibody molecule in the antibody-drug conjugate to be produced is in the range of from 7 to 8 and the antibody is an anti-HER3 antibody (preferably, an antibody comprising a heavy chain consisting of the amino acid sequence represented by SEQ ID NO: 3 and a light chain consisting of the amino acid sequence represented by SEQ ID NO: 4, or a variant of the antibody in which a lysine residue at the carboxyl terminus of the heavy chain is deleted), a method of administering 1.6 mg/kg, 3.2 mg/kg, 4.8 mg/kg, 5.6 mg/kg, 6.4 mg/kg, 8.0 mg/kg, 9.6 mg/kg, or 12.8 mg/kg of the antibody-drug conjugate once every three weeks can be preferably exemplified, and a method of administering 4.8 mg/kg, 5.6 mg/kg, or 6.4 mg/kg of the antibody-drug conjugate once every three weeks can be more preferably exemplified.

The pharmaceutical composition of the present invention can be used for treating cancer, and can be preferably used for treating at least one cancer selected from the group consisting of breast cancer, gastric cancer (also called gastric adenocarcinoma), colorectal cancer (also called colon and rectal cancer and including colon cancer and rectal cancer), lung cancer (including small cell lung cancer and non-small cell lung cancer), esophageal cancer, head-and-neck cancer (including salivary gland cancer and pharyngeal cancer), esophagogastric junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulvar cancer, thyroid cancer, penis cancer, leukemia, malignant lymphoma, plasmacytoma, myeloma, glioblastoma multiforme, osteosarcoma, and melanoma.

The pharmaceutical composition of the present invention can be selectively used as an agent for drug therapy, which is a main method for treating cancer, and as a result can delay development of cancer cells, inhibit growth thereof, and further kill cancer cells. These effects can allow cancer patients to be free from symptoms caused by cancer or achieve improvement in QOL of cancer patients and attain a therapeutic effect by sustaining the lives of the cancer patients. Even if the pharmaceutical composition of the present invention does not accomplish killing cancer cells, it can achieve higher QOL of cancer patients while achieving longer-term survival, by inhibiting or controlling the growth of cancer cells.

In such drug therapy, the pharmaceutical composition of the present invention can be used as an agent alone and, in addition, it can be used as an agent in combination with an additional therapy in adjuvant therapy and can be combined with surgical operation, radiotherapy, hormone therapy, or the like. Furthermore, it can also be used as an agent for drug therapy in neoadjuvant therapy.

In addition to the therapeutic use as described above, for example, a prophylactic effect such as suppressing the growth of small metastatic cancer cells and further killing them can also be expected for the pharmaceutical composition of the present invention. For example, an effect of inhibiting and killing cancer cells in a body fluid in the course of metastasis or an effect of, for example, inhibiting and killing small cancer cells immediately after implantation in any tissue can be expected. Accordingly, inhibition of cancer metastasis or a prophylactic effect can be expected, particularly,

after surgical removal of cancer.

The pharmaceutical composition of the present invention can be administered in combination with other cancer treating agents. The antitumor effect may be enhanced accordingly. Other cancer treating agents used for such purpose may be administered to an individual simultaneously with, separately from, or subsequently to the pharmaceutical composition of the present invention, and may be administered while varying the administration interval for each. Such a cancer treating agent is not limited as long as it has antitumor activity, and an example thereof is at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, a tegafur/gimeracil/oteracil-containing agent, cetuximab, panitumumab, bevacizumab, ramucirumab, regorafenib, a trifluridine/tipiracil-containing agent, gefitinib, erlotinib, afatinib, methotrexate, pemetrexed, trastuzumab emtansin, trastuzumab, pertuzumab, tamoxifen, toremifene, fulvestrant, leuprorelin, goserelin, letrozole, anastrozole, and a progesterone formulation.

Examples

The present invention is more specifically described with reference to the Examples shown below. However, the present invention is not limited to these.

[Example 1] Examination of buffer solution

A solution containing an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2) was diluted with various buffer solutions (10 mM acetate buffer solution, 10 mM phosphate buffer solution, or 20 mM PIPES buffer solution), to which 0.5 mol/L aqueous solution of EDTA was added, and various weakly alkaline aqueous solutions (0.3 mol/L aqueous solution of disodium hydrogen phosphate (hereinafter, also referred to as "aq. $Na_2HPO_4$"), saturated aqueous solution of sodium acetate (hereinafter, also referred to as "aq. $CH_3COONa$"), or 0.5 mol/L aqueous solution of sodium hydrogen carbonate (hereinafter, also referred to as "aq. $NaHCO_3$")) were then added thereto to adjust the pH to 7. Under stirring at 37°C, 4.8 equivalents or 4.9 equivalents of an aqueous solution of tris(2-carboxyethyl)phosphine hydrochloride (hereinafter, also referred to as "TCEP") (1 mg/mL) per antibody molecule were added, and the resultant was stirred to reduce the interchain disulfide of the antibody.

To the reaction solution obtained, 9.2 equivalents or 9.8 equivalents of a compound represented by formula (2) :

[Chem. 36]

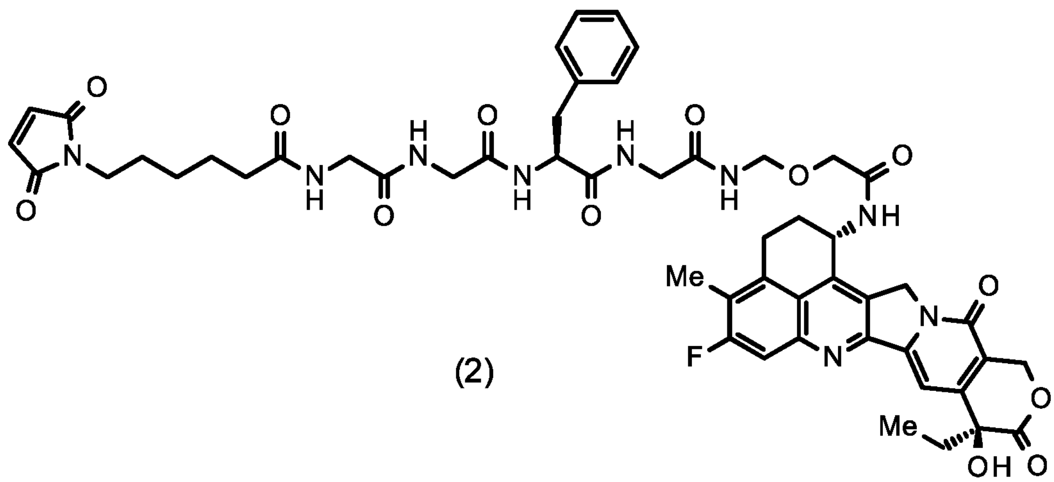

(2)

(also referred to as compound (2)) dissolved in 80% aqueous solution of dimethylsulfoxide were added with stirring at an inner temperature of 15°C or 25°C to bind compound (2) to the antibody. Next, 50 mmol/L aqueous solution of N-acetylcysteine was added thereto to quench an excess portion of compound (2). This provided an anti-HER2 antibody-drug conjugate in which a drug-linker represented by a formula:

[Chem. 37]

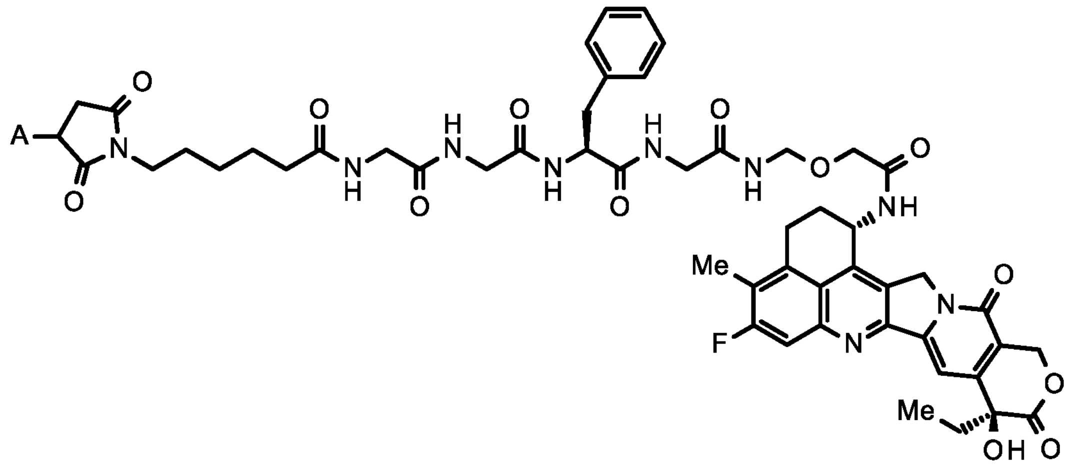

wherein A represents the connecting position to an antibody,
is conjugated to the anti-HER2 antibody via a thioether bond.

The DAR of the anti-HER2 antibody-drug conjugate obtained was measured through an HPLC method. The aggregate content was measured through SEC.

The results are shown in Table 1.

[Table 1]

| Buffer | pH adjustment | Reduction step | | Conjugation step | | DAR | Aggregate |
|---|---|---|---|---|---|---|---|
| | | TCEP | Inner temperature | Compound (2) | Inner temperature | | |
| Acetate buffer solution | aq. $Na_2HPO_4$ | 4.8 equivalents | 37°C | 9.2 equivalents | 15°C | 7.89 | 0.66% |
| Acetate buffer solution | aq. $Na_2HPO_4$ | 4.8 equivalents | 37°C | 9.2 equivalents | 25°C | 7.88 | 0.92% |
| Phosphate buffer solution | aq. $Na_2HPO_4$ | 4.8 equivalents | 37°C | 9.2 equivalents | 15°C | 7.89 | 1.54% |
| PIPES buffer solution | aq. $Na_2HPO_4$ | 4.8 equivalents | 37°C | 9.2 equivalents | 15°C | 7.89 | 1.61% |
| Acetate buffer solution | aq. $CH_3COONa$ | 4.9 equivalents | 37°C | 9.8 equivalents | 15°C | 7.80 | 1.13% |
| Acetate buffer solution | aq. $NaHCO_3$ | 4.9 equivalents | 37°C | 9.8 equivalents | 15°C | 7.81 | 2.13% |

Among the results in Table 1, the case that acetate buffer solution subjected to pH adjustment by using an aqueous solution of disodium hydrogen phosphate showed the lowest aggregate content.

[Example 2] Examination of ultrafiltration step (1)

A solution containing an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2) was diluted with 10 mM acetate buffer solution (pH 5.5), to which 0.5 mol/L aqueous solution of EDTA (5 equivalents per antibody molecule) was added, and 0.3 mol/L aqueous solution of disodium hydrogen phosphate was then added thereto to adjust the pH to 7.4.

Under stirring at 35 to 39°C, 0.010 mol/L aqueous solution of tris(2-carboxyethyl)phosphine hydrochloride (6 equivalents per antibody molecule) was added, and the resultant was stirred at an inner temperature of 35 to 39°C for 2.5 hours to reduce the interchain disulfide of the antibody.

To the reaction solution obtained, compound (2) (12.5 equivalents per antibody molecule) dissolved in 80% aqueous solution of dimethylsulfoxide was added with stirring at an inner temperature of 13 to 17°C to bind compound (2) to the antibody. Next, 0.1 mol/L aqueous solution of N-acetylcysteine (35 equivalents per antibody molecule) was added thereto, the resultant was further stirred at the same temperature for 1 hour to quench an excess portion of compound (2), and then the pH of the reaction solution was adjusted to 5.0 using 10% aqueous solution of acetic acid. This provided a reaction solution containing an anti-HER2 antibody-drug conjugate in which a drug-linker represented by a formula:

[Chem. 38]

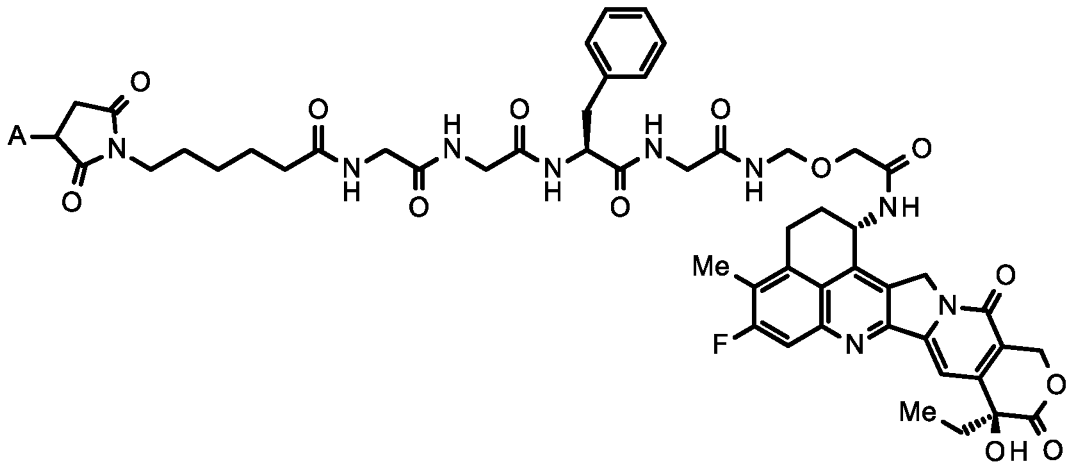

wherein A represents the connecting position to an antibody,
is conjugated to the anti-HER2 antibody via a thioether bond.

The reaction solution obtained was circulated for ultrafiltration through the ultrafiltration membrane Pellicon (R) XL Cassette Ultracel (R) (produced by Merck KGaA) with a roller pump while 26 mM histidine buffer solution (pH 5.0) or 0.5% sodium chloride-containing 26 mM histidine buffer solution (pH 5.0) was added thereto. The 26 mM histidine buffer solution (pH 5.0) and 0.5% sodium chloride-containing 26 mM histidine buffer solution (pH 5.0) used were in amounts of 5 times, 10 times, or 15 times the amount of the reaction solution obtained. Compound (2)-derived by-product content based on the antibody-drug conjugate after the ultrafiltration was measured. For comparison, compound (2)-derived by-product content without ultrafiltration was measured (in such cases, the amount of buffer solution used was indicated as 0).

Examples of compound (2)-derived by-products include a compound in which tris(2-carboxyethyl)phosphine is added to the maleimidyl group of compound (2), that is, a compound represented by formula (3):

[Chem. 39]

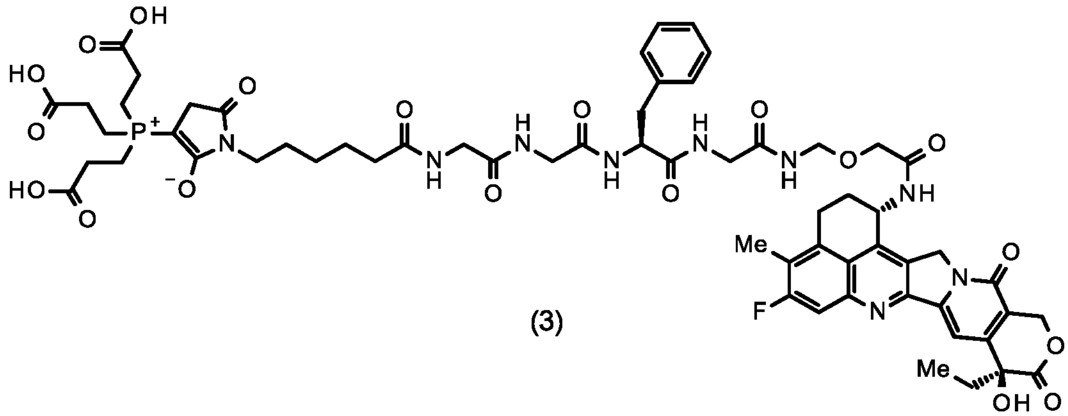

(3)

(also referred to as compound (3)), and a compound in which N-acetylcysteine is added to the maleimidyl group of compound (2), that is, a compound represented by formula (4):

[Chem. 40]

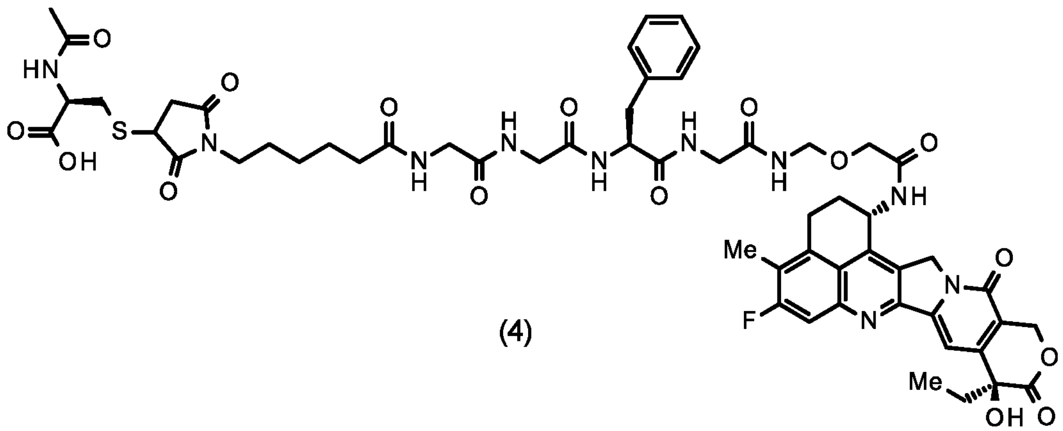

(4)

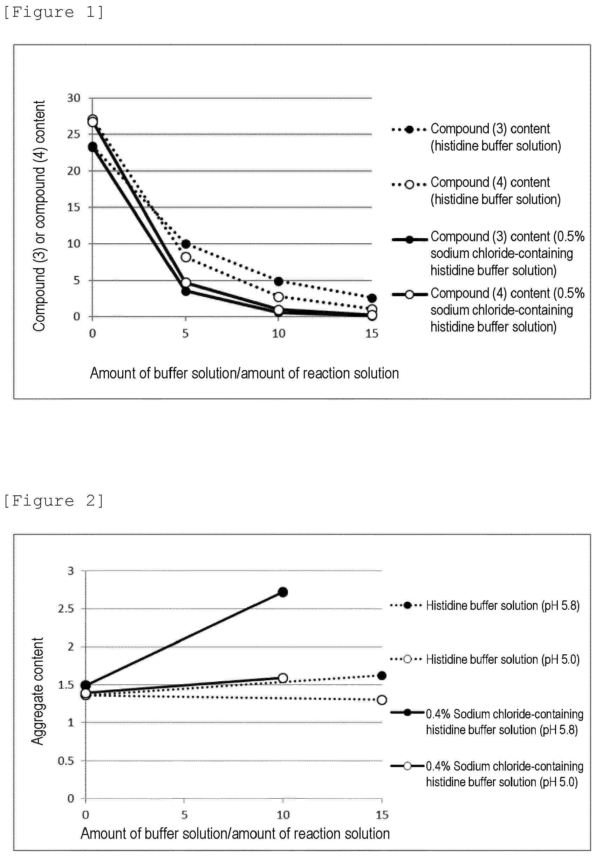

(also referred to as compound (4)). In measurement of compound (2)-derived by-product content, an HPLC method was performed to determine compound (3) and compound (4) content based on the antibody-drug conjugate. The results are shown in Table 2 and Figure 1.

[Table 2]

| Amount of buffer solution/ amount of reaction solution | Histidine buffer solution | | 0.5% sodium chloride-containing histidine buffer solution | |
|---|---|---|---|---|
| | Compound (3) content (%) | Compound (4) content (%) | Compound (3) content (%) | Compound (4) content (%) |
| 0 | 23.37 | 27.12 | 23.25 | 26.78 |
| 5 | 10.03 | 8.19 | 3.53 | 4.67 |
| 10 | 4.91 | 2.76 | 0.58 | 0.98 |
| 15 | 2.60 | 1.04 | 0.11 | 0.25 |

Comparison of the results in Table 2 with the same amount of buffer solution/amount of reaction solution shows that compound (3) and compound (4) content when sodium chloride-containing histidine buffer solution was used were

lower than those when histidine buffer solution was used.

[Example 3] Examination of ultrafiltration step (2)

A solution containing an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2) was diluted with 10 mM acetate buffer solution, to which 0.5 mol/L aqueous solution of EDTA was added, and 0.3 mol/L aqueous solution of disodium hydrogen phosphate was then added thereto to adjust the pH to 7.3. Thereto, 1 mg/mL aqueous solution of tris(2-carboxyethyl)phosphine hydrochloride was added, and the resultant was stirred to reduce the interchain disulfide of the antibody.

To the reaction solution obtained, compound (2) dissolved in 80% aqueous solution of dimethylsulfoxide was added to bind compound (2) to the antibody. Next, 50 mmol/L aqueous solution of N-acetylcysteine was added thereto to quench an excess portion of compound (2). This provided a reaction solution containing an anti-HER2 antibody-drug conjugate in which a drug-linker represented by a formula:

[Chem. 41]

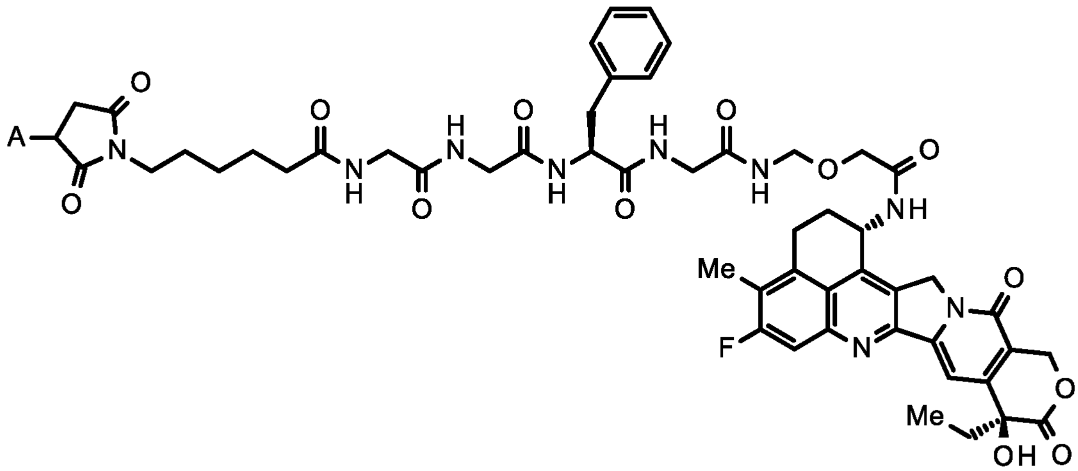

wherein A represents the connecting position to an antibody,
is conjugated to the anti-HER2 antibody via a thioether bond.

The reaction solution obtained was circulated for ultrafiltration through Pellicon (R) XL Cassette Ultracel (R) (produced by Merck KGaA) with a roller pump while histidine buffer solution (pH 5.0 or pH 5.8) or 0.4% sodium chloride-containing histidine buffer solution (pH 5.0 or pH 5.8) was added thereto. The histidine buffer solutions used each had a concentration of 10.8 mM. The histidine buffer solutions (pH 5.0 and pH 5.8) used were each in an amount of 15 times the amount of the reaction solution obtained. The 0.4% sodium chloride-containing histidine buffer solutions (pH 5.0 and pH 5.8) used was each in an amount 10 times the amount of the reaction solution obtained. Aggregate content after the ultrafiltration was measured through SEC. For comparison, aggregate content without ultrafiltration was measured (in such cases, the amount of buffer solution used was indicated as 0). The results are shown in Table 3 and Figure 2.

[Table 3]

| Buffer | pH | Amount of buffer solution/amount of reaction solution | Aggregate content |
|---|---|---|---|
| Histidine buffer solution | 5.8 | 0 | 1.36 |
| | | 15 | 1.62 |
| Histidine buffer solution | 5.0 | 0 | 1.36 |
| | | 15 | 1.3 |

(continued)

| Buffer | pH | Amount of buffer solution/amount of reaction solution | Aggregate content |
|---|---|---|---|
| 0.4% sodium chloride-containing histidine buffer solution | 5.8 | 0 | 1.49 |
| | | 10 | 2.72 |
| 0.4% sodium chloride-containing histidine buffer solution | 5.0 | 0 | 1.39 |
| | | 10 | 1.59 |

The results in Table 3 show that, in the case of ultrafiltration performed with buffer solution of pH 5.8, the aggregate content when sodium chloride-containing histidine buffer solution was used was higher than that when histidine buffer solution was used. In the case of ultrafiltration performed with buffer solution of pH 5.0, on the other hand, the aggregate content when sodium chloride-containing histidine buffer solution was used was comparable to that when histidine buffer solution was used.

[Example 4] Production of pharmaceutical composition containing anti-HER2 antibody-drug conjugate (1)

A solution containing an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2) (weight of solution: 5.4 kg; corresponding to 120 g of the antibody) was placed in a glass reaction vessel, and 0.01 mol/L acetate buffer solution (4.3 L, pH 5.5) was further added thereto. To this solution, 0.5 mol/L aqueous solution of EDTA (8.3 mL; 5 equivalents per antibody) was added, and 0.3 mol/L aqueous solution of disodium hydrogen phosphate was then added thereto to adjust the pH to 7.3. Under stirring at 35 to 39°C, 0.010 mol/L aqueous solution of tris(2-carboxyethyl)phosphine hydrochloride (390 mL; 4.7 equivalents per antibody molecule) was added thereto, and the resultant was stirred at an inner temperature of 35 to 39°C for 2 hours to reduce the interchain disulfide of the antibody.

The reaction solution obtained was cooled, and compound (2) (8.2 g; 9.7 equivalents per antibody molecule) dissolved in 80% aqueous solution of dimethylsulfoxide (900 mL) was added thereto under stirring at an inner temperature of 13 to 17°C over 20 minutes, and the resultant was stirred at the same temperature for 1 hour to bind compound (2) to the antibody. Next, 0.1 mol/L aqueous solution of N-acetylcysteine (210 mL; 25 equivalents per antibody molecule) was added thereto and the resultant was further stirred at the same temperature for 1 hour to quench an excess portion of compound (2), and then the pH was adjusted to 5.0 using 10% aqueous solution of acetic acid. This provided a solution containing an anti-HER2 antibody-drug conjugate in which a drug-linker represented by a formula:

[Chem. 42]

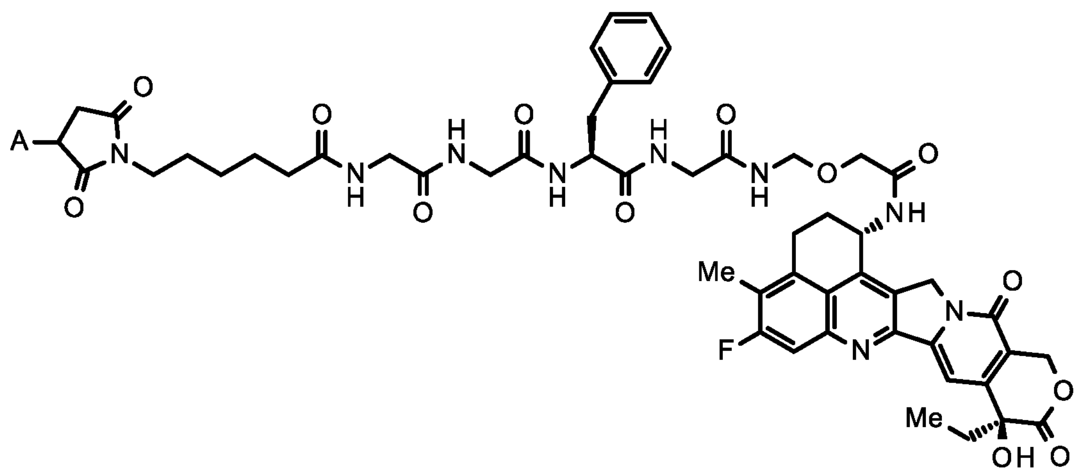

wherein A represents the connecting position to an antibody,
is conjugated to the anti-HER2 antibody via a thioether bond.

The solution obtained was circulated for ultrafiltration through three membranes of Pellicon (R) 2 Mini Cassette Ultracel (R) (produced by Merck KGaA, 0.1 $m^2$) with a roller pump while 0.5% sodium chloride-containing 26 mM histidine buffer solution (pH 5.0) was added thereto to remove compound (2)-derived by-products. Further, the solution was circulated for ultrafiltration while 26 mM histidine buffer solution (pH 5.0) was added thereto to remove sodium chloride. Next, the pH of the resultant was adjusted to 5.5 by using an aqueous solution of histidine, and further concentrated to obtain approximately 4 L of a solution containing an anti-HER2 antibody-drug conjugate.

Further, a portion of 3.86 kg was taken from the solution, to which 364 g of sucrose was added to dissolve therein. Furthermore, 9% sucrose-containing histidine buffer solution (pH 5.5) was added thereto to adjust the protein concentration to approximately 20 mg/mL, and thus a pharmaceutical composition (5.7 kg) containing the anti-HER2 antibody-drug conjugate was obtained. The protein concentration of the pharmaceutical composition, the protein yield, and the average number of conjugated drug molecules per antibody molecule (n) were 20.4 mg/mL, 112 g, and 7.7, respectively.

[Example 5] Production of pharmaceutical composition containing anti-HER2 antibody-drug conjugate (2)

A solution containing an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2) (weight of solution: 89 g; corresponding to 2.0 g of the antibody) was placed in a glass reaction vessel, and 0.01 mol/L acetate buffer solution (72 mL, pH 5.5) was further added thereto. To this solution, 0.5 mol/L aqueous solution of EDTA (0.14 mL; 5 equivalents per antibody) was added, and 0.3 mol/L aqueous solution of disodium hydrogen phosphate was then added thereto to adjust the pH to 7.3. Under stirring at 35 to 39°C, 0.010 mol/L aqueous solution of tris(2-carboxyethyl)phosphine hydrochloride (6.3 mL; 4.6 equivalents per antibody molecule) was added thereto, and the resultant was stirred at an inner temperature of 35 to 39°C for 2 hours to reduce the interchain disulfide of the antibody.

The reaction solution obtained was cooled, and compound (2) (140 mg; 9.6 equivalents per antibody molecule) dissolved in 80% aqueous solution of dimethylsulfoxide (13 mL) was added thereto under stirring at an inner temperature of 13 to 17°C, and the resultant was stirred at the same temperature for 1 hour to bind compound (2) to the antibody. Next, 0.1 mol/L aqueous solution of N-acetylcysteine (3.4 mL; 25 equivalents per antibody molecule) was added thereto and the resultant was further stirred at the same temperature for 40 minutes to quench an excess portion of compound (2), and then the pH was adjusted to 5.0 using 10% aqueous solution of acetic acid. This provided a solution containing an anti-HER2 antibody-drug conjugate in which a drug-linker represented by a formula:

[Chem. 43]

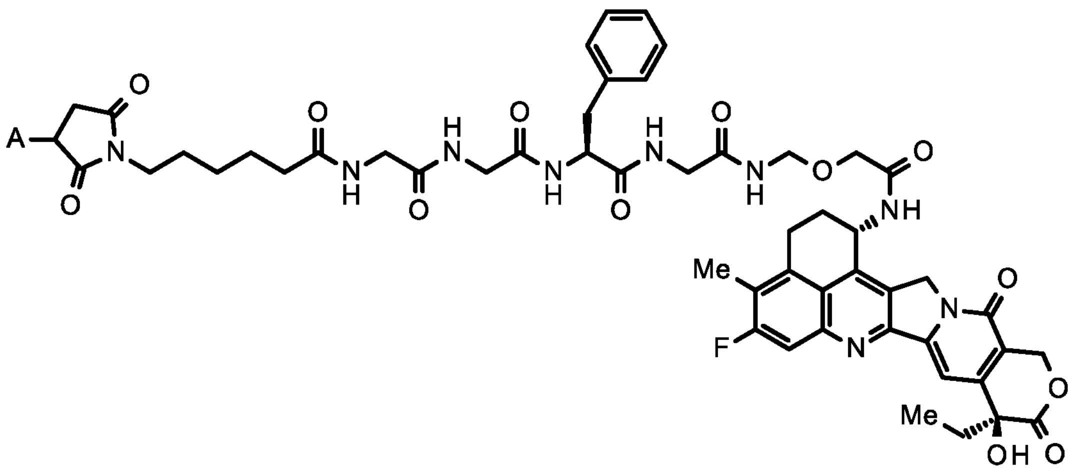

wherein A represents the connecting position to an antibody,
is conjugated to the anti-HER2 antibody via a thioether bond.

After 1 g of sodium chloride was added to the solution obtained, the solution was circulated for ultrafiltration through a Pellicon (R) XL Ultracel (R) (produced by Merck KGaA, 50 $cm^2$) with a roller pump while 0.5% sodium chloride-containing 26 mM histidine buffer solution (pH 5.0) was added thereto to remove compound (2)-derived by-products. Further, the solution was circulated for ultrafiltration while 26 mM histidine buffer solution (pH 5.0) was added thereto to

remove sodium chloride. Next, the resultant was concentrated while the pH was adjusted to 5.5 by using an aqueous solution of histidine, and thus approximately 65 g of a solution containing an anti-HER2 antibody-drug conjugate (64.5 g, 64.1 mL, protein concentration: 28.5 mg/mL, protein yield: 1.83 g) was obtained.

Further, a portion of 64 g was taken from the solution, to which 19 mL of histidine buffer solution (pH 5.5) containing 7.7 g of sucrose was added, and furthermore 9% sucrose-containing histidine buffer solution (pH 5.5) was added thereto to adjust the protein concentration to approximately 20 mg/mL, and thus a pharmaceutical composition (93 g) containing the anti-HER2 antibody-drug conjugate was obtained. The protein concentration of the pharmaceutical composition, the protein yield, and the average number of conjugated drug molecules per antibody molecule (n) were 20.3 mg/mL, 1.8 g, and 7.8, respectively.

[Example 6] Production of pharmaceutical composition containing anti-HER3 antibody-drug conjugate

A solution containing an anti-HER3 antibody (an antibody comprising a heavy chain consisting of the amino acid sequence represented by SEQ ID NO: 3 and a light chain consisting of the amino acid sequence represented by SEQ ID NO: 4) (weight of solution: 43.95 kg; corresponding to 3.00 kg of the antibody) was placed in a 400 L single-use reactor, to which 0.01 mol/L acetate buffer solution (pH 5.5, 255 kg) containing polysorbate 20 (60.0 g) and 0.5 mol/L aqueous solution of EDTA (224.5 g; 5 equivalents per antibody) was further added thereto. To this solution, 0.3 mol/L aqueous solution of disodium hydrogen phosphate was added to adjust the pH to 7.0. Under stirring at 33 to 37°C, an aqueous solution (12.3 kg) containing tris(2-carboxyethyl)phosphine hydrochloride (35.1 g: 6.0 equivalents per antibody molecule) was added thereto, and the resultant was stirred at an inner temperature of 33 to 37°C for 2.5 hours to reduce the interchain disulfide of the antibody.

The reaction solution obtained was cooled, and compound (2) (200.5 g; 9.5 equivalents per antibody molecule) dissolved in 80% aqueous solution of dimethylsulfoxide (21.1 kg) containing 10% aqueous solution of acetic acid (116.4 g) was added thereto under stirring at an inner temperature of 12 to 17°C over 50 minutes, and the resultant was stirred at the same temperature for 0.5 hours to bind compound (2) to the antibody. Next, 0.1 mol/L aqueous solution of N-acetylcysteine (3.11 kg; 15 equivalents per antibody molecule) was added thereto and the resultant was further stirred at the same temperature for 0.5 hours to quench an excess portion of compound (2), and then the pH was adjusted to 5.0 using 10% aqueous solution of acetic acid. This provided a solution containing an anti-HER3 antibody-drug conjugate in which a drug-linker represented by a formula:

[Chem. 44]

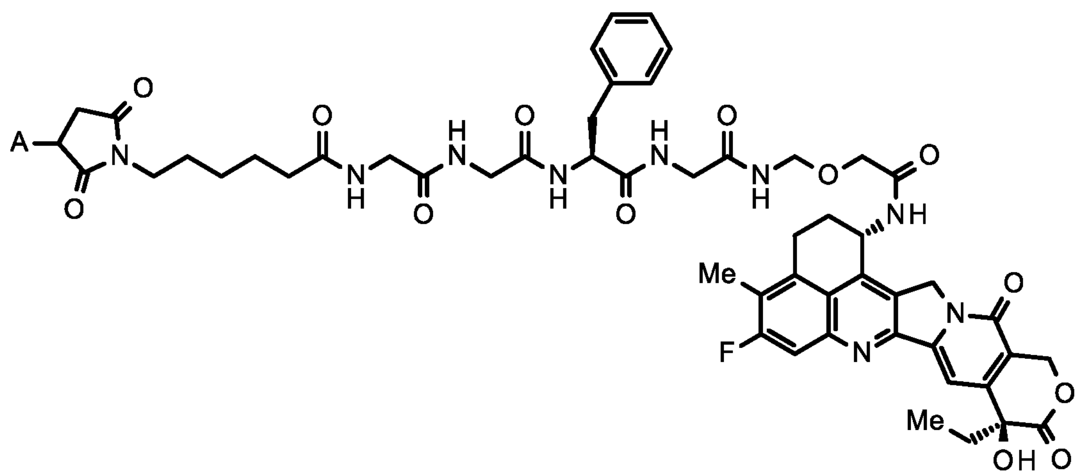

wherein A represents the connecting position to an antibody,
is conjugated to the anti-HER3 antibody via a thioether bond.

The solution obtained was circulated for ultrafiltration through four membranes of Pellicon (R) 2 Ultracel (R) (produced by Merck KGaA, 2.5 $m^2$) with an automated ultrafiltration apparatus while 0.5% sodium chloride-containing 26.5 mM histidine buffer solution (pH 5.0) was added thereto to remove compound (2)-derived by-products. Further, the solution was circulated for ultrafiltration while 26.5 mM histidine buffer solution (pH 5.0) was added thereto to remove sodium chloride. Next, the resultant was concentrated while the pH was adjusted to 5.4 using 26.5 mM aqueous solution of histidine, and thus 97.4 kg of a solution containing an anti-HER3 antibody-drug conjugate (96.5 L, protein concentration:

30.5 mg/mL, protein yield: 2.94 kg) was obtained.

Further, a portion of 48.6 kg was taken from the solution, to which histidine buffer solution (pH 5.4, 24.3 kg) containing 6.29 kg of sucrose was added, and furthermore 9% sucrose-containing histidine buffer solution (pH 5.4, 4.25 kg) containing polysorbate 20 (7.6 g) was added thereto to adjust the protein concentration to approximately 20 mg/mL, and thus a pharmaceutical composition (77.1 kg) containing the anti-HER3 antibody-drug conjugate was obtained. The protein concentration of the pharmaceutical composition, the protein yield, and the average number of conjugated drug molecules per antibody molecule (n) were 19.9 mg/mL, 1.49 kg, and 7.6, respectively.

[Example 7] Production of pharmaceutical composition containing anti-GPR20 antibody-drug conjugate

A solution containing an anti-GPR20 antibody (an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6) (weight of solution: 24.2 kg; corresponding to 0.94 kg of the antibody) was placed in a 200 L reactor, to which 26 mM aqueous solution of histidine (57.6 kg) containing polysorbate 80 (14.4 g) was further added thereto. The temperature was increased to 30°C, an aqueous solution (3.17 kg) containing tris(2-carboxyethyl)phosphine hydrochloride (9.06 g; 4.9 equivalents per antibody molecule) was added to the mixture, and the resultant was stirred at an inner temperature of 30°C for 3 hours to reduce the interchain disulfide of the antibody.

The reaction solution obtained was cooled, and compound (2) (63.5 g; 9.0 equivalents per antibody molecule) dissolved in 80% aqueous solution of dimethylsulfoxide (7.26 kg) containing acetic acid (3.7 g) was added thereto under stirring at an inner temperature of 15°C over 55 minutes, and the resultant was stirred at the same temperature for 0.5 hours to bind compound (2) to the antibody. Next, 0.1 M aqueous solution of N-acetylcysteine (1.00 kg; 15 equivalents per antibody molecule) was added thereto and the resultant was further stirred at the same temperature for 0.5 hours to quench an excess portion of compound (2), and then the pH was adjusted to 5.0 using 10% aqueous solution of acetic acid. This provided a solution containing an anti-GPR20 antibody-drug conjugate in which a drug-linker represented by a formula:

[Chem. 45]

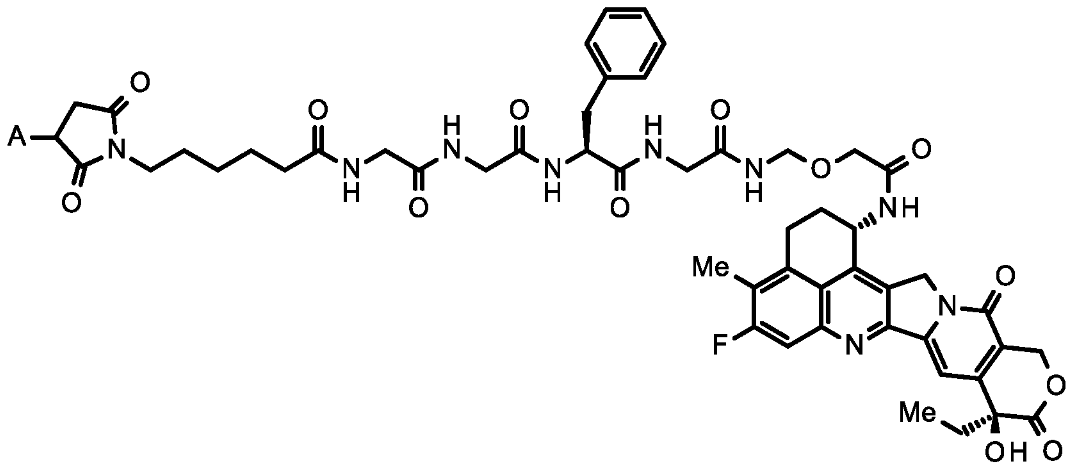

wherein A represents the connecting position to an antibody,
is conjugated to the anti-GPR20 antibody via a thioether bond.

The solution obtained was circulated for ultrafiltration through two membranes of Pellicon (R) 2 Ultracel (R) (produced by Merck KGaA, 2.5 $m^2$) with an ultrafiltration apparatus while 0.5% sodium chloride-containing 11 mM histidine buffer solution (pH 5.0) was added thereto to remove compound (2)-derived by-products. Further, the solution was circulated for ultrafiltration while 11 mM histidine buffer solution (pH 5.0) was added thereto to remove sodium chloride. Next, the resultant was concentrated while the pH was adjusted to 5.4 using 11 mM aqueous solution of histidine, and thus a solution containing an anti-GPR20 antibody-drug conjugate (31.8 kg, protein concentration: 29.9 mg/mL, protein yield: 0.94 kg) was obtained.

Further, to this solution, histidine buffer solution (pH 5.4, 14.9 kg) containing 4.00 kg of sucrose was added, and furthermore 9% sucrose-containing histidine buffer solution (pH 5.4, 3.00 kg) containing polysorbate 80 (4.5 g) was

added thereto to adjust the protein concentration to approximately 20 mg/mL, and thus a pharmaceutical composition (49.3 kg) containing the anti-GPR20 antibody-drug conjugate was obtained. The protein concentration of the pharmaceutical composition, the protein yield, and the average number of conjugated drug molecules per antibody molecule (n) were 19.9 mg/mL, 0.94 kg, and 7.8, respectively.

[Example 8] Production of pharmaceutical composition containing anti-CDH6 antibody-drug conjugate

A solution containing an anti-CDH6 antibody (an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8) (weight of solution: 2.56 kg; corresponding to 100 g of the antibody) was placed in a 14 L reactor, to which 100 mM aqueous solution of histidine (6.00 kg) containing polysorbate 80 (1.50 g) and 0.5 M aqueous solution of EDTA (7.54 g; 5 equivalents per antibody) was further added thereto. The temperature was increased to 30°C, an aqueous solution (330 g) containing tris(2-carboxyethyl)phosphine hydrochloride (945 mg; 4.8 equivalents per antibody molecule) was added to the mixture, and the resultant was stirred at an inner temperature of 30°C for 3 hours to reduce the interchain disulfide of the antibody.

The reaction solution obtained was cooled, and compound (2) (6.96 g; 9.1 equivalents per antibody molecule) dissolved in 80% aqueous solution of dimethylsulfoxide (687 g) containing 10% aqueous solution of acetic acid (3.89 g) was added thereto under stirring at an inner temperature of 15°C over 41 minutes, and the resultant was stirred at the same temperature for 0.4 hours to bind compound (2) to the antibody. Next, 0.1 M aqueous solution of N-acetylcysteine (103 g; 15 equivalents per antibody molecule) was added thereto and the resultant was further stirred at the same temperature for 0.6 hours to quench an excess portion of compound (2), and then the pH was adjusted to 5.0 using 10% aqueous solution of acetic acid. This provided a solution containing an anti-CDH6 antibody-drug conjugate in which a drug-linker represented by a formula:

[Chem. 46]

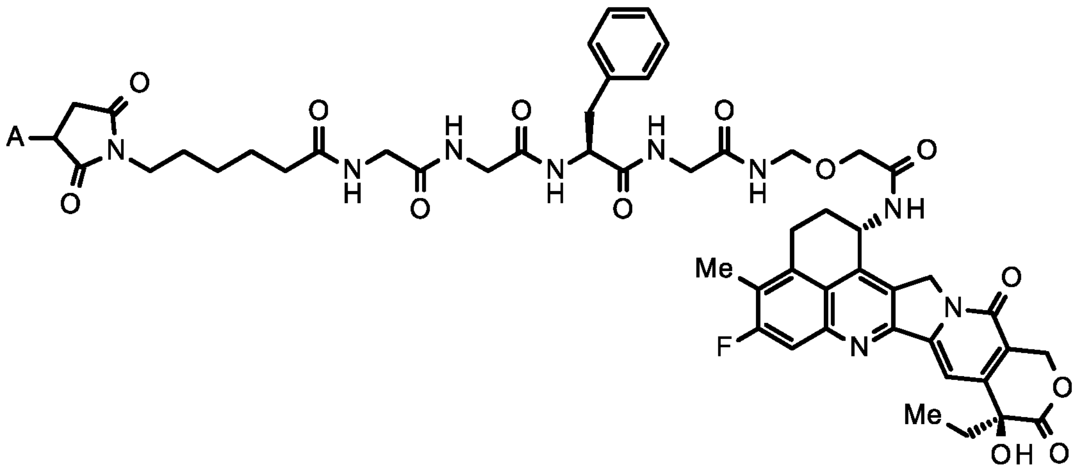

wherein A represents the connecting position to an antibody, is conjugated to the anti-CDH6 antibody via a thioether bond.

The solution obtained was circulated for ultrafiltration through one membrane of Pellicon (R) 2 Ultracel (R) (produced by Merck KGaA, 0.5 $m^2$) with an ultrafiltration apparatus while 0.5% sodium chloride-containing 11 mM histidine buffer solution (pH 5.0) was added thereto to remove compound (2)-derived by-products. Further, the solution was circulated for ultrafiltration while 11 mM histidine buffer solution (pH 5.0) was added thereto to remove sodium chloride. Next, the resultant was concentrated while the pH was adjusted to 5.4 using 11 mM aqueous solution of histidine, and thus a solution containing an anti-CDH6 antibody-drug conjugate (2.97 kg, protein concentration: 31.8 mg/mL, protein yield: 93.7 g) was obtained.

Further, to this solution, histidine buffer solution (pH 5.4, 1.67 kg) containing sucrose (401 g) was added, and furthermore 9% sucrose-containing histidine buffer solution (pH 5.4, 276 g) containing polysorbate 80 (437 mg) was added thereto to adjust the protein concentration to approximately 20 mg/mL, and thus a pharmaceutical composition (4.81 kg) containing the anti-CDH6 antibody-drug conjugate was obtained. The protein concentration of the pharmaceutical composition, the protein yield, and the average number of conjugated drug molecules per antibody molecule

(n) were 20.2 mg/mL, 93.2 g, and 7.8, respectively.

Free Text of Sequence Listing

SEQ ID NO: 1 - Amino acid sequence of a heavy chain of the anti-HER2 antibody
SEQ ID NO: 2 - Amino acid sequence of a light chain of the anti-HER2 antibody
SEQ ID NO: 3 - Amino acid sequence of a heavy chain of the anti-HER3 antibody
SEQ ID NO: 4 - Amino acid sequence of a light chain of the anti-HER3 antibody
SEQ ID NO: 5 - Amino acid sequence of a heavy chain of the anti-GPR20 antibody
SEQ ID NO: 6 - Amino acid sequence of a light chain of the anti-GPR20 antibody
SEQ ID NO: 7 - Amino acid sequence of a heavy chain of the anti-CDH6 antibody
SEQ ID NO: 8 - Amino acid sequence of a light chain of the anti-CDH6 antibody

## Claims

1. A method for producing an antibody-drug conjugate, in which a drug-linker represented by formula (1)

[Chem. 1]

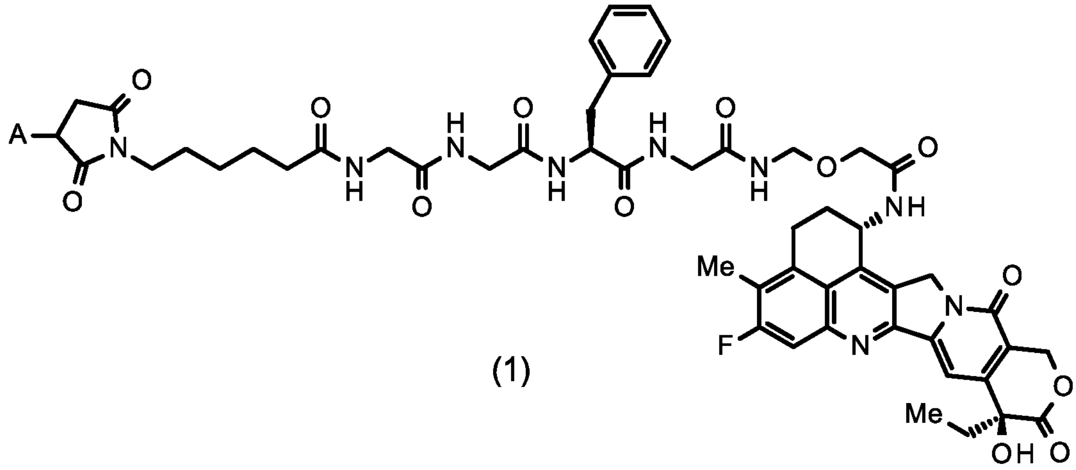

(1)

wherein A represents the connecting position to an antibody, is conjugated to the antibody via a thioether bond, wherein the method comprises the steps of:

(i) reducing the antibody with a reducing agent;
(ii) reacting a compound represented by formula (2)

[Chem. 2]

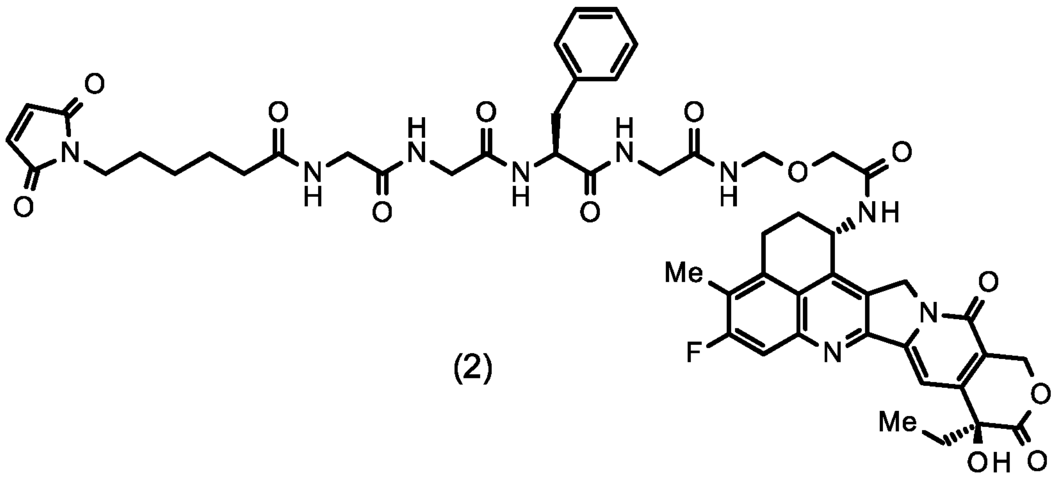

(2)

with the antibody reduced in step (i);
(iii) adding a reagent having a thiol group to react with the residual compound represented by formula (2) in step (ii); and then
(iv) removing a compound in which the reducing agent used in step (i) is added to the maleimidyl group of the compound represented by formula (2), and a compound in which the reagent having a thiol group used in step (iii) is added to the maleimidyl group of the compound represented by formula (2), through ultrafiltration using a buffer solution containing a salt consisting of a strong acid and a strong base.

**2.** The production method according to claim 1, wherein the reducing agent used in step (i) is tris(2-carboxyethyl)phosphine or a salt thereof.

**3.** The production method according to claim 1, wherein the reducing agent used in step (i) is tris(2-carboxyethyl)phosphine hydrochloride.

**4.** The production method according to any one of claims 1 to 3, wherein step (i) is performed in a buffer solution.

**5.** The production method according to claim 4, wherein the pH of the buffer solution is adjusted to 6 to 8 by using an aqueous solution of disodium hydrogen phosphate.

**6.** The production method according to claim 4 or 5, wherein the buffer solution is an acetate buffer solution.

**7.** The production method according to any one of claims 1 to 6, wherein step (i) is performed in the presence of a chelating agent.

**8.** The production method according to claim 7, wherein the chelating agent is ethylenediaminetetraacetic acid.

**9.** The production method according to any one of claims 4 to 8, wherein the buffer solution used in step (i) contains a surfactant.

**10.** The production method according to claim 9, wherein the surfactant is polysorbate 20.

**11.** The production method according to claim 9, wherein the surfactant is polysorbate 80.

**12.** The production method according to any one of claims 1 to 11, wherein the reagent having a thiol group used in step (iii) is N-acetylcysteine.

**13.** The production method according to any one of claims 1 to 12, wherein the pH of the buffer solution used in step (iv) is about 5.

**14.** The production method according to any one of claims 1 to 13, wherein the buffer solution used in step (iv) is a histidine buffer solution.

**15.** The production method according to any one of claims 1 to 14, wherein the concentration of the salt consisting of a strong acid and a strong base used in step (iv) is in the range of from 0.2 wt% to 1 wt% with respect to the buffer solution used in step (iv).

**16.** The production method according to any one of claims 1 to 14, wherein the concentration of the salt consisting of a strong acid and a strong base used in step (iv) is about 0.5 wt% with respect to the buffer solution used in step (iv).

**17.** The production method according to any one of claims 1 to 16, wherein the salt consisting of a strong acid and a strong base used in step (iv) is sodium chloride.

**18.** The production method according to any one of claims 1 to 17, comprising a step subsequent to step (iv) of
(v) removing the salt consisting of a strong acid and a strong base through ultrafiltration using a buffer solution.

**19.** The production method according to claim 18, wherein the pH of the buffer solution used in step (v) is in the range of from 4 to 6.

**20.** The production method according to claim 18, wherein the pH of the buffer solution used in step (v) is about 5.

**21.** The production method according to any one of claims 18 to 20, wherein the buffer solution used in step (v) is a histidine buffer solution.

**22.** The production method according to any one of claims 1 to 21, wherein the average number of units of the drug-linker conjugated per antibody molecule in the antibody-drug conjugate is in the range of from 7 to 8.

**23.** The production method according to any one of claims 1 to 21, wherein the average number of units of the drug-linker conjugated per antibody molecule in the antibody-drug conjugate is in the range of from 7.5 to 8.

**24.** The production method according to any one of claims 1 to 23, wherein the antibody is an anti-HER2 antibody.

**25.** The production method according to claim 24, wherein the anti-HER2 antibody is an antibody comprising a heavy

chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**26.** The production method according to claim 24, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**27.** The production method according to any one of claims 1 to 23, wherein the antibody is an anti-HER3 antibody.

**28.** The production method according to claim 27, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**29.** The production method according to claim 28, wherein the anti-HER3 antibody lacks a lysine residue at the carboxyl terminus of the heavy chain.

**30.** The production method according to any one of claims 1 to 23, wherein the antibody is an anti-GPR20 antibody.

**31.** The production method according to claim 30, wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.

**32.** The production method according to claim 31, wherein the anti-GPR20 antibody lacks a lysine residue at the carboxyl terminus of the heavy chain.

**33.** The production method according to any one of claims 1 to 23, wherein the antibody is an anti-CDH6 antibody.

**34.** The production method according to claim 33, wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**35.** The production method according to claim 34, wherein the anti-CDH6 antibody lacks a lysine residue at the carboxyl terminus of the heavy chain.

**36.** The production method according to any one of claims 1 to 35, comprising no purification step involving chromatography.

**37.** The production method according to claim 36, wherein the chromatography is at least one selected from the group consisting of gel filtration chromatography, ion exchange chromatography, hydrophobic chromatography, and affinity chromatography.

**38.** A method for producing a pharmaceutical composition containing an antibody-drug conjugate, a buffer solution, and an excipient, by producing the antibody-drug conjugate by the production method according to any one of claims 1 to 37, and then
performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also

performing the step of
(ix) adding the excipient to the solution containing the antibody-drug conjugate.

**39.** The production method according to claim 38, wherein the buffer solution is a histidine buffer solution.

**40.** The production method according to claim 38 or 39, wherein the excipient is sucrose.

**41.** The production method according to claim 38 or 39, wherein the excipient is trehalose.

**42.** A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of claims 38 to 41, and then performing the step of
(x) adding the surfactant to the pharmaceutical composition.

**43.** The production method according to claim 42, wherein the surfactant is polysorbate 80.

**44.** The production method according to claim 42, wherein the surfactant is polysorbate 20.

**45.** A method for producing an antibody-drug conjugate represented by formula (6)

[Chem. 3]

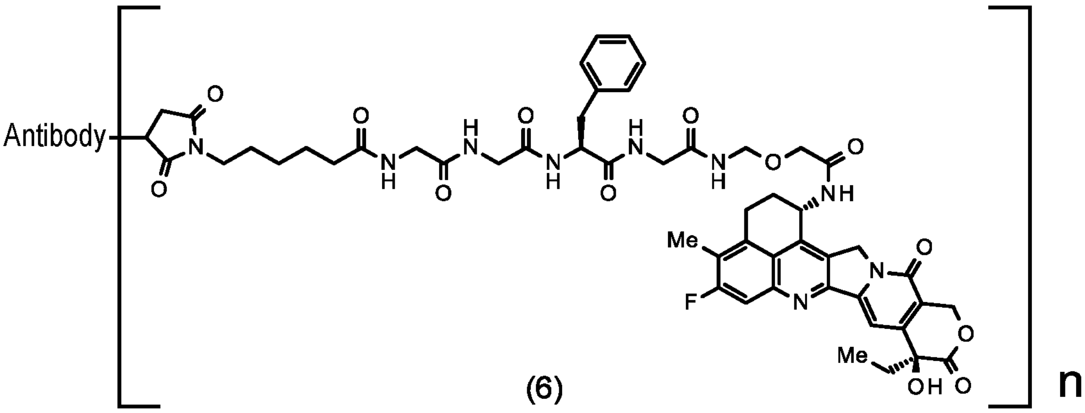

(6)

wherein a drug-linker is conjugated to the antibody via a thioether bond, and n represents the average number of units of the drug-linker conjugated per antibody molecule,
wherein the method comprises the steps of:

(i) reducing the antibody with tris(2-carboxyethyl)phosphine hydrochloride;
(ii) reacting a compound represented by formula (2)

[Chem. 4]

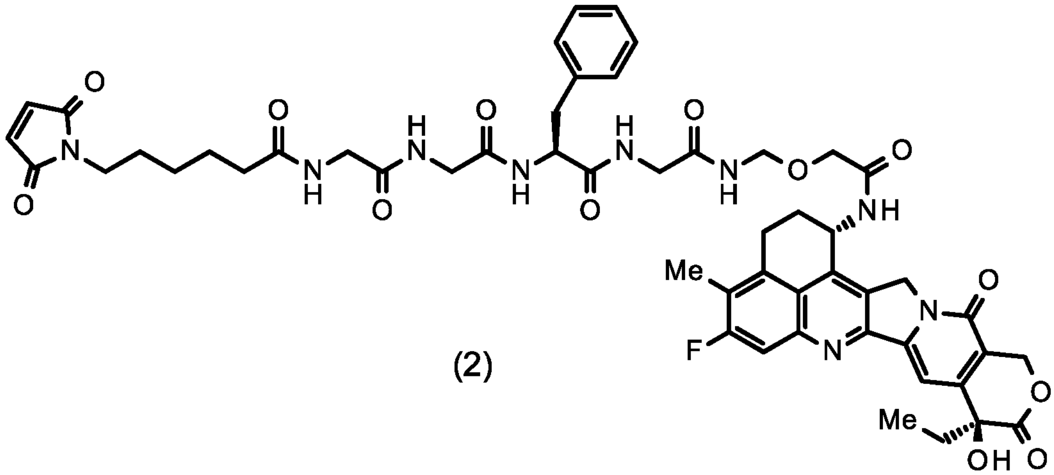

(2)

with the antibody reduced in step (i);
(iii) adding N-acetylcysteine to react with the residual compound represented by formula (2) in step (ii); and then

(iv) removing a compound represented by formula (3)

[Chem. 5]

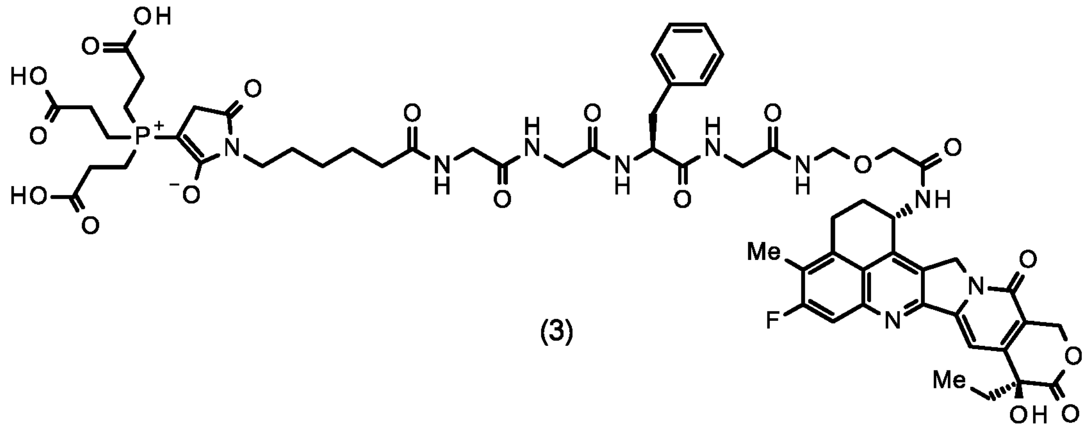

(3)

and a compound represented by formula (4)

[Chem. 6]

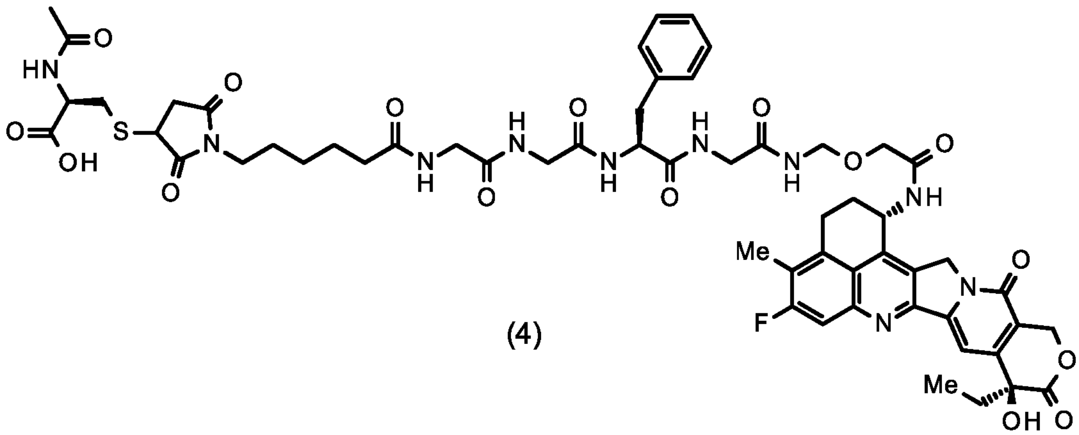

(4)

through ultrafiltration using a histidine buffer solution containing sodium chloride.

**46.** The production method according to claim 45, wherein step (i) is performed in a buffer solution.

**47.** The production method according to claim 46, wherein the pH of the buffer solution is adjusted to 6 to 8 by using an aqueous solution of disodium hydrogen phosphate.

**48.** The production method according to claim 46 or 47, wherein the buffer solution is an acetate buffer solution.

**49.** The production method according to any one of claims 45 to 48, wherein step (i) is performed in the presence of a chelating agent.

**50.** The production method according to claim 49, wherein the chelating agent is ethylenediaminetetraacetic acid.

**51.** The production method according to any one of claims 45 to 50, wherein the buffer solution used in step (i) contains a surfactant.

**52.** The production method according to claim 51, wherein the surfactant is polysorbate 20.

**53.** The production method according to claim 51, wherein the surfactant is polysorbate 80.

**54.** The production method according to any one of claims 45 to 53, wherein the pH of the buffer solution used in step (iv) is about 5.

**55.** The production method according to any one of claims 45 to 54, wherein the concentration of sodium chloride used in step (iv) is in the range of from 0.2 wt% to 1 wt% with respect to the buffer solution used in step (iv).

**56.** The production method according to any one of claims 45 to 54, wherein the concentration of sodium chloride used in step (iv) is about 0.5 wt% with respect to the buffer solution used in step (iv).

**57.** The production method according to any one of claims 45 to 56, comprising a step subsequent to step (iv) of
(v) removing sodium chloride through ultrafiltration using a histidine buffer solution.

**58.** The production method according to claim 57, wherein the pH of the buffer solution used in step (v) is in the range of from 4 to 6.

**59.** The production method according to claim 57, wherein the pH of the buffer solution used in step (v) is about 5.

**60.** The production method according to any one of claims 45 to 59, wherein the average number of units of the drug-linker conjugated per antibody molecule in the antibody-drug conjugate is in the range of from 7 to 8.

**61.** The production method according to any one of claims 45 to 59, wherein the average number of units of the drug-linker conjugated per antibody molecule in the antibody-drug conjugate is in the range of from 7.5 to 8.

**62.** The production method according to any one of claims 45 to 61, wherein the antibody is an anti-HER2 antibody.

**63.** The production method according to claim 62, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**64.** The production method according to claim 62, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**65.** The production method according to any one of claims 45 to 61, wherein the antibody is an anti-HER3 antibody.

**66.** The production method according to claim 65, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**67.** The production method according to claim 66, wherein the anti-HER3 antibody lacks a lysine residue at the carboxyl terminus of the heavy chain.

**68.** The production method according to any one of claims 45 to 61, wherein the antibody is an anti-GPR20 antibody.

**69.** The production method according to claim 68, wherein the anti-GPR20 antibody is an antibody comprising a heavy chain consisting of an amino acid sequence consisting of amino acid residues 20 to 472 of SEQ ID NO: 5 and a light chain consisting of an amino acid sequence consisting of amino acid residues 21 to 234 of SEQ ID NO: 6.

**70.** The production method according to claim 69, wherein the anti-GPR20 antibody lacks a lysine residue at the carboxyl terminus of the heavy chain.

**71.** The production method according to any one of claims 45 to 61, wherein the antibody is an anti-CDH6 antibody.

**72.** The production method according to claim 71, wherein the anti-CDH6 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**73.** The production method according to claim 72, wherein the anti-CDH6 antibody lacks a lysine residue at the carboxyl terminus of the heavy chain.

**74.** The production method according to any one of claims 45 to 73, comprising no purification step involving chromatography.

**75.** The production method according to claim 74, wherein the chromatography is at least one selected from the group consisting of gel filtration chromatography, ion exchange chromatography, hydrophobic chromatography, and affinity chromatography.

**76.** A method for producing a pharmaceutical composition containing an antibody-drug conjugate, a buffer solution, and an excipient, by producing the antibody-drug conjugate by the production method according to any one of claims 45 to 75, and then
performing the steps of at least one selected from the group consisting of:

(vi) adding a buffer solution to a solution containing the antibody-drug conjugate;
(vii) concentrating the solution containing the antibody-drug conjugate; and
(viii) adjusting the pH of the solution containing the antibody-drug conjugate to a predetermined pH; and also performing the step of
(ix) adding the excipient to the solution containing the antibody-drug conjugate.

**77.** The production method according to claim 76, wherein the buffer solution is a histidine buffer solution.

**78.** The production method according to claim 76 or 77, wherein the excipient is sucrose.

**79.** The production method according to claim 76 or 77, wherein the excipient is trehalose.

**80.** A method for producing a pharmaceutical composition containing an antibody-drug conjugate, a buffer solution, an excipient, and a surfactant, by producing the pharmaceutical composition containing an antibody-drug conjugate, a buffer solution, and an excipient by the production method according to any one of claims 76 to 79, and then performing the step of
(x) adding the surfactant to the pharmaceutical composition.

**81.** The production method according to claim 80, wherein the surfactant is polysorbate 80.

**82.** The production method according to claim 80, wherein the surfactant is polysorbate 20.

[Figure 1]

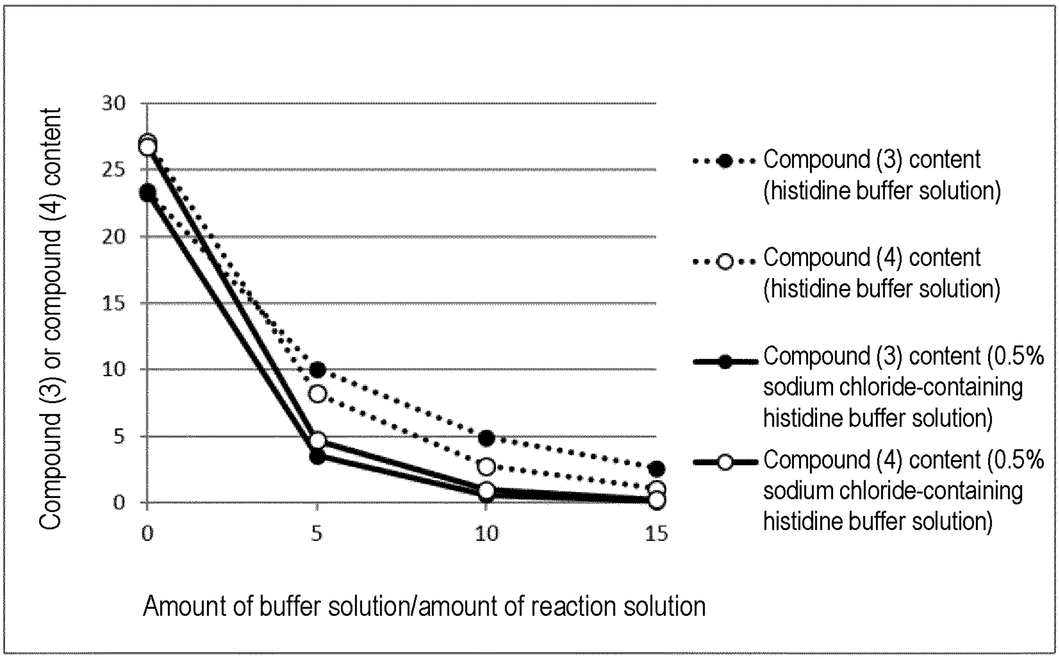


[Figure 2]

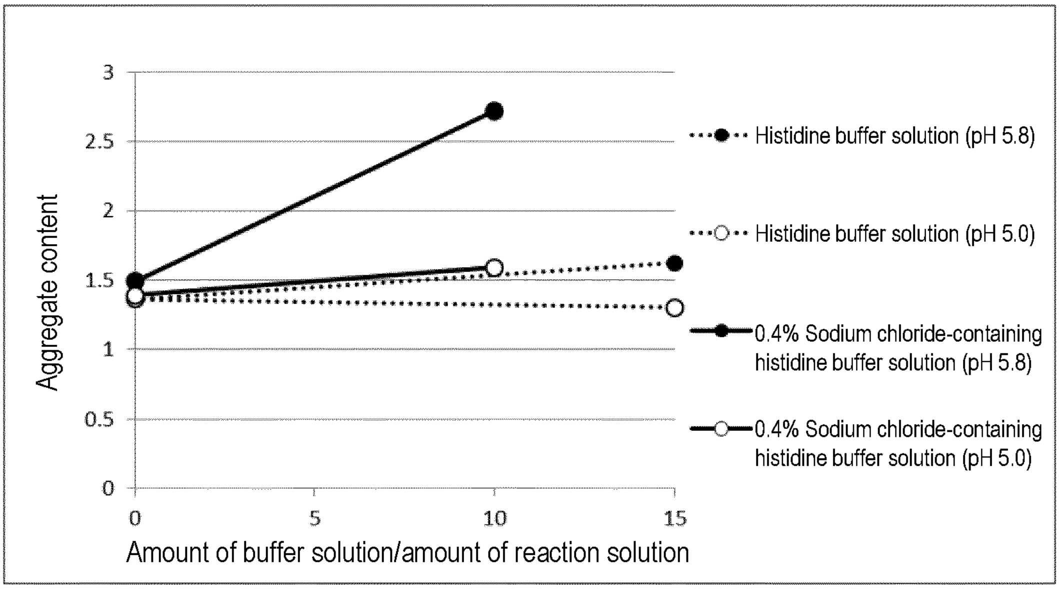

[Figure 3]

SEQ ID NO: 1 - Amino acid sequence of a heavy chain of the anti-HER2 antibody

EVQLVESGGGLVQPGGSLRLSCAASGFNIKDTYIHWVR
QAPGKGLEWVARIYPTNGYTRYADSVKGRFTISADTSK
NTAYLQMNSLRAEDTAVYYCSRWGGDGFYAMDYWGQGT
LVTVSSASTKGPSVFPLAPSSKSTSGGTAALGCLVKDY
FPEPVTVSWNSGALTSGVHTFPAVLQSSGLYSLSSVVT
VPSSSLGTQTYICNVNHKPSNTKVDKKVEPKSCDKTHT
CPPCPAPELLGGPSVFLFPPKPKDTLMISRTPEVTCVV
VDVSHEDPEVKFNWYVDGVEVHNAKTKPREEQYNSTYR
VVSVLTVLHQDWLNGKEYKCKVSNKALPAPIEKTISKA
KGQPREPQVYTLPPSREEMTKNQVSLTCLVKGFYPSDI
AVEWESNGQPENNYKTTPPVLDSDGSFFLYSKLTVDKS
RWQQGNVFSCSVMHEALHNHYTQKSLSLSPGK

[Figure 4]

SEQ ID NO: 2 - Amino acid sequence of a light chain of the anti-HER2 antibody

DIQMTQSPSSLSASVGDRVTITCRASQDVNTAVAWYQQ
KPGKAPKLLIYSASFLYSGVPSRFSGSRSGTDFTLTIS
SLQPEDFATYYCQQHYTTPPTFGQGTKVEIKRTVAAPS
VFIFPPSDEQLKSGTASVVCLLNNFYPREAKVQWKVDN
ALQSGNSQESVTEQDSKDSTYSLSSTLTLSKADYEKHK
VYACEVTHQGLSSPVTKSFNRGEC

[Figure 5]

SEQ ID NO: 3 - Amino acid sequence of a heavy chain of the anti-HER3 antibody

QVQLQQWGAGLLKPSETLSLTCAVYGGSFSGYYWSWIR
QPPGKGLEWIGEINHSGSTNYNPSLKSRVTISVETSKN
QFSLKLSSVTAADTAVYYCARDKWTWYFDLWGRGTLVT
VSSASTKGPSVFPLAPSSKSTSGGTAALGCLVKDYFPE
PVTVSWNSGALTSGVHTFPAVLQSSGLYSLSSVVTVPS
SSLGTQTYICNVNHKPSNTKVDKRVEPKSCDKTHTCPP
CPAPELLGGPSVFLFPPKPKDTLMISRTPEVTCVVVDV
SHEDPEVKFNWYVDGVEVHNAKTKPREEQYNSTYRVVS
VLTVLHQDWLNGKEYKCKVSNKALPAPIEKTISKAKGQ
PREPQVYTLPPSREEMTKNQVSLTCLVKGFYPSDIAVE
WESNGQPENNYKTTPPVLDSDGSFFLYSKLTVDKSRWQ
QGNVFSCSVMHEALHNHYTQKSLSLSPGK

[Figure 6]

SEQ ID NO: 4 - Amino acid sequence of a light chain of the anti-HER3 antibody

DIEMTQSPDSLAVSLGERATINCRSSQSVLYSSSNRNY
LAWYQQNPGQPPKLLIYWASTRESGVPDRFSGSGSGTD
FTLTISSLQAEDVAVYYCQQYYSTPRTFGQGTKVEIKR
TVAAPSVFIFPPSDEQLKSGTASVVCLLNNFYPREAKV
QWKVDNALQSGNSQESVTEQDSKDSTYSLSSTLTLSKA
DYEKHKVYACEVTHQGLSSPVTKSFNRGEC

[Figure 7]

SEQ ID NO: 5 - Amino acid sequence of a heavy chain of the anti-GPR20 antibody

MKHLWFFLLLVAAPRWVLSEVQLVQSGAEVKKPGASVK
VSCKASGYTFTSYYISWIRQAPGQGLKYMGFINPGSGH
TNYNEKFKGRVTITADKSSSTATMELSSLRSEDTAVYY
CARGAGGFLRIITKFDYWGQGTLVTVSSASTKGPSVFP
LAPSSKSTSGGTAALGCLVKDYFPEPVTVSWNSGALTS
GVHTFPAVLQSSGLYSLSSVVTVPSSSLGTQTYICNVN
HKPSNTKVDKRVEPKSCDKTHTCPPCPAPELLGGPSVF
LFPPKPKDTLMISRTPEVTCVVVDVSHEDPEVKFNWYV
DGVEVHNAKTKPREEQYNSTYRVVSVLTVLHQDWLNGK
EYKCKVSNKALPAPIEKTISKAKGQPREPQVYTLPPSR
EEMTKNQVSLTCLVKGFYPSDIAVEWESNGQPENNYKT
TPPVLDSDGSFFLYSKLTVDKSRWQQGNVFSCSVMHEA
LHNHYTQKSLSLSPGK

Signal sequence (1-19), Variable region (20-142), Constant region (143-472)

[Figure 8]

SEQ ID NO: 6 - Amino acid sequence of a light chain of the anti-GPR20 antibody

MVLQTQVFISLLLWISGAYGDTQLTQSPSSLSASVGDR
VTITCRASKSVSTYIHWYQQKPGKQPKLLIYSAGNLES
GVPSRFSGSGSGTDFTLTISSLQPEDFANYYCQQINEL
PYTFGQGTKVEIKRTVAAPSVFIFPPSDEQLKSGTASV
VCLLNNFYPREAKVQWKVDNALQSGNSQESVTEQDSKD
STYSLSSTLTLSKADYEKHKVYACEVTHQGLSSPVTKS
FNRGEC

Signal sequence (1-20), Variable region (21-129), Constant region (130-234)

[Figure 9]

SEQ ID NO: 7 - Amino acid sequence of a heavy chain of the anti-CDH6 antibody

MKHLWFFLLLVAAPRWVLSEVQLVQSGAEVKKPGASVK
VSCKASGYTFTRNFMHWVRQAPGQGLEWMGWIYPGDGE
TEYAQKFQGRVTITADTSTSTAYMELSSLRSEDTAVYY
CARGVYGGFAGGYFDFWGQGTLVTVSSASTKGPSVFPL
APSSKSTSGGTAALGCLVKDYFPEPVTVSWNSGALTSG
VHTFPAVLQSSGLYSLSSVVTVPSSSLGTQTYICNVNH
KPSNTKVDKRVEPKSCDKTHTCPPCPAPELLGGPSVFL
FPPKPKDTLMISRTPEVTCVVVDVSHEDPEVKFNWYVD
GVEVHNAKTKPREEQYNSTYRVVSVLTVLHQDWLNGKE
YKCKVSNKALPAPIEKTISKAKGQPREPQVYTLPPSRE
EMTKNQVSLTCLVKGFYPSDIAVEWESNGQPENNYKTT
PPVLDSDGSFFLYSKLTVDKSRWQQGNVFSCSVMHEAL
HNHYTQKSLSLSPGK

Signal sequence (1-19), Variable region (20-141), Constant region (142-471)

[Figure 10]

SEQ ID NO: 8 - Amino acid sequence of a light chain of the anti-CDH6 antibody

MVLQTQVFISLLLWISGAYGDIQMTQSPSSLSASVGDR
VTITCKASQNIYKNLAWYQQKPGKAPKLLIYDANTLQT
GVPSRFSGSGSGSDFTLTISSLQPEDFATYFCQQYYSG
WAFGQGTKVEIKRTVAAPSVFIFPPSDEQLKSGTASVV
CLLNNFYPREAKVQWKVDNALQSGNSQESVTEQDSKDS
TYSLSSTLTLSKADYEKHKVYACEVTHQGLSSPVTKSF
NRGEC

Signal sequence (1-20), Variable region (21-128), Constant region (129-233)

## INTERNATIONAL SEARCH REPORT

International application No. PCT/JP2019/028949

A. CLASSIFICATION OF SUBJECT MATTER
Int.Cl. C07K16/32(2006.01)i, A61K9/08(2006.01)i, A61K47/18(2006.01)i, A61K47/22(2006.01)i, A61K47/26(2006.01)i, A61K47/68(2017.01)i, A61P35/00(2006.01)i, A61K31/4745(2006.01)n, A61K39/395(2006.01)n

According to International Patent Classification (IPC) or to both national classification and IPC

B. FIELDS SEARCHED

Minimum documentation searched (classification system followed by classification symbols)
Int.Cl. C07K16/32, A61K9/08, A61K47/18, A61K47/22, A61K47/26, A61K47/68, A61P35/00, A61K31/4745, A61K39/395

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)
CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2017/002776 A1 (DAIICHI SANKYO CO., LTD.) 05 January 2017, claims, examples & US 2018/0147292 A, claims, examples & EP 3315512 A | 1-82 |
| Y | JP 2013-508387 A (ABBOTT LABORATORIES) 07 March 2013, paragraph [0109] & US 2011/0206687 A1, paragraph [0132] & WO 2011/050071 A2 & EP 2491055 A | 1-82 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 October 2019 (09.10.2019) | 21 October 2019 (21.10.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/028949

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-202444 A (JAPAN SCIENCE AND TECHNOLOGY AGENCY) 16 August 2007, paragraph [0167] & WO 2007/088823 A1 | 1-82 |
| Y | XENOPOULOS, Alex. et al., "Selectivity improvements in highly charged UF membranes", Desalination, 2006, vol. 199, page 538, abstract | 1-82 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2002098883 A
- WO 2005037992 A
- WO 2005084390 A
- WO 2006086733 A
- WO 2007024536 A
- WO 2010141566 A
- WO 2011039724 A
- WO 2012135517 A
- WO 2015104359 A
- WO 2014057687 A
- WO 2015098099 A
- WO 2015115091 A
- WO 2015155998 A
- WO 2017002776 A
- WO 9007861 A
- US 5821337 A
- WO 9954342 A
- WO 0061739 A
- WO 0231140 A
- WO 0100245 A
- WO 2007077028 A
- WO 2008100624 A
- WO 2018135501 A
- WO 2018212136 A
- WO 2019044947 A


### Non-patent literature cited in the description


- **DUCRY, L. et al.** *Bioconjugate Chem.,* 2010, vol. 21, 5-13
- **ALLEY, S. C. et al.** *Current Opinion in Chemical Biology,* 2010, vol. 14, 529-537
- **DAMLE N. K.** *Expert Opin. Biol. Ther.,* 2004, vol. 4, 1445-1452
- **SENTER P. D. et al.** *Nature Biotechnology,* 2012, vol. 30, 631-637
- **HOWARD A. et al.** *J Clin Oncol,* vol. 29, 398-405
- **OGITANI Y. et al.** *Clinical Cancer Research,* 2016, vol. 22 (20), 5097-5108
- **OGITANI Y. et al.** *Cancer Science,* 2016, vol. 107, 1039-1046
- **DOI T et al.** *Lancet Oncol,* 2017, vol. 18, 1512-22
- **TAKEGAWA N et al.** *Int. J. Cancer,* 2017, vol. 141, 1682-1689
- **OGITANI Y. et al.** *Clinical Cancer Research,* 15 October 2016, vol. 22 (20), 5097-5108
- *Cell Death and Differentiation,* 2008, vol. 15, 751-761
- *Molecular Biology of the Cell,* December 2004, vol. 15, 5268-5282
- *Bio Techniques,* January 2000, vol. 28, 162-165
- **KOHLER ; MILSTEIN.** *Nature,* 1975, vol. 256, 495-497
- Monoclonal Antibodies. Plenum Press, 1980, 365-367
- *Proc. Natl. Acad. Sci. USA,* 1984, vol. 81, 6851-6855
- *Nature,* 1986, vol. 321, 522-525
- **TOMIZUKA, K. et al.** *Nature Genetics,* 1997, vol. 16, 133-143
- **KUROIWA, Y.** *Nucl. Acids Res.,* 1998, vol. 26, 3447-3448
- **YOSHIDA, H.** Animal Cell Technology:Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- **WORMSTONE, I. M.** *Investigative Ophthalmology & Visual Science,* 2002, vol. 43 (7), 2301-2308
- **CARMEN, S.** *Briefings in Functional Genomics and Proteomics,* 2002, vol. 1 (2), 189-203
- **SIRIWARDENA, D.** *Ophthalmology,* 2002, vol. 109 (3), 427-431
- *Journal of Chromatography A,* 1995, vol. 705, 129-134
- *Analytical Biochemistry,* 2007, vol. 360, 75-83
- *Protein Science,* 1995, vol. 4, 2411-2423

SEQUENCE LISTING

<110> DAIICHI SANKYO COMPANY, LIMITED

<120> EFFECTIVE MANUFACTURING METHOD OF ANTIBODY-DRUG CONJUGATE

<130> DSPCT-FP1910

<150> JP2018-139633
<151> 2018-07-25

<160> 8

<170> PatentIn version 3.5

<210> 1
<211> 450
<212> PRT
<213> Artificial Sequence

<220>
<223> Heavy chain of anti-HER2 antibody

<400> 1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160
```

```
Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
```

405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 2
<211> 214
<212> PRT
<213> Artificial Sequence

<220>
<223> Light chain of anti-HER2 antibody

<400> 2

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 3
<211> 447
<212> PRT
<213> Artificial Sequence

<220>
<223> Heavy chain of anti-HER3 antibody

<400> 3

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Gly Tyr
20 25 30

Tyr Trp Ser Trp Ile Arg Gln Pro Pro Gly Lys Gly Leu Glu Trp Ile
35 40 45

Gly Glu Ile Asn His Ser Gly Ser Thr Asn Tyr Asn Pro Ser Leu Lys
50 55 60

Ser Arg Val Thr Ile Ser Val Glu Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Asp Lys Trp Thr Trp Tyr Phe Asp Leu Trp Gly Arg Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

Leu Val Lys Asp Tyr Phe Pro Glu Pro Val Thr Val Ser Trp Asn Ser
145 150 155 160

Gly Ala Leu Thr Ser Gly Val His Thr Phe Pro Ala Val Leu Gln Ser
165 170 175

Ser Gly Leu Tyr Ser Leu Ser Ser Val Val Thr Val Pro Ser Ser Ser
180 185 190

Leu Gly Thr Gln Thr Tyr Ile Cys Asn Val Asn His Lys Pro Ser Asn
195 200 205

Thr Lys Val Asp Lys Arg Val Glu Pro Lys Ser Cys Asp Lys Thr His
210 215 220

Thr Cys Pro Pro Cys Pro Ala Pro Glu Leu Leu Gly Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Lys Phe Asn Trp Tyr Val Asp Gly Val Glu Val His Asn Ala Lys
275 280 285

Thr Lys Pro Arg Glu Glu Gln Tyr Asn Ser Thr Tyr Arg Val Val Ser
290 295 300

Val Leu Thr Val Leu His Gln Asp Trp Leu Asn Gly Lys Glu Tyr Lys
305 310 315 320

Cys Lys Val Ser Asn Lys Ala Leu Pro Ala Pro Ile Glu Lys Thr Ile
325 330 335

Ser Lys Ala Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 4
<211> 220
<212> PRT
<213> Artificial Sequence

<220>
<223> Light chain of anti-HER3 antibody

<400> 4

Asp Ile Glu Met Thr Gln Ser Pro Asp Ser Leu Ala Val Ser Leu Gly
1 5 10 15

Glu Arg Ala Thr Ile Asn Cys Arg Ser Ser Gln Ser Val Leu Tyr Ser
20 25 30

Ser Ser Asn Arg Asn Tyr Leu Ala Trp Tyr Gln Gln Asn Pro Gly Gln
35 40 45

Pro Pro Lys Leu Leu Ile Tyr Trp Ala Ser Thr Arg Glu Ser Gly Val
50 55 60

Pro Asp Arg Phe Ser Gly Ser Gly Ser Gly Thr Asp Phe Thr Leu Thr
65 70 75 80

Ile Ser Ser Leu Gln Ala Glu Asp Val Ala Val Tyr Tyr Cys Gln Gln
85 90 95

Tyr Tyr Ser Thr Pro Arg Thr Phe Gly Gln Gly Thr Lys Val Glu Ile
100 105 110

Lys Arg Thr Val Ala Ala Pro Ser Val Phe Ile Phe Pro Pro Ser Asp
115 120 125

Glu Gln Leu Lys Ser Gly Thr Ala Ser Val Val Cys Leu Leu Asn Asn
130 135 140

Phe Tyr Pro Arg Glu Ala Lys Val Gln Trp Lys Val Asp Asn Ala Leu
145 150 155 160

Gln Ser Gly Asn Ser Gln Glu Ser Val Thr Glu Gln Asp Ser Lys Asp
165 170 175

Ser Thr Tyr Ser Leu Ser Ser Thr Leu Thr Leu Ser Lys Ala Asp Tyr
180 185 190

Glu Lys His Lys Val Tyr Ala Cys Glu Val Thr His Gln Gly Leu Ser
195 200 205

Ser Pro Val Thr Lys Ser Phe Asn Arg Gly Glu Cys
210 215 220

<210> 5
<211> 472
<212> PRT
<213> Artificial Sequence

<220>
<223> Heavy chain of anti-GPR20 antibody

<400> 5

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Ser Tyr Tyr Ile Ser Trp Ile Arg Gln Ala Pro Gly Gln Gly Leu
50 55 60

Lys Tyr Met Gly Phe Ile Asn Pro Gly Ser Gly His Thr Asn Tyr Asn
65 70 75 80

Glu Lys Phe Lys Gly Arg Val Thr Ile Thr Ala Asp Lys Ser Ser Ser
85 90 95

Thr Ala Thr Met Glu Leu Ser Ser Leu Arg Ser Glu Asp Thr Ala Val
100 105 110

Tyr Tyr Cys Ala Arg Gly Ala Gly Gly Phe Leu Arg Ile Ile Thr Lys
115 120 125

Phe Asp Tyr Trp Gly Gln Gly Thr Leu Val Thr Val Ser Ser Ala Ser
130 135 140

```
Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
305                 310                 315                 320

Tyr Asn Ser Thr Tyr Arg Val Val Ser Val Leu Thr Val Leu His Gln
                325                 330                 335

Asp Trp Leu Asn Gly Lys Glu Tyr Lys Cys Lys Val Ser Asn Lys Ala
            340                 345                 350

Leu Pro Ala Pro Ile Glu Lys Thr Ile Ser Lys Ala Lys Gly Gln Pro
        355                 360                 365

Arg Glu Pro Gln Val Tyr Thr Leu Pro Pro Ser Arg Glu Glu Met Thr
    370                 375                 380

Lys Asn Gln Val Ser Leu Thr Cys Leu Val Lys Gly Phe Tyr Pro Ser
385                 390                 395                 400
```

```
Asp Ile Ala Val Glu Trp Glu Ser Asn Gly Gln Pro Glu Asn Asn Tyr
                405                 410                 415

Lys Thr Thr Pro Pro Val Leu Asp Ser Asp Gly Ser Phe Phe Leu Tyr
            420                 425                 430

Ser Lys Leu Thr Val Asp Lys Ser Arg Trp Gln Gln Gly Asn Val Phe
        435                 440                 445

Ser Cys Ser Val Met His Glu Ala Leu His Asn His Tyr Thr Gln Lys
    450                 455                 460

Ser Leu Ser Leu Ser Pro Gly Lys
465                 470


<210>  6
<211>  234
<212>  PRT
<213>  Artificial Sequence

<220>
<223>  Light chain of anti-GPR20 antibody

<400>  6

Met Val Leu Gln Thr Gln Val Phe Ile Ser Leu Leu Leu Trp Ile Ser
1               5                   10                  15

Gly Ala Tyr Gly Asp Thr Gln Leu Thr Gln Ser Pro Ser Ser Leu Ser
            20                  25                  30

Ala Ser Val Gly Asp Arg Val Thr Ile Thr Cys Arg Ala Ser Lys Ser
        35                  40                  45

Val Ser Thr Tyr Ile His Trp Tyr Gln Gln Lys Pro Gly Lys Gln Pro
    50                  55                  60

Lys Leu Leu Ile Tyr Ser Ala Gly Asn Leu Glu Ser Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Asn Tyr Tyr Cys Gln Gln Ile Asn
            100                 105                 110

Glu Leu Pro Tyr Thr Phe Gly Gln Gly Thr Lys Val Glu Ile Lys Arg
        115                 120                 125
```

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 7
<211> 471
<212> PRT
<213> Artificial Sequence

<220>
<223> Heavy chain of anti-CDH6 antibody

<400> 7

Met Lys His Leu Trp Phe Phe Leu Leu Leu Val Ala Ala Pro Arg Trp
1 5 10 15

Val Leu Ser Glu Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Arg Asn Phe Met His Trp Val Arg Gln Ala Pro Gly Gln Gly Leu
50 55 60

Glu Trp Met Gly Trp Ile Tyr Pro Gly Asp Gly Glu Thr Glu Tyr Ala
65 70 75 80

Gln Lys Phe Gln Gly Arg Val Thr Ile Thr Ala Asp Thr Ser Thr Ser
85 90 95

Thr Ala Tyr Met Glu Leu Ser Ser Leu Arg Ser Glu Asp Thr Ala Val

```
                100                 105                 110

Tyr Tyr Cys Ala Arg Gly Val Tyr Gly Gly Phe Ala Gly Gly Tyr Phe
        115                 120                 125

Asp Phe Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350
```

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 8
<211> 233
<212> PRT
<213> Artificial Sequence

<220>
<223> Light chain of anti-CDH6 antibody

<400> 8

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Lys Ala Ser Gln Asn
35 40 45

Ile Tyr Lys Asn Leu Ala Trp Tyr Gln Gln Lys Pro Gly Lys Ala Pro
50 55 60

Lys Leu Leu Ile Tyr Asp Ala Asn Thr Leu Gln Thr Gly Val Pro Ser
65 70 75 80

Arg Phe Ser Gly Ser Gly Ser Gly Ser Asp Phe Thr Leu Thr Ile Ser

85 90 95

Ser Leu Gln Pro Glu Asp Phe Ala Thr Tyr Phe Cys Gln Gln Tyr Tyr
100 105 110

Ser Gly Trp Ala Phe Gly Gln Gly Thr Lys Val Glu Ile Lys Arg Thr
115 120 125

Val Ala Ala Pro Ser Val Phe Ile Phe Pro Pro Ser Asp Glu Gln Leu
130 135 140

Lys Ser Gly Thr Ala Ser Val Val Cys Leu Leu Asn Asn Phe Tyr Pro
145 150 155 160

Arg Glu Ala Lys Val Gln Trp Lys Val Asp Asn Ala Leu Gln Ser Gly
165 170 175

Asn Ser Gln Glu Ser Val Thr Glu Gln Asp Ser Lys Asp Ser Thr Tyr
180 185 190

Ser Leu Ser Ser Thr Leu Thr Leu Ser Lys Ala Asp Tyr Glu Lys His
195 200 205

Lys Val Tyr Ala Cys Glu Val Thr His Gln Gly Leu Ser Ser Pro Val
210 215 220

Thr Lys Ser Phe Asn Arg Gly Glu Cys
225 230